# EUROPEAN PATENT APPLICATION

(11) **EP 4 039 704 A1**
(43) Date of publication of application: **10.08.2022**
(21) Application number: 20870685.3
(22) Date of filing: 21.09.2020
(51) Int. Cl.: C07K 16/28, C12N 15/13, C12N 15/63, C12N 1/21, A61K 39/395, A61K 48/00

(54) **ANTI-PD-1 ANTIBODY AND USE THEREOF**

(30) Priority: 30.09.2019 CN 201910939944
(71) Applicant: Sichuan Kelun-Biotech Biopharmaceutical Co., Ltd., Chengdu, Sichuan 611138 (CN)
(72) Inventor: LIU, Dengnian, Chengdu, Sichuan 611138 (CN); YU, Haimin, Chengdu, Sichuan 611138 (CN); HU, Jiangjiang, Chengdu, Sichuan 611138 (CN); LIU, Hanjie, Chengdu, Sichuan 611138 (CN); WU, Tao, Chengdu, Sichuan 611138 (CN); XIAO, Liang, Chengdu, Sichuan 611138 (CN); XUE, Tongtong, Chengdu, Sichuan 611138 (CN); WANG, Jingyi, Chengdu, Sichuan 611138 (CN)
(74) Representative: Hoffmann Eitle
(86) International application number: PCT/CN2020/116417
(87) International publication number: WO 2021/063201

(57) **Abstract**

An anti-PD-1 antibody or an antigen-binding fragment thereof, nucleic acid molecules encoding same, and methods for preparing same. The anti-PD-1 antibody or the antigen-binding fragment thereof has high specificity and high affinity to PD-1, and can effectively block the binding of PD-1/PD-L1 and PD-1/PD-L2 so as to activate the immune system, thereby achieving the effect of inhibiting tumor growth. Therefore, a pharmaceutical composition containing the antibody or the antigen-binding fragment thereof and a use of the pharmaceutical composition in preparation of drugs are further comprised. The drugs are used for preventing and/or treating tumors, infections, or autoimmune diseases.

Sequence listing

<110>  SICHUAN KELUN-BIOTECH BIOPHARMACEUTICAL CO., LTD.

<120>  ANTI-PD-1 ANTIBODY AND USE THEREOF

<130>  P2020TC1201

<150>  CN201910939944.8
<151>  2019-09-30

<160>  90

<170>  PatentIn version 3.5

<210>  1
<211>  121
<212>  PRT
<213>  Mus musculus

<400>  1

Gln Val Gln Leu Glu Glu Ser Gly Pro Glu Leu Val Lys Pro Gly Ala
1               5                   10                  15

Ser Val Lys Ile Ser Cys Lys Thr Ser Gly Tyr Thr Phe Thr Glu Tyr
            20                  25                  30

Thr Ile Tyr Trp Val Lys Gln Ser His Gly Lys Ser Leu Glu Trp Ile
        35                  40                  45

Gly Gly Ile Asn Pro Lys Asn Gly Ala Thr Leu Tyr Asn Gln Lys Phe
    50                  55                  60

Lys Val Lys Ala Thr Leu Thr Val Asp Lys Ser Ser Ser Thr Ala Tyr
65                  70                  75                  80

Met Glu Leu Arg Ser Leu Thr Ser Glu Asp Ser Ala Val Tyr Tyr Cys
                85                  90                  95

Val Arg Asp Tyr His Gly Asn Ser His Asp Ser Met Asp Tyr Trp Gly
            100                 105                 110

Gln Gly Thr Ser Val Thr Val Ser Ser
        115                 120


<210>  2
<211>  106
<212>  PRT
<213>  Mus musculus

<400>  2

Gln Ile Val Leu Thr Gln Ser Pro Ala Ile Met Ser Ala Ser Pro Gly
1               5                   10                  15

Glu Lys Val Thr Ile Thr Cys Ser Ala Ser Ser Ser Val Ser Tyr Met
            20                  25                  30

His Trp Phe Arg Gln Lys Pro Gly Thr Ser Pro Lys Leu Trp Ile Tyr
        35                  40                  45

Tyr Thr Ser Asn Leu Ala Ser Gly Val Pro Ala His Phe Ser Gly Ser
    50                  55                  60

Gly Ser Gly Thr Ser Tyr Ser Leu Thr Ile Ser Arg Met Glu Ala Glu
65                  70                  75                  80

Asp Ala Ala Thr Tyr Tyr Cys Gln Gln Arg Ser Asp Tyr Pro Pro Thr
                85                  90                  95

Phe Gly Gly Gly Thr Lys Leu Glu Ile Lys
            100                 105

<210>  3
<211>  8
<212>  PRT
<213>  Mus musculus

<400>  3

Gly Tyr Thr Phe Thr Glu Tyr Thr
1               5

<210>  4
<211>  8
<212>  PRT
<213>  Mus musculus

<400>  4

Ile Asn Pro Lys Asn Gly Ala Thr
1               5

<210>  5
<211>  14
<212>  PRT
<213>  Mus musculus

<400>  5

Val Arg Asp Tyr His Gly Asn Ser His Asp Ser Met Asp Tyr
1               5                   10

<210>  6
<211>  5
<212>  PRT
<213>  Mus musculus

<400>  6

Ser Ser Val Ser Tyr
1               5



<210>  7
<211>  3
<212>  PRT
<213>  Mus musculus

<400>  7

Tyr Thr Ser
1



<210>  8
<211>  9
<212>  PRT
<213>  Mus musculus

<400>  8

Gln Gln Arg Ser Asp Tyr Pro Pro Thr
1               5



<210>  9
<211>  5
<212>  PRT
<213>  Mus musculus

<400>  9

Glu Tyr Thr Ile Tyr
1               5



<210>  10
<211>  17
<212>  PRT
<213>  Mus musculus

<400>  10

Gly Ile Asn Pro Lys Asn Gly Ala Thr Leu Tyr Asn Gln Lys Phe Lys
1               5                   10                  15


Val



<210>  11
<211>  218
<212>  PRT
<213>  Artificial Sequence

<220>
<223>  35E6-HZ1-IgG1-1D light chain

<400>  11

Glu Ile Val Leu Thr Gln Ser Pro Ala Thr Leu Ser Leu Ser Pro Gly
1               5                   10                  15

Glu Arg Ala Thr Leu Ser Cys Arg Ala Ser Glu Ser Val Glu Tyr Tyr
            20                  25                  30

Gly Thr Asn Leu Met Gln Trp Tyr Gln Gln Lys Pro Gly Gln Pro Pro
        35                  40                  45

Arg Leu Leu Ile Tyr Phe Ala Ser Asn Val Ala Ser Gly Ile Pro Ala
    50                  55                  60

Arg Phe Ser Gly Ser Gly Ser Gly Thr Asp Phe Thr Leu Thr Ile Ser
65                  70                  75                  80

Ser Leu Glu Pro Glu Asp Phe Ala Val Tyr Phe Cys Gln Gln Ser Lys
                85                  90                  95

Lys Val Pro Ser Thr Phe Gly Gln Gly Thr Lys Val Glu Ile Lys Arg
            100                 105                 110

Thr Val Ala Ala Pro Ser Val Phe Ile Phe Pro Pro Ser Asp Glu Gln
        115                 120                 125

Leu Lys Ser Gly Thr Ala Ser Val Val Cys Leu Leu Asn Asn Phe Tyr
    130                 135                 140

Pro Arg Glu Ala Lys Val Gln Trp Lys Val Asp Asn Ala Leu Gln Ser
145                 150                 155                 160

Gly Asn Ser Gln Glu Ser Val Thr Glu Gln Asp Ser Lys Asp Ser Thr
                165                 170                 175

Tyr Ser Leu Ser Ser Thr Leu Thr Leu Ser Lys Ala Asp Tyr Glu Lys
            180                 185                 190

His Lys Val Tyr Ala Cys Glu Val Thr His Gln Gly Leu Ser Ser Pro
        195                 200                 205

Val Thr Lys Ser Phe Asn Arg Gly Glu Cys
    210                 215

<210>  12
<211>  12
<212>  PRT
<213>  Mus musculus

<400>  12

Asp Tyr His Gly Asn Ser His Asp Ser Met Asp Tyr
1               5                   10


<210>  13
<211>  10
<212>  PRT
<213>  Mus musculus

<400>  13

Ser Ala Ser Ser Ser Val Ser Tyr Met His
1               5                   10


<210>  14
<211>  7
<212>  PRT
<213>  Mus musculus

<400>  14

Tyr Thr Ser Asn Leu Ala Ser
1               5


<210>  15
<211>  9
<212>  PRT
<213>  Mus musculus

<400>  15

Gln Gln Arg Ser Asp Tyr Pro Pro Thr
1               5


<210>  16
<211>  121
<212>  PRT
<213>  Artificial Sequence

<220>
<223>  humanized 29C6-HZ1 heavy chain variable region

<400>  16

Gln Val Gln Leu Val Gln Ser Gly Ala Glu Val Lys Lys Pro Gly Ala
1               5                   10                  15


Ser Val Lys Val Ser Cys Lys Thr Ser Gly Tyr Thr Phe Thr Glu Tyr
            20                  25                  30


Thr Ile Tyr Trp Val Arg Gln Ala Pro Gly Gln Gly Leu Glu Trp Ile
        35                  40                  45


Gly Gly Ile Asn Pro Lys Asn Gly Ala Thr Leu Tyr Asn Gln Lys Phe
    50                  55                  60

Gln Gly Arg Val Thr Leu Thr Val Asp Lys Ser Thr Ser Thr Ala Tyr
65                  70                  75                  80

Met Glu Leu Arg Ser Leu Arg Ser Asp Asp Thr Ala Val Tyr Tyr Cys
                85                  90                  95

Val Arg Asp Tyr His Gly Asn Ser His Asp Ser Met Asp Tyr Trp Gly
            100                 105                 110

Gln Gly Thr Leu Val Thr Val Ser Ser
        115                 120

<210>  17
<211>  121
<212>  PRT
<213>  Artificial Sequence

<220>
<223>  humanized 29C6-HZ2 heavy chain variable region

<400>  17

Gln Val Gln Leu Val Gln Ser Gly Val Glu Val Lys Lys Pro Gly Ala
1               5                   10                  15

Ser Val Lys Val Ser Cys Lys Thr Ser Gly Tyr Thr Phe Thr Glu Tyr
            20                  25                  30

Thr Ile Tyr Trp Val Arg Gln Ala Pro Gly Gln Gly Leu Glu Trp Ile
        35                  40                  45

Gly Gly Ile Asn Pro Lys Asn Gly Ala Thr Leu Tyr Asn Gln Lys Phe
    50                  55                  60

Lys Val Arg Ala Thr Leu Thr Val Asp Lys Ser Thr Thr Thr Ala Tyr
65                  70                  75                  80

Met Glu Leu Lys Ser Leu Gln Phe Asp Asp Thr Ala Val Tyr Tyr Cys
                85                  90                  95

Val Arg Asp Tyr His Gly Asn Ser His Asp Ser Met Asp Tyr Trp Gly
            100                 105                 110

Gln Gly Thr Thr Val Thr Val Ser Ser
        115                 120

<210>  18
<211>  106
<212>  PRT

<213>  Artificial Sequence

<220>
<223>  humanized 29C6-HZ1 light chain variable region

<400>  18

Glu Ile Val Leu Thr Gln Ser Pro Ala Thr Leu Ser Leu Ser Pro Gly
1               5                   10                  15

Glu Arg Ala Thr Leu Ser Cys Ser Ala Ser Ser Ser Val Ser Tyr Met
            20                  25                  30

His Trp Phe Gln Gln Lys Pro Gly Gln Ala Pro Arg Leu Trp Ile Tyr
        35                  40                  45

Tyr Thr Ser Asn Leu Ala Ser Gly Val Pro Ala Arg Phe Ser Gly Ser
    50                  55                  60

Gly Ser Gly Thr Asp Tyr Thr Leu Thr Ile Ser Ser Leu Glu Pro Glu
65                  70                  75                  80

Asp Phe Ala Val Tyr Tyr Cys Gln Gln Arg Ser Asp Tyr Pro Pro Thr
                85                  90                  95

Phe Gly Gly Gly Thr Lys Val Glu Ile Lys
            100                 105


<210>  19
<211>  106
<212>  PRT
<213>  Artificial Sequence

<220>
<223>  humanized 29C6-HZ2 light chain variable region

<400>  19

Glu Ile Val Leu Thr Gln Ser Pro Ala Thr Leu Ser Leu Ser Pro Gly
1               5                   10                  15

Glu Arg Ala Thr Leu Ser Cys Ser Ala Ser Ser Ser Val Ser Tyr Met
            20                  25                  30

His Trp Phe Gln Gln Lys Pro Gly Gln Ala Pro Arg Leu Leu Ile Tyr
        35                  40                  45

Tyr Thr Ser Asn Leu Ala Ser Gly Val Pro Ala Arg Phe Ser Gly Ser
    50                  55                  60

Gly Ser Gly Thr Asp Tyr Thr Leu Thr Ile Ser Ser Leu Glu Pro Glu
65                  70                  75                  80

Asp Phe Ala Val Tyr Tyr Cys Gln Gln Arg Ser Asp Tyr Pro Pro Thr
                85                  90                  95

Phe Gly Gly Gly Thr Lys Val Glu Ile Lys
            100                 105

<210>  20
<211>  113
<212>  PRT
<213>  Mus musculus

<400>  20

Asp Val Gln Leu Gln Glu Ser Gly Pro Gly Leu Val Lys Pro Ser Gln
1               5                   10                  15

Ser Leu Ser Leu Thr Cys Ser Val Thr Gly Tyr Ser Ile Thr Ser Asp
            20                  25                  30

Tyr Trp Ser Trp Ile Arg Gln Phe Pro Gly Asn Lys Leu Glu Trp Met
        35                  40                  45

Gly Tyr Ile Asn Tyr Asp Gly Asp Asn Asn Tyr Asn Pro Ser Leu Lys
    50                  55                  60

Asn Arg Ile Ser Ile Thr Arg Asp Thr Ser Lys Asn Gln Phe Phe Leu
65                  70                  75                  80

Asn Leu Ser Ser Val Thr Ser Glu Asp Thr Ala Thr Tyr Tyr Cys Ala
                85                  90                  95

Arg Gly Gly Phe Asp Tyr Trp Gly Gln Gly Thr Thr Ile Thr Val Ser
            100                 105                 110

Ser

<210>  21
<211>  111
<212>  PRT
<213>  Mus musculus

<400>  21

Cys Ile Val Leu Thr Gln Ser Pro Ala Ser Leu Ala Val Ser Leu Gly
1               5                   10                  15

Gln Ser Val Thr Ile Ser Cys Arg Ala Ser Glu Ser Val Glu Tyr Tyr
            20                  25                  30

Gly Thr Asn Leu Met Gln Trp Tyr Gln Gln Lys Pro Gly Gln Pro Pro
        35                  40                  45

Lys Leu Leu Ile Tyr Phe Ala Ser Asn Val Ala Ser Gly Val Pro Ala
    50                  55                  60

Arg Phe Ser Gly Ser Gly Ser Gly Thr Asp Phe Ser Leu Asn Ile His
65                  70                  75                  80

Pro Val Glu Gly Asp Asp Ile Ala Arg Tyr Phe Cys Gln Gln Ser Lys
                85                  90                  95

Lys Val Pro Ser Thr Phe Gly Gly Gly Thr Lys Leu Glu Ile Lys
            100                 105                 110

<210>  22
<211>  8
<212>  PRT
<213>  Mus musculus

<400>  22

Gly Tyr Ser Ile Thr Ser Asp Tyr
1               5

<210>  23
<211>  7
<212>  PRT
<213>  Mus musculus

<400>  23

Ile Asn Tyr Asp Gly Asp Asn
1               5

<210>  24
<211>  7
<212>  PRT
<213>  Mus musculus

<400>  24

Ala Arg Gly Gly Phe Asp Tyr
1               5

<210>  25
<211>  10
<212>  PRT
<213>  Mus musculus

<400>  25

Glu Ser Val Glu Tyr Tyr Gly Thr Asn Leu
1               5                   10

<210>  26
<211>  3
<212>  PRT
<213>  Mus musculus

<400>  26

Phe Ala Ser
1

<210>  27
<211>  9
<212>  PRT
<213>  Mus musculus

<400>  27

Gln Gln Ser Lys Lys Val Pro Ser Thr
1               5

<210>  28
<211>  5
<212>  PRT
<213>  Mus musculus

<400>  28

Ser Asp Tyr Trp Ser
1               5

<210>  29
<211>  16
<212>  PRT
<213>  Mus musculus

<400>  29

Tyr Ile Asn Tyr Asp Gly Asp Asn Asn Tyr Asn Pro Ser Leu Lys Asn
1               5                   10                  15

<210>  30
<211>  5
<212>  PRT
<213>  Mus musculus

<400>  30

Gly Gly Phe Asp Tyr
1               5

<210>  31
<211>  15
<212>  PRT
<213>  Mus musculus

<400>  31

Arg Ala Ser Glu Ser Val Glu Tyr Tyr Gly Thr Asn Leu Met Gln
1               5                   10                  15

<210>  32
<211>  7
<212>  PRT
<213>  Mus musculus

<400>  32

Phe Ala Ser Asn Val Ala Ser
1               5

<210>  33
<211>  9
<212>  PRT
<213>  Mus musculus

<400>  33

Gln Gln Ser Lys Lys Val Pro Ser Thr
1               5

<210>  34
<211>  113
<212>  PRT
<213>  Artificial Sequence

<220>
<223>  humanized 35E6-HZ1 heavy chain variable region

<400>  34

Gln Val Gln Leu Gln Glu Ser Gly Pro Gly Leu Val Lys Pro Ser Glu
1               5                   10                  15

Thr Leu Ser Leu Thr Cys Thr Val Ser Gly Tyr Ser Ile Thr Ser Asp
            20                  25                  30

Tyr Trp Ser Trp Ile Arg Gln Pro Pro Gly Lys Gly Leu Glu Trp Ile
        35                  40                  45

Gly Tyr Ile Asn Tyr Asp Gly Asp Asn Asn Tyr Asn Pro Ser Leu Lys
    50                  55                  60

Ser Arg Val Thr Ile Ser Arg Asp Thr Ser Lys Asn Gln Phe Ser Leu
65                  70                  75                  80

Lys Leu Ser Ser Val Thr Ala Ala Asp Thr Ala Val Tyr Tyr Cys Ala
                85                  90                  95

Arg Gly Gly Phe Asp Tyr Trp Gly Gln Gly Thr Leu Val Thr Val Ser
            100                 105                 110

Ser

<210>  35
<211>  111
<212>  PRT
<213>  Artificial Sequence

<220>
<223>  humanized 35E6-HZ1 light chain variable region

<400>  35

Glu Ile Val Leu Thr Gln Ser Pro Ala Thr Leu Ser Leu Ser Pro Gly
1               5                   10                  15

Glu Arg Ala Thr Leu Ser Cys Arg Ala Ser Glu Ser Val Glu Tyr Tyr
            20                  25                  30

Gly Thr Asn Leu Met Gln Trp Tyr Gln Gln Lys Pro Gly Gln Pro Pro
        35                  40                  45

Arg Leu Leu Ile Tyr Phe Ala Ser Asn Val Ala Ser Gly Ile Pro Ala
    50                  55                  60

Arg Phe Ser Gly Ser Gly Ser Gly Thr Asp Phe Thr Leu Thr Ile Ser
65                  70                  75                  80

Ser Leu Glu Pro Glu Asp Phe Ala Val Tyr Phe Cys Gln Gln Ser Lys
                85                  90                  95

Lys Val Pro Ser Thr Phe Gly Gln Gly Thr Lys Val Glu Ile Lys
            100                 105                 110

<210>  36
<211>  116
<212>  PRT
<213>  Mus musculus

<400>  36

Glu Val Gln Leu Gln Gln Ser Gly Pro Glu Leu Val Lys Pro Gly Ala
1               5                   10                  15

Ser Val Lys Ile Ser Cys Arg Ala Ser Gly Tyr Thr Phe Thr Asp Tyr
            20                  25                  30

Tyr Met Asn Trp Val Lys Glu Ser His Gly Lys Ser Leu Glu Trp Ile
        35                  40                  45

Gly Glu Ile Asn Pro Lys Tyr Gly Asp Thr Thr Tyr Asn Gln Lys Phe
    50                  55                  60

Lys Asp Lys Ala Thr Leu Thr Val Asp Lys Ser Ser Ser Thr Ala Ser
65                  70                  75                  80

Val Glu Leu Arg Ser Leu Thr Ser Glu Asp Ser Ala Val Tyr Tyr Cys
                85                  90                  95

Ala Lys Gly Ile Arg Leu Phe Asp Ser Trp Gly Gln Gly Thr Thr Val
            100                 105                 110

Thr Val Ser Ser
        115

<210>  37
<211>  107
<212>  PRT
<213>  Mus musculus

<400>  37

Asp Ile Gln Leu Thr Gln Ser Pro Ala Ser Leu Ser Ala Ser Val Gly
1               5                   10                  15

Glu Thr Val Thr Ile Thr Cys Arg Pro Ser Gly Asn Val His Asn Tyr
            20                  25                  30

Phe Ala Trp Tyr Gln Gln Lys Gln Gly Lys Ser Pro Gln Leu Leu Val
        35                  40                  45

Tyr Asn Ala Lys Thr Leu Ala Glu Gly Val Pro Ser Arg Phe Ser Gly
    50                  55                  60

Ser Gly Ser Gly Thr Gln Tyr Ser Leu Lys Ile Asn Ser Leu Gln Pro
65                  70                  75                  80

Glu Asp Phe Gly Thr Tyr Tyr Cys His His Tyr Tyr Thr Ala Pro Phe
                85                  90                  95

Thr Phe Gly Ser Gly Thr Lys Leu Glu Ile Lys
            100                 105

<210>  38
<211>  8
<212>  PRT
<213>  Mus musculus

<400>  38

Gly Tyr Thr Phe Thr Asp Tyr Tyr

1               5


<210>  39
<211>  8
<212>  PRT
<213>  Mus musculus

<400>  39

Ile Asn Pro Lys Tyr Gly Asp Thr
1               5


<210>  40
<211>  9
<212>  PRT
<213>  Mus musculus

<400>  40

Ala Lys Gly Ile Arg Leu Phe Asp Ser
1               5


<210>  41
<211>  6
<212>  PRT
<213>  Mus musculus

<400>  41

Gly Asn Val His Asn Tyr
1               5


<210>  42
<211>  3
<212>  PRT
<213>  Mus musculus

<400>  42

Asn Ala Lys
1


<210>  43
<211>  9
<212>  PRT
<213>  Mus musculus

<400>  43

His His Tyr Tyr Thr Ala Pro Phe Thr
1               5


<210>  44
<211>  5
<212>  PRT
<213>  Mus musculus

<400>  44

Asp Tyr Tyr Met Asn
1               5

<210>  45
<211>  17
<212>  PRT
<213>  Mus musculus

<400>  45

Glu Ile Asn Pro Lys Tyr Gly Asp Thr Thr Tyr Asn Gln Lys Phe Lys
1               5                   10                  15

Asp

<210>  46
<211>  657
<212>  DNA
<213>  Artificial Sequence

<220>
<223>  35E6-HZ1-IgG1-1D light chain

<400>  46
gagatcgtgc tgacccagtc ccctgccaca ctgtccctga gcccaggaga gagggccacc      60

ctgagctgtc gggcttctga gtccgtggag tactatggca caaacctgat gcagtggtac     120

cagcagaagc caggacagcc acccaggctg ctgatctatt tcgccagcaa tgtggcttct     180

ggcatccccg ctcggttcag cggctctggc tccggcaccg actttaccct gacaatctcc     240

agcctggagc ccgaggattt cgccgtgtac ttttgccagc agtctaagaa ggtgccttcc     300

accttcggcc agggcacaaa ggtggagatc aagagaaccg tggccgctcc tagcgtgttc     360

atctttccac cctctgacga gcagctgaag tccggcacag ctagcgtggt gtgcctgctg     420

aacaacttct acccacgcga ggccaaggtg cagtggaagg tggataacgc tctgcagagc     480

ggcaattctc aggagtccgt gaccgagcag gacagcaagg attctacata ttccctgtct     540

tccaccctga cactgtctaa ggccgactac gagaagcaca aggtgtatgc ttgcgaggtg     600

acccatcagg gcctgagctc tcccgtgaca aagtccttta acaggggcga gtgttga        657

<210>  47
<211>  7
<212>  PRT
<213>  Mus musculus

<400>  47

Gly Ile Arg Leu Phe Asp Ser
1               5

<210>  48
<211>  11
<212>  PRT
<213>  Mus musculus

<400>  48

Arg Pro Ser Gly Asn Val His Asn Tyr Phe Ala
1               5                   10


<210>  49
<211>  7
<212>  PRT
<213>  Mus musculus

<400>  49

Asn Ala Lys Thr Leu Ala Glu
1               5


<210>  50
<211>  9
<212>  PRT
<213>  Mus musculus

<400>  50

His His Tyr Tyr Thr Ala Pro Phe Thr
1               5


<210>  51
<211>  116
<212>  PRT
<213>  Artificial Sequence

<220>
<223>  humanized 91D5-HZ1 heavy chain variable region

<400>  51

Glu Val Gln Leu Val Gln Ser Gly Ala Glu Val Lys Lys Pro Gly Ala
1               5                   10                  15


Ser Val Lys Val Ser Cys Lys Ala Ser Gly Tyr Thr Phe Thr Asp Tyr
            20                  25                  30


Tyr Met Asn Trp Val Arg Glu Ala Pro Gly Gln Gly Leu Glu Trp Met
        35                  40                  45


Gly Glu Ile Asn Pro Lys Tyr Gly Asp Thr Thr Tyr Ala Gln Lys Leu
    50                  55                  60


Gln Gly Arg Val Thr Leu Thr Val Asp Lys Ser Thr Ser Thr Ala Tyr
65                  70                  75                  80

Met Glu Leu Ser Ser Leu Arg Ser Asp Asp Thr Ala Val Tyr Tyr Cys
                85                  90                  95

Ala Lys Gly Ile Arg Leu Phe Asp Ser Trp Gly Gln Gly Thr Leu Val
            100                 105                 110

Thr Val Ser Ser
        115

<210>  52
<211>  116
<212>  PRT
<213>  Artificial Sequence

<220>
<223>  humanized 91D5-HZ2 heavy chain variable region

<400>  52

Glu Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asp Tyr
            20                  25                  30

Tyr Met Asn Trp Val Arg Glu Ala Pro Gly Gln Gly Leu Glu Trp Met
        35                  40                  45

Gly Glu Ile Asn Pro Lys Tyr Gly Asp Thr Thr Tyr Asn Gln Lys Phe
    50                  55                  60

Lys Asp Arg Ala Thr Leu Thr Val Asp Lys Ser Thr Ser Thr Ala Tyr
65                  70                  75                  80

Met Glu Leu Ser Ser Leu Arg Ser Asp Asp Thr Ala Val Tyr Tyr Cys
                85                  90                  95

Ala Lys Gly Ile Arg Leu Phe Asp Ser Trp Gly Gln Gly Thr Leu Val
            100                 105                 110

Thr Val Ser Ser
        115

<210>  53
<211>  107
<212>  PRT
<213>  Artificial Sequence

<220>
<223>  humanized 91D5-HZ1/HZ2 light chain variable region

<400>  53

Asp Ile Gln Leu Thr Gln Ser Pro Ser Ser Leu Ser Ala Ser Val Gly
1               5                   10                  15

Asp Arg Val Thr Ile Thr Cys Arg Pro Ser Gly Asn Val His Asn Tyr
            20                  25                  30

Phe Ala Trp Tyr Gln Gln Lys Pro Gly Lys Ser Pro Lys Leu Leu Val
        35                  40                  45

Tyr Asn Ala Lys Thr Leu Ala Glu Gly Val Pro Ser Arg Phe Ser Gly
    50                  55                  60

Ser Gly Ser Gly Thr Asp Tyr Thr Leu Thr Ile Ser Ser Leu Gln Pro
65                  70                  75                  80

Glu Asp Phe Ala Thr Tyr Tyr Cys His His Tyr Tyr Thr Ala Pro Phe
                85                  90                  95

Thr Phe Gly Gly Gly Thr Lys Val Glu Ile Lys
            100                 105


<210>  54
<211>  118
<212>  PRT
<213>  Mus musculus

<400>  54

Glu Val Gln Leu Val Glu Ser Gly Gly Gly Leu Val Lys Pro Gly Gly
1               5                   10                  15

Ser Leu Lys Leu Ser Cys Ala Ala Ser Gly Phe Thr Phe Ser Ser Tyr
            20                  25                  30

Gly Met Ser Trp Gly Arg Gln Thr Pro Glu Lys Arg Leu Glu Trp Val
        35                  40                  45

Ala Thr Ile Ser Gly Gly Gly Ser Asp Thr Tyr Tyr Pro Asp Ser Val
    50                  55                  60

Lys Gly Arg Phe Thr Ile Ser Arg Asp Asn Val Lys Asn Asn Leu Tyr
65                  70                  75                  80

Leu Gln Met Ser Ser Leu Arg Ser Glu Asp Thr Ala Leu Tyr Tyr Cys
                85                  90                  95

Ala Arg Gln Tyr Gly Ile Thr Trp Phe Ala Tyr Trp Gly Gln Gly Thr
            100                 105                 110

Leu Val Thr Val Ser Ala
        115

<210>  55
<211>  111
<212>  PRT
<213>  Mus musculus

<400>  55

Cys Ile Val Leu Thr Gln Ser Pro Ala Ser Leu Thr Val Ser Leu Gly
1               5                   10                  15

Gln Arg Ala Thr Ile Ser Cys Arg Ala Ser Glu Ser Val Asp Asp Ser
            20                  25                  30

Gly Ile Ser Phe Met His Trp Phe Gln Gln Lys Pro Gly Gln Pro Pro
        35                  40                  45

Lys Leu Leu Ile Tyr Ala Ala Ser Asn Leu Gly Ser Gly Val Pro Ala
    50                  55                  60

Arg Phe Ser Gly Ser Gly Ser Gly Thr Asp Phe Ser Leu Asn Ile His
65                  70                  75                  80

Pro Met Glu Glu Asp Asp Pro Ala Met Tyr Phe Cys Gln Gln Ser Lys
                85                  90                  95

Glu Val Pro Trp Thr Phe Gly Gly Gly Thr Lys Leu Glu Ile Lys
            100                 105                 110

<210>  56
<211>  8
<212>  PRT
<213>  Mus musculus

<400>  56

Gly Phe Thr Phe Ser Ser Tyr Gly
1               5

<210>  57
<211>  8
<212>  PRT
<213>  Mus musculus

<400>  57

Ile Ser Gly Gly Gly Ser Asp Thr
1               5

<210>  58
<211>  11
<212>  PRT
<213>  Mus musculus

<400>  58

Ala Arg Gln Tyr Gly Ile Thr Trp Phe Ala Tyr
1               5                   10


<210>  59
<211>  10
<212>  PRT
<213>  Mus musculus

<400>  59

Glu Ser Val Asp Asp Ser Gly Ile Ser Phe
1               5                   10


<210>  60
<211>  3
<212>  PRT
<213>  Mus musculus

<400>  60

Ala Ala Ser
1


<210>  61
<211>  9
<212>  PRT
<213>  Mus musculus

<400>  61

Gln Gln Ser Lys Glu Val Pro Trp Thr
1               5


<210>  62
<211>  5
<212>  PRT
<213>  Mus musculus

<400>  62

Ser Tyr Gly Met Ser
1               5


<210>  63
<211>  17
<212>  PRT
<213>  Mus musculus

<400>  63

Thr Ile Ser Gly Gly Gly Ser Asp Thr Tyr Tyr Pro Asp Ser Val Lys

1               5                   10                  15

Gly

<210>  64
<211>  9
<212>  PRT
<213>  Mus musculus

<400>  64

Gln Tyr Gly Ile Thr Trp Phe Ala Tyr
1               5

<210>  65
<211>  21
<212>  PRT
<213>  Mus musculus

<400>  65

Arg Ala Thr Ile Ser Cys Arg Ala Ser Glu Ser Val Asp Asp Ser Gly
1               5                   10                  15

Ile Ser Phe Met His
            20

<210>  66
<211>  8
<212>  PRT
<213>  Mus musculus

<400>  66

Tyr Ala Ala Ser Asn Leu Gly Ser
1               5

<210>  67
<211>  9
<212>  PRT
<213>  Mus musculus

<400>  67

Gln Gln Ser Lys Glu Val Pro Trp Thr
1               5

<210>  68
<211>  118
<212>  PRT
<213>  Mus musculus

<400>  68

Gln Val Gln Leu Lys Glu Ser Gly Ala Glu Leu Ala Lys Pro Gly Ala

1               5                   10                  15

Ser Val Lys Met Ser Cys Glu Ala Ser Gly Tyr Ala Leu Thr Ser Tyr
            20                  25                  30

Trp Met His Trp Val Lys Gln Gly Pro Gly Gln Gly Leu Glu Trp Ile
        35                  40                  45

Gly Tyr Ile His Pro Ser Thr Gly Tyr Thr Glu Tyr Asn Gln Ile Phe
    50                  55                  60

Glu Asp Lys Ala Thr Leu Ser Ala Asp Lys Ser Ser Asn Thr Ala Tyr
65                  70                  75                  80

Met Gln Leu Thr Ser Leu Thr Ser Asp Asp Ser Ala Val Tyr Tyr Cys
                85                  90                  95

Ala Arg Gly Ala Gly Pro Tyr Tyr Phe Asp Tyr Trp Gly Gln Gly Thr
            100                 105                 110

Thr Leu Thr Val Ser Ser
        115

<210>  69
<211>  112
<212>  PRT
<213>  Mus musculus

<400>  69

Asp Ile Gln Leu Thr Gln Ser Pro Ser Ser Leu Ala Val Ser Ala Gly
1               5                   10                  15

Glu Lys Val Ile Met Ser Cys Lys Ser Ser Gln Ser Leu Leu Asn Ser
            20                  25                  30

Gly Thr Arg Lys Asn Tyr Leu Ala Trp Tyr Gln Gln Lys Pro Gly Gln
        35                  40                  45

Ser Pro Lys Leu Leu Ile Phe Trp Ala Ser Thr Arg Glu Ser Gly Val
    50                  55                  60

Pro Asp Arg Phe Thr Gly Ser Gly Ser Gly Thr Asp Phe Thr Leu Thr
65                  70                  75                  80

Ile Ser Ser Phe Gln Ala Glu Asp Leu Ala Val Tyr Tyr Cys Lys Gln
                85                  90                  95

Ser Tyr Asp Leu Ile Thr Phe Gly Pro Gly Thr Lys Leu Glu Ile Lys

            100                 105                 110

<210> 70
<211> 8
<212> PRT
<213> Mus musculus

<400> 70

Gly Tyr Ala Leu Thr Ser Tyr Trp
1               5

<210> 71
<211> 8
<212> PRT
<213> Mus musculus

<400> 71

Ile His Pro Ser Thr Gly Tyr Thr
1               5

<210> 72
<211> 11
<212> PRT
<213> Mus musculus

<400> 72

Ala Arg Gly Ala Gly Pro Tyr Tyr Phe Asp Tyr
1               5                   10

<210> 73
<211> 12
<212> PRT
<213> Mus musculus

<400> 73

Gln Ser Leu Leu Asn Ser Gly Thr Arg Lys Asn Tyr
1               5                   10

<210> 74
<211> 3
<212> PRT
<213> Mus musculus

<400> 74

Trp Ala Ser
1

<210> 75
<211> 8
<212> PRT
<213> Mus musculus

<400>  75

Lys Gln Ser Tyr Asp Leu Ile Thr
1               5

<210>  76
<211>  5
<212>  PRT
<213>  Mus musculus

<400>  76

Ser Tyr Trp Met His
1               5

<210>  77
<211>  17
<212>  PRT
<213>  Mus musculus

<400>  77

Tyr Ile His Pro Ser Thr Gly Tyr Thr Glu Tyr Asn Gln Ile Phe Glu
1               5                   10                  15

Asp

<210>  78
<211>  9
<212>  PRT
<213>  Mus musculus

<400>  78

Gly Ala Gly Pro Tyr Tyr Phe Asp Tyr
1               5

<210>  79
<211>  17
<212>  PRT
<213>  Mus musculus

<400>  79

Lys Ser Ser Gln Ser Leu Leu Asn Ser Gly Thr Arg Lys Asn Tyr Leu
1               5                   10                  15

Ala

<210>  80
<211>  7
<212>  PRT
<213>  Mus musculus

<400>  80

Trp Ala Ser Thr Arg Glu Ser
1               5


<210>  81
<211>  8
<212>  PRT
<213>  Mus musculus

<400>  81

Lys Gln Ser Tyr Asp Leu Ile Thr
1               5


<210>  82
<211>  327
<212>  PRT
<213>  Artificial Sequence

<220>
<223>  IgG4(S228P) mutant constant region

<400>  82

Ala Ser Thr Lys Gly Pro Ser Val Phe Pro Leu Ala Pro Cys Ser Arg
1               5                   10                  15

Ser Thr Ser Glu Ser Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Lys Thr
65                  70                  75                  80

Tyr Thr Cys Asn Val Asp His Lys Pro Ser Asn Thr Lys Val Asp Lys
                85                  90                  95

Arg Val Glu Ser Lys Tyr Gly Pro Pro Cys Pro Pro Cys Pro Ala Pro
            100                 105                 110

Glu Phe Leu Gly Gly Pro Ser Val Phe Leu Phe Pro Pro Lys Pro Lys
        115                 120                 125

Asp Thr Leu Met Ile Ser Arg Thr Pro Glu Val Thr Cys Val Val Val
    130                 135                 140

Asp Val Ser Gln Glu Asp Pro Glu Val Gln Phe Asn Trp Tyr Val Asp
145                 150                 155                 160

Gly Val Glu Val His Asn Ala Lys Thr Lys Pro Arg Glu Glu Gln Phe
                165                 170                 175

Asn Ser Thr Tyr Arg Val Val Ser Val Leu Thr Val Leu His Gln Asp
            180                 185                 190

Trp Leu Asn Gly Lys Glu Tyr Lys Cys Lys Val Ser Asn Lys Gly Leu
        195                 200                 205

Pro Ser Ser Ile Glu Lys Thr Ile Ser Lys Ala Lys Gly Gln Pro Arg
    210                 215                 220

Glu Pro Gln Val Tyr Thr Leu Pro Pro Ser Gln Glu Glu Met Thr Lys
225                 230                 235                 240

Asn Gln Val Ser Leu Thr Cys Leu Val Lys Gly Phe Tyr Pro Ser Asp
                245                 250                 255

Ile Ala Val Glu Trp Glu Ser Asn Gly Gln Pro Glu Asn Asn Tyr Lys
            260                 265                 270

Thr Thr Pro Pro Val Leu Asp Ser Asp Gly Ser Phe Phe Leu Tyr Ser
        275                 280                 285

Arg Leu Thr Val Asp Lys Ser Arg Trp Gln Glu Gly Asn Val Phe Ser
    290                 295                 300

Cys Ser Val Met His Glu Ala Leu His Asn His Tyr Thr Gln Lys Ser
305                 310                 315                 320

Leu Ser Leu Ser Leu Gly Lys
                325

<210>  83
<211>  330
<212>  PRT
<213>  Artificial Sequence

<220>
<223>  Human IgG1 heavy chain constant region (mutant)

<400>  83

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr

20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Arg Val Glu Pro Lys Ser Cys Asp Lys Thr His Thr Cys Pro Pro Cys
            100                 105                 110

Pro Ala Pro Glu Ala Ala Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Ala Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Glu Glu
225                 230                 235                 240

Met Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Ala His Tyr Thr
305                 310                 315                 320

Gln Lys Ser Leu Ser Leu Ser Pro Gly Lys
                325                 330

<210>  84
<211>  107
<212>  PRT
<213>  Homo sapiens

<400>  84

<210>  85
<211>  1341
<212>  DNA
<213>  Artificial Sequence

<220>
<223>  91D5-HZ2-IgG1-1D heavy chain

<400>  85
gaggtgcagc tggtgcagag cggagctgag gtgaagaagc caggagcttc tgtgaaggtg        60

tcctgtaagg ccagcggcta caccttcaca gactactata tgaactgggt gagggaggct       120

cctggacagg gactggagtg gatgggagag atcaacccaa agtacggcga taccacatat 180

aatcagaagt ttaaggaccg ggccaccctg acagtggata agagcacctc tacagcttat 240

atggagctgt ccagcctgag atctgacgat accgccgtgt actattgcgc taagggcatc 300

cgcctgttcg actcctgggg acagggcacc ctggtgacag tgtcttccgc ctctacaaag 360

ggcccttccg tgtttccact ggctcccagc tctaagtcca ccagcggagg aacagccgct 420

ctgggctgtc tggtgaagga ttacttccca gagcccgtga ccgtgagctg gaactctggc 480

gccctgacct ccggagtgca cacatttcct gctgtgctgc agtccagcgg cctgtacagc 540

ctgtcttccg tggtgaccgt gccaagctct tccctgggca cccagacata tatctgcaac 600

gtgaatcaca agccatccaa tacaaaggtg gacaagagag tggagcccaa gagctgtgat 660

aagacccata catgcccccc ttgtcctgct ccagaggctg ctggaggacc atccgtgttc 720

ctgtttccac ccaagcctaa ggacaccctg atgatctctc gcaccccaga ggtgacatgc 780

gtggtggtgg ccgtgtccca cgaggacccc gaggtgaagt tcaactggta cgtggatggc 840

gtggaggtgc ataatgctaa gaccaagcca agggaggagc agtacaacag cacctatcgg 900

gtggtgtctg tgctgacagt gctgcaccag gattggctga acggcaagga gtataagtgc 960

aaggtgagca ataaggccct gcccgctcct atcgagaaga ccatctctaa ggccaagggc 1020

cagcctagag agccacaggt gtacacactg cctccaagcc gcgaggagat gaccaagaac 1080

caggtgtctc tgacatgtct ggtgaagggc ttctatccct ctgacatcgc tgtggagtgg 1140

gagtccaatg gccagcctga gaacaattac aagaccacac cccctgtgct ggactccgat 1200

ggcagcttct ttctgtattc caagctgacc gtggataaga gcaggtggca gcagggcaac 1260

gtgttctcct gttccgtgat gcatgaggcc ctgcacgctc attacacaca gaagagcctg 1320

tctctgtccc ctggcaagtg a 1341

<210> 86
<211> 446
<212> PRT
<213> Artificial Sequence

<220>
<223> 91D5-HZ2-IgG1-1D heavy chain

<400> 86

Glu Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asp Tyr
            20                  25                  30

Tyr Met Asn Trp Val Arg Glu Ala Pro Gly Gln Gly Leu Glu Trp Met

35                  40                  45

Gly Glu Ile Asn Pro Lys Tyr Gly Asp Thr Thr Tyr Asn Gln Lys Phe
    50                  55                  60

Lys Asp Arg Ala Thr Leu Thr Val Asp Lys Ser Thr Ser Thr Ala Tyr
65                  70                  75                  80

Met Glu Leu Ser Ser Leu Arg Ser Asp Asp Thr Ala Val Tyr Tyr Cys
                85                  90                  95

Ala Lys Gly Ile Arg Leu Phe Asp Ser Trp Gly Gln Gly Thr Leu Val
            100                 105                 110

Thr Val Ser Ser Ala Ser Thr Lys Gly Pro Ser Val Phe Pro Leu Ala
        115                 120                 125

Pro Ser Ser Lys Ser Thr Ser Gly Gly Thr Ala Ala Leu Gly Cys Leu
    130                 135                 140

Val Lys Asp Tyr Phe Pro Glu Pro Val Thr Val Ser Trp Asn Ser Gly
145                 150                 155                 160

Ala Leu Thr Ser Gly Val His Thr Phe Pro Ala Val Leu Gln Ser Ser
                165                 170                 175

Gly Leu Tyr Ser Leu Ser Ser Val Val Thr Val Pro Ser Ser Ser Leu
            180                 185                 190

Gly Thr Gln Thr Tyr Ile Cys Asn Val Asn His Lys Pro Ser Asn Thr
        195                 200                 205

Lys Val Asp Lys Arg Val Glu Pro Lys Ser Cys Asp Lys Thr His Thr
    210                 215                 220

Cys Pro Pro Cys Pro Ala Pro Glu Ala Ala Gly Gly Pro Ser Val Phe
225                 230                 235                 240

Leu Phe Pro Pro Lys Pro Lys Asp Thr Leu Met Ile Ser Arg Thr Pro
                245                 250                 255

Glu Val Thr Cys Val Val Val Ala Val Ser His Glu Asp Pro Glu Val
            260                 265                 270

Lys Phe Asn Trp Tyr Val Asp Gly Val Glu Val His Asn Ala Lys Thr
        275                 280                 285

Lys Pro Arg Glu Glu Gln Tyr Asn Ser Thr Tyr Arg Val Val Ser Val
    290                 295                 300

Leu Thr Val Leu His Gln Asp Trp Leu Asn Gly Lys Glu Tyr Lys Cys
305                 310                 315                 320

Lys Val Ser Asn Lys Ala Leu Pro Ala Pro Ile Glu Lys Thr Ile Ser
                325                 330                 335

Lys Ala Lys Gly Gln Pro Arg Glu Pro Gln Val Tyr Thr Leu Pro Pro
            340                 345                 350

Ser Arg Glu Glu Met Thr Lys Asn Gln Val Ser Leu Thr Cys Leu Val
        355                 360                 365

Lys Gly Phe Tyr Pro Ser Asp Ile Ala Val Glu Trp Glu Ser Asn Gly
    370                 375                 380

Gln Pro Glu Asn Asn Tyr Lys Thr Thr Pro Pro Val Leu Asp Ser Asp
385                 390                 395                 400

Gly Ser Phe Phe Leu Tyr Ser Lys Leu Thr Val Asp Lys Ser Arg Trp
                405                 410                 415

Gln Gln Gly Asn Val Phe Ser Cys Ser Val Met His Glu Ala Leu His
            420                 425                 430

Ala His Tyr Thr Gln Lys Ser Leu Ser Leu Ser Pro Gly Lys
        435                 440                 445

<210>  87
<211>  645
<212>  DNA
<213>  Artificial Sequence

<220>
<223>  91D5-HZ2-IgG1-1D light chain

<400>  87
gacatccagc tgacacagtc cccatccagc ctgtctgcct ccgtgggcga tagggtgacc      60

atcacatgtc ggccttctgg caacgtgcac aattacttcg cttggtatca gcagaagcct     120

ggcaagtccc ccaagctgct ggtgtacaac gccaagaccc tggctgaggg cgtgcctagc     180

agattcagcg gctctggctc cggcacagac tataccctga caatctcttc cctgcagcct     240

gaggattttg ccacctacta ttgccaccat tactataccg ctccattcac atttggcggc     300

ggcaccaagg tggagatcaa gaggacagtg gccgctccat ccgtgttcat ctttccccct     360

agcgacgagc agctgaagag cggcaccgcc tctgtggtgt gcctgctgaa caatttctac     420

ccccgggagg ccaaggtgca gtggaaggtg gataacgctc tgcagagcgg caattctcag 480

gagtccgtga ccgagcagga cagcaaggat tctacatatt ccctgagctc taccctgaca 540

ctgtctaagg ccgactacga gaagcacaag gtgtatgctt gcgaggtgac ccatcagggc 600

ctgtccagcc cagtgacaaa gtcctttaat cgcggcgagt gttga 645

<210> 88
<211> 214
<212> PRT
<213> Artificial Sequence

<220>
<223> 91D5-HZ2-IgG1-1D light chain

<400> 88

Asp Ile Gln Leu Thr Gln Ser Pro Ser Ser Leu Ser Ala Ser Val Gly
1               5                   10                  15

Asp Arg Val Thr Ile Thr Cys Arg Pro Ser Gly Asn Val His Asn Tyr
            20                  25                  30

Phe Ala Trp Tyr Gln Gln Lys Pro Gly Lys Ser Pro Lys Leu Leu Val
        35                  40                  45

Tyr Asn Ala Lys Thr Leu Ala Glu Gly Val Pro Ser Arg Phe Ser Gly
    50                  55                  60

Ser Gly Ser Gly Thr Asp Tyr Thr Leu Thr Ile Ser Ser Leu Gln Pro
65                  70                  75                  80

Glu Asp Phe Ala Thr Tyr Tyr Cys His His Tyr Tyr Thr Ala Pro Phe
                85                  90                  95

Thr Phe Gly Gly Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  89
<211>  1332
<212>  DNA
<213>  Artificial Sequence

<220>
<223>  35E6-HZ1-IgG1-1D heavy chain

<400>  89
caggtgcagc tgcaggagtc cggaccagga ctggtgaagc catctgagac cctgtccctg      60

acctgtacag tgtctggcta ctccatcaca agcgactatt ggagctggat cagacagcca     120

cctggcaagg gactggagtg gatcggctac atcaactatg acggcgataa caattacaat     180

ccatctctga agtccagggt gaccatcagc cgggatacat ctaagaacca gttctctctg     240

aagctgtcca gcgtgaccgc cgctgacaca gccgtgtact attgcgctcg cggcggcttt     300

gattattggg gccagggcac cctggtgaca gtgtcttccg cctccaccaa gggaccaagc     360

gtgttccctc tggctccaag ctctaagagc acctctggag gaacagccgc tctgggctgt     420

ctggtgaagg actacttccc tgagccagtg accgtgagct ggaactctgg cgccctgacc     480

agcggagtgc acacatttcc cgctgtgctg cagtccagcg gcctgtactc tctgtcttcc     540

gtggtgaccg tgcctagctc ttccctgggc acccagacat atatctgcaa cgtgaatcac     600

aagcccagca atacaaaggt ggacaagagg gtggagccta agtcttgtga taagacccat     660

acatgcccac catgtccagc tcctgaggct gctggaggac cttccgtgtt cctgtttcct     720

ccaaagccaa aggacaccct gatgatctcc cggacccctg aggtgacatg cgtggtggtg     780

gccgtgagcc acgaggaccc agaggtgaag tttaactggt acgtggatgg cgtggaggtg     840

cataatgcta agaccaagcc tagagaggag cagtacaact ctacctatcg cgtggtgtcc     900

gtgctgacag tgctgcacca ggattggctg aacggcaagg agtataagtg caaggtgtct     960

aataaggccc tgccagctcc catcgagaag accatctcca aggctaaggg acagcccagg    1020

gagcctcagg tgtacacact gcccccttct cgggaggaga tgaccaagaa ccaggtgtcc    1080

ctgacatgtc tggtgaaggg cttttatcca tccgacatcg ctgtggagtg ggagagcaat    1140

ggccagcccg agaacaatta caagaccaca ccacccgtgc tggactccga tggcagcttc    1200

tttctgtata gcaagctgac cgtggataag tctaggtggc agcagggcaa cgtgttctcc    1260

tgtagcgtga tgcatgaggc cctgcacgct cattacacac agaagtctct gtccctgagc 1320

cctggcaagt ga 1332

<210> 90
<211> 443
<212> PRT
<213> Artificial Sequence

<220>
<223> 35E6-HZ1-IgG1-1D heavy chain

<400> 90

Gln Val Gln Leu Gln Glu Ser Gly Pro Gly Leu Val Lys Pro Ser Glu
1               5                   10                  15

Thr Leu Ser Leu Thr Cys Thr Val Ser Gly Tyr Ser Ile Thr Ser Asp
            20                  25                  30

Tyr Trp Ser Trp Ile Arg Gln Pro Pro Gly Lys Gly Leu Glu Trp Ile
        35                  40                  45

Gly Tyr Ile Asn Tyr Asp Gly Asp Asn Asn Tyr Asn Pro Ser Leu Lys
    50                  55                  60

Ser Arg Val Thr Ile Ser Arg Asp Thr Ser Lys Asn Gln Phe Ser Leu
65                  70                  75                  80

Lys Leu Ser Ser Val Thr Ala Ala Asp Thr Ala Val Tyr Tyr Cys Ala
                85                  90                  95

Arg Gly Gly Phe Asp Tyr Trp Gly Gln Gly Thr Leu Val Thr Val Ser
            100                 105                 110

Ser Ala Ser Thr Lys Gly Pro Ser Val Phe Pro Leu Ala Pro Ser Ser
        115                 120                 125

Lys Ser Thr Ser Gly Gly Thr Ala Ala Leu Gly Cys Leu Val Lys Asp
    130                 135                 140

Tyr Phe Pro Glu Pro Val Thr Val Ser Trp Asn Ser Gly Ala Leu Thr
145                 150                 155                 160

Ser Gly Val His Thr Phe Pro Ala Val Leu Gln Ser Ser Gly Leu Tyr
                165                 170                 175

Ser Leu Ser Ser Val Val Thr Val Pro Ser Ser Ser Leu Gly Thr Gln
            180                 185                 190

Thr Tyr Ile Cys Asn Val Asn His Lys Pro Ser Asn Thr Lys Val Asp
        195                 200                 205

Lys Arg Val Glu Pro Lys Ser Cys Asp Lys Thr His Thr Cys Pro Pro
    210                 215                 220

Cys Pro Ala Pro Glu Ala Ala Gly Gly Pro Ser Val Phe Leu Phe Pro
225                 230                 235                 240

Pro Lys Pro Lys Asp Thr Leu Met Ile Ser Arg Thr Pro Glu Val Thr
                245                 250                 255

Cys Val Val Val Ala Val Ser His Glu Asp Pro Glu Val Lys Phe Asn
            260                 265                 270

Trp Tyr Val Asp Gly Val Glu Val His Asn Ala Lys Thr Lys Pro Arg
        275                 280                 285

Glu Glu Gln Tyr Asn Ser Thr Tyr Arg Val Val Ser Val Leu Thr Val
    290                 295                 300

Leu His Gln Asp Trp Leu Asn Gly Lys Glu Tyr Lys Cys Lys Val Ser
305                 310                 315                 320

Asn Lys Ala Leu Pro Ala Pro Ile Glu Lys Thr Ile Ser Lys Ala Lys
                325                 330                 335

Gly Gln Pro Arg Glu Pro Gln Val Tyr Thr Leu Pro Pro Ser Arg Glu
            340                 345                 350

Glu Met Thr Lys Asn Gln Val Ser Leu Thr Cys Leu Val Lys Gly Phe
        355                 360                 365

Tyr Pro Ser Asp Ile Ala Val Glu Trp Glu Ser Asn Gly Gln Pro Glu
    370                 375                 380

Asn Asn Tyr Lys Thr Thr Pro Pro Val Leu Asp Ser Asp Gly Ser Phe
385                 390                 395                 400

Phe Leu Tyr Ser Lys Leu Thr Val Asp Lys Ser Arg Trp Gln Gln Gly
                405                 410                 415

Asn Val Phe Ser Cys Ser Val Met His Glu Ala Leu His Ala His Tyr
            420                 425                 430

Thr Gln Lys Ser Leu Ser Leu Ser Pro Gly Lys
        435                 440

## Description

### Technical Field

The invention relates to the field of therapeutic monoclonal antibodies, and more particularly, to an antibody against PD-1; and to use of the antibody in preventing and/or treating a tumor, an infection, or an autoimmune disease.

### Background Art

Programmed cell death receptor-1 (PD-1) is a type I membrane protein having 288 amino acids, which is known as one of the main immune checkpoints (Blank et al., 2005, Cancer Immunotherapy, 54: 307-314). The PD-1/PD-L1 signaling pathway plays an important role in regulating immune tolerance, microbial infection, and tumor immune escape. Blocking the interaction between PD-1 and one of its ligands, PD-L1, improves the immunity of tumor-specific CD8 ⁺ T cells, thereby attacking cancer cells, thus helping the immune system to eliminate tumor cells. Blocking PD-1/PD-L1 signaling can promote proliferation of tumor antigen-specific T cells, play a role in killing tumor cells, thereby inhibiting local tumor growth (Julie R et al., 2012, N Engl J Med. 366: 2455-2465); PD-1 and relevant research thereof were reviewed in detail in Molecular and Biochemical Aspects of the PD-1 Checkpoint Pathway, Vassiliki A. Boussiotis, NEJM n engl j med 375; 18 nejm.org November 3, 2016. The expression of PD-1 (on tumor infiltrating lymphocytes) and/or PD-L1 on tumor cells has been found on a variety of human primary tumors by immunohistochemical evaluation of biopsies. Such tissues include lung cancer, liver cancer, ovarian cancer, cervical cancer, skin cancer, colon cancer, rectal cancer, glioma, bladder cancer, breast cancer, kidney cancer, esophageal cancer, gastric cancer, oral squamous cell cancer, urethral epithelial cell cancer, pancreatic cancer and head and neck tumors, etc. It can be seen from that blocking the interaction of PD-1/PD-L1 may improve the immunocompetence of tumor-specific T cells, helping the immune system to eliminate tumor cells. Recently, studies have shown that PD-1 is highly expressed in T cells from HIV-infected individuals, additionally, an anti-PD-1 antibody can also enhance immunity to chronic viral infection. Moreover, Zhao et al. have found that depletion of PD-1-positive expressing immune cells in mice can delay the onset of a diabetic disease model and ameliorate the paralysis caused by experimental autoimmune encephalomyelitis (Zhao P. Et al. Depletion of PD-1-positive cells ameliorates autoimmune disease. Nature Biomedical Engineering 3, 292-305, 2019). Therefore, blockade of PD-1/PD-L1 may be useful in preventing and/or treating a tumor, an infection, or an autoimmune disease.

An anti-PD-1 monoclonal antibody Pembrolizumab (Keytruda, Merck) can reduce the risk of death by 31% compared to chemotherapy in PD-L1 expression positive patients with esophageal cancer, however, Pembrolizumab causes not only nausea and vomiting, but also anemia, hyperglycemia, hypoalbuminemia, hyponatremia, pain in extremity, dyspnea, motor skills disorder, and other side effects. Recurrent or refractory Hodgkin's lymphoma responds to sintilimab, an anti-PD-1 monoclonal antibody, however, 18% of patients have an adverse event of Grade 3 or 4, and 15% of patients have severe adverse events. A Phase I clinical trial of the treatment of anti-PD-1 monoclonal antibody Tislelizumab in combination with Pamiparib showed that some patients developed hepatitis or autoimmune hepatitis, and the efficacy of Tislelizumab in urethral cancer and liver cancer is still investigated in clinical trials. Therefore, the research and development of a new anti-PD-1 monoclonal antibody has become an urgent technical problem to be solved.

### Summary of the Invention

In the present application, firstly, the inventors have developed murine antibodies with excellent properties which is capable of specifically recognizing PD-1. Based on that, the inventors have further developed humanized antibodies of the murine antibodies by expensing creative works on deep study and modification of the murine antibodies.

The antibodies according to the invention, especially the humanized antibodies, are extremely advantageous, which not only retains (or even enhances) the functions and properties of the parental murine antibody, such as binding to PD-1 with high affinity and specificity, but also have an extremely high extent of humanization and can be safely administered to a human subject without triggering an immunogenic reaction. Therefore, the antibodies according to the invention are of great clinical value.

### Antibody of the invention

In one aspect, the invention provides an antibody or antigen-binding fragment thereof capable of specifically binding to PD-1, the antibody or antigen-binding fragment thereof comprising the following complementary determining regions (CDRs):
(a) a CDR-H1 contained in a heavy chain variable region (VH) as set forth in SEQ ID NO: 1 or a variant thereof having a substitution, deletion or addition of one or several amino acids (for example, a substitution, deletion or addition of 1, 2 or 3 amino acids) as compared to the sequence of the CDR-H1, a CDR-H2 contained in a heavy chain variable region (VH) as set forth in SEQ ID NO: 1 or a variant thereof having a substitution, deletion or addition of one or several amino acids (for example, a substitution, deletion or addition of 1, 2 or 3 amino acids) as compared to the sequence of the CDR-H2, and a CDR-H3 contained in a heavy chain variable region (VH) as set forth in SEQ ID NO: 1 or a variant thereof having a substitution, deletion or addition of one or several amino acids (for example, a substitution, deletion or addition of 1, 2 or 3 amino acids) as compared to the sequence of the CDR-H3; and/or
   a CDR-L1 contained in a light chain variable region (VL) as set forth in SEQ ID NO: 2 or a variant thereof having a substitution, deletion or addition of one or several amino acids (for example, a substitution, deletion or addition of 1, 2 or 3 amino acids) as compared to the sequence of the CDR-L1, a CDR-L2 contained in a light chain variable region (VL) as set forth in SEQ ID NO: 2 or a variant thereof having a substitution, deletion or addition of one or several amino acids (for example, a substitution, deletion or addition of 1, 2 or 3 amino acids) as compared to the sequence of the CDR-L2, and a CDR-L3 contained in a light chain variable region (VL) as set forth in SEQ ID NO: 2 or a variant thereof having a substitution, deletion or addition of one or several amino acids (for example, a substitution, deletion or addition of 1, 2 or 3 amino acids) as compared to the sequence of the CDR-L3;
(b) a CDR-H1 contained in a VH as set forth in SEQ ID NO: 20 or a variant thereof having a substitution, deletion or addition of one or several amino acids (for example, a substitution, deletion or addition of 1, 2 or 3 amino acids) as compared to the sequence of the CDR-H1, a CDR-H2 contained in a VH as set forth in SEQ ID NO: 20 or a variant thereof having a substitution, deletion or addition of one or several amino acids (for example, a substitution, deletion or addition of 1, 2 or 3 amino acids) as compared to the sequence of the CDR-H2, and a CDR-H3 contained in a VH as set forth in SEQ ID NO: 20 or a variant thereof having a substitution, deletion or addition of one or several amino acids (for example, a substitution, deletion or addition of 1, 2 or 3 amino acids) as compared to the sequence of the CDR-H3; and/or
   a CDR-L1 contained in a VL as set forth in SEQ ID NO: 21 or a variant thereof having a substitution, deletion or addition of one or several amino acids (for example, a substitution, deletion or addition of 1, 2 or 3 amino acids) as compared to the sequence of the CDR-L1, a CDR-L2 contained in a VL as set forth in SEQ ID NO: 21 or a variant thereof having a substitution, deletion or addition of one or several amino acids (for example, a substitution, deletion or addition of 1, 2 or 3 amino acids) as compared to the sequence of the CDR-L2, and a CDR-L3 contained in a VL as set forth in SEQ ID NO: 21 or a variant thereof having a substitution, deletion or addition of one or several amino acids (for example, a substitution, deletion or addition of 1, 2 or 3 amino acids) as compared to the sequence of the CDR-L3;
(c) a CDR-H1 contained in a VH as set forth in SEQ ID NO: 36 or a variant thereof having a substitution, deletion or addition of one or several amino acids (for example, a substitution, deletion or addition of 1, 2 or 3 amino acids) as compared to the sequence of the CDR-H1, a CDR-H2 contained in a VH as set forth in SEQ ID NO: 36 or a variant thereof having a substitution, deletion or addition of one or several amino acids (for example, a substitution, deletion or addition of 1, 2 or 3 amino acids) as compared to the sequence of the CDR-H2, and a CDR-H3 contained in a VH as set forth in SEQ ID NO: 36 or a variant thereof having a substitution, deletion or addition of one or several amino acids (for example, a substitution, deletion or addition of 1, 2 or 3 amino acids) as compared to the sequence of the CDR-H3; and/or
   a CDR-L1 contained in a VL as set forth in SEQ ID NO: 37 or a variant thereof having a substitution, deletion or addition of one or several amino acids (for example, a substitution, deletion or addition of 1, 2 or 3 amino acids) as compared to the sequence of the CDR-L1, a CDR-L2 contained in a VL as set forth in SEQ ID NO: 37 or a variant thereof having a substitution, deletion or addition of one or several amino acids (for example, a substitution, deletion or addition of 1, 2 or 3 amino acids) as compared to the sequence of the CDR-L2, and a CDR-L3 contained in a VL as set forth in SEQ ID NO: 37 or a variant thereof having a substitution, deletion or addition of one or several amino acids (for example, a substitution, deletion or addition of 1, 2 or 3 amino acids) as compared to the sequence of the CDR-L3;
(d) CDR-H1 contained in a VH as set forth in SEQ ID NO: 54 or a variant thereof having a substitution, deletion or addition of one or several amino acids (for example, a substitution, deletion or addition of 1, 2 or 3 amino acids) as compared to the sequence of the CDR-H1, a CDR-H2 contained in a VH as set forth in SEQ ID NO: 54 or a variant thereof having a substitution, deletion or addition of one or several amino acids (for example, a substitution, deletion or addition of 1, 2 or 3 amino acids) as compared to the sequence of the CDR-H2, and a CDR-H3 contained in a VH as set forth in SEQ ID NO: 54 or a variant thereof having a substitution, deletion or addition of one or several amino acids (for example, a substitution, deletion or addition of 1, 2 or 3 amino acids) as compared to the sequence of the CDR-H3; and/or
   a CDR-L1 contained in a VL as set forth in SEQ ID NO: 55 or a variant thereof having a substitution, deletion or addition of one or several amino acids (for example, a substitution, deletion or addition of 1, 2 or 3 amino acids) as compared to the sequence of the CDR-L1, a CDR-L2 contained in a VL as set forth in SEQ ID NO: 55 or a variant thereof having a substitution, deletion or addition of one or several amino acids (for example, a substitution, deletion or addition of 1, 2 or 3 amino acids) as compared to the sequence of the CDR-L2, and a CDR-L3 contained in a VL as set forth in SEQ ID NO: 55 or a variant thereof having a substitution, deletion or addition of one or several amino acids (for example, a substitution, deletion or addition of 1, 2 or 3 amino acids) as compared to the sequence of the CDR-L3;
   or
(e) a CDR-H1 contained in a VH as set forth in SEQ ID NO: 68 or a variant thereof having a substitution, deletion or addition of one or several amino acids (for example, a substitution, deletion or addition of 1, 2 or 3 amino acids) as compared to the sequence of the CDR-H1, a CDR-H2 contained in a VH as set forth in SEQ ID NO: 68 or a variant thereof having a substitution, deletion or addition of one or several amino acids (for example, a substitution, deletion or addition of 1, 2 or 3 amino acids) as compared to the sequence of the CDR-H2, and a CDR-H3 contained in a VH as set forth in SEQ ID NO: 68 or a variant thereof having a substitution, deletion or addition of one or several amino acids (for example, a substitution, deletion or addition of 1, 2 or 3 amino acids) as compared to the sequence of the CDR-H3; and/or
   a CDR-L1 contained in a VL as set forth in SEQ ID NO: 69 or a variant thereof having a substitution, deletion or addition of one or several amino acids (for example, a substitution, deletion or addition of 1, 2 or 3 amino acids) as compared to the sequence of the CDR-L1, a CDR-L2 contained in a VL as set forth in SEQ ID NO: 69 or a variant thereof having a substitution, deletion or addition of one or several amino acids (for example, a substitution, deletion or addition of 1, 2 or 3 amino acids) as compared to the sequence of the CDR-L2, and a CDR-L3 contained in a VL as set forth in SEQ ID NO: 69 or a variant thereof having a substitution, deletion or addition of one or several amino acids (for example, a substitution, deletion or addition of 1, 2 or 3 amino acids) as compared to the sequence of the CDR-L3.

In certain embodiments, the substitution is a conservative substitution.

Preferably, the CDR is defined by the Kabat or IMGT numbering system.

In certain embodiments, the VH and/or VL of the antibody or antigen-binding fragment thereof comprise framework regions (FRs) of an immunoglobulin derived from a human or mouse.

In certain embodiments, the antibody or antigen-binding fragment thereof binds to human PD-1.

In one aspect, the invention provides an antibody or antigen-binding fragment thereof capable of specifically binding to PD-1, the antibody or antigen-binding fragment thereof comprising a heavy chain variable region (VH) and/or a light chain variable region (VL).

In certain embodiments, the antibody or antigen-binding fragment thereof of the invention comprises a heavy chain variable region (VH) and/or a light chain variable region (VL) as follows, wherein the CDRs are defined by the IMGT numbering system:
(a) a heavy chain variable region (VH) comprising three CDRs as follows: CDR-H1 with a sequence as set forth in SEQ ID NO: 3 or a sequence having a substitution, deletion or addition of one or several amino acids (for example, a substitution, deletion or addition of 1, 2 or 3 amino acids) as compared to SEQ ID NO: 3, CDR-H2 with a sequence as set forth in SEQ ID NO: 4 or a sequence having a substitution, deletion or addition of one or several amino acids (for example, a substitution, deletion or addition of 1, 2 or 3 amino acids) as compared to SEQ ID NO: 4, CDR-H3 with a sequence as set forth in SEQ ID NO: 5 or a sequence having a substitution, deletion or addition of one or several amino acids (for example, a substitution, deletion or addition of 1, 2 or 3 amino acids) as compared to SEQ ID NO: 5; and/or,
   a light chain variable region (VL) comprising three CDRs as follows: CDR-L1 with a sequence as set forth in SEQ ID NO: 6 or a sequence having a substitution, deletion or addition of one or several amino acids (for example, a substitution, deletion or addition of 1, 2 or 3 amino acids) as compared to SEQ ID NO: 6, CDR-L2 with a sequence as set forth in SEQ ID NO: 7 or a sequence having a substitution, deletion or addition of one or several amino acids (for example, a substitution, deletion or addition of 1, 2 or 3 amino acids) as compared to SEQ ID NO: 7, CDR-L3 with a sequence as set forth in SEQ ID NO: 8 or a sequence having a substitution, deletion or addition of one or several amino acids (for example, a substitution, deletion or addition of 1, 2 or 3 amino acids) as compared to SEQ ID NO: 8;
(b) a heavy chain variable region (VH) comprising three CDRs as follows: CDR-H1 with a sequence as set forth in SEQ ID NO: 22 or a sequence having a substitution, deletion or addition of one or several amino acids (for example, a substitution, deletion or addition of 1, 2 or 3 amino acids) as compared to SEQ ID NO: 22, CDR-H2 with a sequence as set forth in SEQ ID NO: 23 or a sequence having a substitution, deletion or addition of one or several amino acids (for example, a substitution, deletion or addition of 1, 2 or 3 amino acids) as compared to SEQ ID NO: 23, CDR-H3 with a sequence as set forth in SEQ ID NO: 24 or a sequence having a substitution, deletion or addition of one or several amino acids (for example, a substitution, deletion or addition of 1, 2 or 3 amino acids) as compared to SEQ ID NO: 24; and/or
   a light chain variable region (VL) comprising three CDRs as follows: CDR-L1 with a sequence as set forth in SEQ ID NO: 25 or a sequence having a substitution, deletion or addition of one or several amino acids (for example, a substitution, deletion or addition of 1, 2 or 3 amino acids) as compared to SEQ ID NO: 25, CDR-L2 with a sequence as set forth in SEQ ID NO: 26 or a sequence having a substitution, deletion or addition of one or several amino acids (for example, a substitution, deletion or addition of 1, 2 or 3 amino acids) as compared to SEQ ID NO: 26, CDR-L3 with a sequence as set forth in SEQ ID NO: 27 or a sequence having a substitution, deletion or addition of one or several amino acids (for example, a substitution, deletion or addition of 1, 2 or 3 amino acids) as compared to SEQ ID NO: 27;
(c) a heavy chain variable region (VH) comprising three CDRs as follows: CDR-H1 with a sequence as set forth in SEQ ID NO: 38 or a sequence having a substitution, deletion or addition of one or several amino acids (for example, a substitution, deletion or addition of 1, 2 or 3 amino acids) as compared to SEQ ID NO: 38, CDR-H2 with a sequence as set forth in SEQ ID NO: 39 or a sequence having a substitution, deletion or addition of one or several amino acids (for example, a substitution, deletion or addition of 1, 2 or 3 amino acids) as compared to SEQ ID NO: 39, CDR-H3 with a sequence as set forth in SEQ ID NO: 40 or a sequence having a substitution, deletion or addition of one or several amino acids (for example, a substitution, deletion or addition of 1, 2 or 3 amino acids) as compared to SEQ ID NO: 40; and/or,
   a light chain variable region (VL) comprising three CDRs as follows: CDR-L1 with a sequence as set forth in SEQ ID NO: 41 or a sequence having a substitution, deletion or addition of one or several amino acids (for example, a substitution, deletion or addition of 1, 2 or 3 amino acids) as compared to SEQ ID NO: 41, CDR-L2 with a sequence as set forth in SEQ ID NO: 42 or a sequence having a substitution, deletion or addition of one or several amino acids (for example, a substitution, deletion or addition of 1, 2 or 3 amino acids) as compared to SEQ ID NO: 42, CDR-L3 with a sequence as set forth in SEQ ID NO: 43 or a sequence having a substitution, deletion or addition of one or several amino acids (for example, a substitution, deletion or addition of 1, 2 or 3 amino acids) as compared to SEQ ID NO: 43;
(d) a heavy chain variable region (VH) comprising three CDRs as follows: CDR-H1 with a sequence as set forth in SEQ ID NO: 56 or a sequence having a substitution, deletion or addition of one or several amino acids (for example, a substitution, deletion or addition of 1, 2 or 3 amino acids) as compared to SEQ ID NO: 56, CDR-H2 with a sequence as set forth in SEQ ID NO: 57 or a sequence having a substitution, deletion or addition of one or several amino acids (for example, a substitution, deletion or addition of 1, 2 or 3 amino acids) as compared to SEQ ID NO: 57, CDR-H3 with a sequence as set forth in SEQ ID NO: 58 or a sequence having a substitution, deletion or addition of one or several amino acids (for example, a substitution, deletion or addition of 1, 2 or 3 amino acids) as compared to SEQ ID NO: 58; and/or,
   a light chain variable region (VL) comprising three CDRs as follows: CDR-L1 with a sequence as set forth in SEQ ID NO: 59 or a sequence having a substitution, deletion or addition of one or several amino acids (for example, a substitution, deletion or addition of 1, 2 or 3 amino acids) as compared to SEQ ID NO: 59, CDR-L2 with a sequence as set forth in SEQ ID NO: 60 or a sequence having a substitution, deletion or addition of one or several amino acids (for example, a substitution, deletion or addition of 1, 2 or 3 amino acids) as compared to SEQ ID NO: 60, CDR-L3 with a sequence as set forth in SEQ ID NO: 61 or a sequence having a substitution, deletion or addition of one or several amino acids (for example, a substitution, deletion or addition of 1, 2 or 3 amino acids) as compared to SEQ ID NO: 61;
   or
(e) a heavy chain variable region (VH) comprising three CDRs as follows: CDR-H1 with a sequence as set forth in SEQ ID NO: 70 or a sequence having a substitution, deletion or addition of one or several amino acids (for example, a substitution, deletion or addition of 1, 2 or 3 amino acids) as compared to SEQ ID NO: 70, CDR-H2 with a sequence as set forth in SEQ ID NO: 71 or a sequence having a substitution, deletion or addition of one or several amino acids (for example, a substitution, deletion or addition of 1, 2 or 3 amino acids) as compared to SEQ ID NO: 71, CDR-H3 with a sequence as set forth in SEQ ID NO: 72 or a sequence having a substitution, deletion or addition of one or several amino acids (for example, a substitution, deletion or addition of 1, 2 or 3 amino acids) as compared to SEQ ID NO: 72; and/or,
   a light chain variable region (VL) comprising three CDRs as follows: CDR-L1 with a sequence as set forth in SEQ ID NO: 73 or a sequence having a substitution, deletion or addition of one or several amino acids (for example, a substitution, deletion or addition of 1, 2 or 3 amino acids) as compared to SEQ ID NO: 73, CDR-L2 with a sequence as set forth in SEQ ID NO: 74 or a sequence having a substitution, deletion or addition of one or several amino acids (for example, a substitution, deletion or addition of 1, 2 or 3 amino acids) as compared to SEQ ID NO: 74, CDR-L3 with a sequence as set forth in SEQ ID NO: 75 or a sequence having a substitution, deletion or addition of one or several amino acids (for example, a substitution, deletion or addition of 1, 2 or 3 amino acids) as compared to SEQ ID NO: 75.

In certain embodiments, the antibody or antigen-binding fragment thereof of the invention comprises a heavy chain variable region (VH) and/or a light chain variable region (VL) as follows, wherein the CDRs are defined by the IMGT numbering system:
(a) a heavy chain variable region (VH) comprising three CDRs as follows: CDR-H1 with a sequence as set forth in SEQ ID NO: 3, CDR-H2 with a sequence as set forth in SEQ ID NO: 4, CDR-H3 with a sequence as set forth in SEQ ID NO: 5; and/or,
   a light chain variable region (VL) comprising three CDRs as follows: CDR-L1 with a sequence as set forth in SEQ ID NO: 6, CDR-L2 with a sequence as set forth in SEQ ID NO: 7, CDR-L3 with a sequence as set forth in SEQ ID NO: 8;
(b) a heavy chain variable region (VH) comprising three CDRs as follows: CDR-H1 with a sequence as set forth in SEQ ID NO: 22, CDR-H2 with a sequence as set forth in SEQ ID NO: 23, CDR-H3 with a sequence as set forth in SEQ ID NO: 24; and/or,
   a light chain variable region (VL) comprising three CDRs as follows: CDR-L1 with a sequence as set forth in SEQ ID NO: 25, CDR-L2 with a sequence as set forth in SEQ ID NO: 26, CDR-L3 with a sequence as set forth in SEQ ID NO: 27;
(c) a heavy chain variable region (VH) comprising three CDRs as follows: CDR-H1 with a sequence as set forth in SEQ ID NO: 38, CDR-H2 with a sequence as set forth in SEQ ID NO: 39, CDR-H3 with a sequence as set forth in SEQ ID NO: 40; and/or,
   a light chain variable region (VL) comprising three CDRs as follows: CDR-L1 with a sequence as set forth in SEQ ID NO: 41, CDR-L2 with a sequence as set forth in SEQ ID NO: 42, CDR-L3 with a sequence as set forth in SEQ ID NO: 43;
(d) a heavy chain variable region (VH) comprising three CDRs as follows: CDR-H1 with a sequence as set forth in SEQ ID NO: 56, CDR-H2 with a sequence as set forth in SEQ ID NO: 57, CDR-H3 with a sequence as set forth in SEQ ID NO: 58; and/or,
   a light chain variable region (VL) comprising three CDRs as follows: CDR-L1 with a sequence as set forth in SEQ ID NO: 59, CDR-L2 with a sequence as set forth in SEQ ID NO: 60, CDR-L3 with a sequence as set forth in SEQ ID NO: 61;
   or
(e) a heavy chain variable region (VH) comprising three CDRs as follows: CDR-H1 with a sequence as set forth in SEQ ID NO: 70, CDR-H2 with a sequence as set forth in SEQ ID NO: 71, CDR-H3 with a sequence as set forth in SEQ ID NO: 72; and/or,
   a light chain variable region (VL) comprising three CDRs as follows: CDR-L1 with a sequence as set forth in SEQ ID NO: 73, CDR-L2 with a sequence as set forth in SEQ ID NO: 74, CDR-L3 with a sequence as set forth in SEQ ID NO: 75.

In certain embodiments, the antibody or antigen-binding fragment thereof of the invention comprises a heavy chain variable region (VH) and/or a light chain variable region (VL) as follows, wherein the CDRs are defined by the Kabat numbering system:
(a) a heavy chain variable region (VH) comprising three CDRs as follows: CDR-H1 with a sequence as set forth in SEQ ID NO: 9 or a sequence having a substitution, deletion or addition of one or several amino acids (for example, a substitution, deletion or addition of 1, 2 or 3 amino acids) as compared to SEQ ID NO: 9, CDR-H2 with a sequence as set forth in SEQ ID NO: 10 or a sequence having a substitution, deletion or addition of one or several amino acids (for example, a substitution, deletion or addition of 1, 2 or 3 amino acids) as compared to SEQ ID NO: 10, CDR-H3 with a sequence as set forth in SEQ ID NO: 12 or a sequence having a substitution, deletion or addition of one or several amino acids (for example, a substitution, deletion or addition of 1, 2 or 3 amino acids) as compared to SEQ ID NO: 12; and/or,
   a light chain variable region (VL) comprising three CDRs as follows: CDR-L1 with a sequence as set forth in SEQ ID NO: 13 or a sequence having a substitution, deletion or addition of one or several amino acids (for example, a substitution, deletion or addition of 1, 2 or 3 amino acids) as compared to SEQ ID NO: 13, CDR-L2 with a sequence as set forth in SEQ ID NO: 14 or a sequence having a substitution, deletion or addition of one or several amino acids (for example, a substitution, deletion or addition of 1, 2 or 3 amino acids) as compared to SEQ ID NO: 14, CDR-L3 with a sequence as set forth in SEQ ID NO: 15 or a sequence having a substitution, deletion or addition of one or several amino acids (for example, a substitution, deletion or addition of 1, 2 or 3 amino acids) as compared to SEQ ID NO: 15;
(b) a heavy chain variable region (VH) comprising three CDRs as follows: CDR-H1 with a sequence as set forth in SEQ ID NO: 28 or a sequence having a substitution, deletion or addition of one or several amino acids (for example, a substitution, deletion or addition of 1, 2 or 3 amino acids) as compared to SEQ ID NO: 28, CDR-H2 with a sequence as set forth in SEQ ID NO: 29 or a sequence having a substitution, deletion or addition of one or several amino acids (for example, a substitution, deletion or addition of 1, 2 or 3 amino acids) as compared to SEQ ID NO: 29, CDR-H3 with a sequence as set forth in SEQ ID NO: 30 or a sequence having a substitution, deletion or addition of one or several amino acids (for example, a substitution, deletion or addition of 1, 2 or 3 amino acids) as compared to SEQ ID NO: 30; and/or,
   a light chain variable region (VL) comprising three CDRs as follows: CDR-L1 with a sequence as set forth in SEQ ID NO: 31 or a sequence having a substitution, deletion or addition of one or several amino acids (for example, a substitution, deletion or addition of 1, 2 or 3 amino acids) as compared to SEQ ID NO: 31, CDR-L2 with a sequence as set forth in SEQ ID NO: 32 or a sequence having a substitution, deletion or addition of one or several amino acids (for example, a substitution, deletion or addition of 1, 2 or 3 amino acids) as compared to SEQ ID NO: 32, CDR-L3 with a sequence as set forth in SEQ ID NO: 33 or a sequence having a substitution, deletion or addition of one or several amino acids (for example, a substitution, deletion or addition of 1, 2 or 3 amino acids) as compared to SEQ ID NO: 33;
(c) a heavy chain variable region (VH) comprising three CDRs as follows: CDR-H1 with a sequence as set forth in SEQ ID NO: 44 or a sequence having a substitution, deletion or addition of one or several amino acids (for example, a substitution, deletion or addition of 1, 2 or 3 amino acids) as compared to SEQ ID NO: 44, CDR-H2 with a sequence as set forth in SEQ ID NO: 45 or a sequence having a substitution, deletion or addition of one or several amino acids (for example, a substitution, deletion or addition of 1, 2 or 3 amino acids) as compared to SEQ ID NO: 45, CDR-H3 with a sequence as set forth in SEQ ID NO: 47 or a sequence having a substitution, deletion or addition of one or several amino acids (for example, a substitution, deletion or addition of 1, 2 or 3 amino acids) as compared to SEQ ID NO: 47; and/or,
   a light chain variable region (VL) comprising three CDRs as follows: CDR-L1 with a sequence as set forth in SEQ ID NO: 48 or a sequence having a substitution, deletion or addition of one or several amino acids (for example, a substitution, deletion or addition of 1, 2 or 3 amino acids) as compared to SEQ ID NO: 48, CDR-L2 with a sequence as set forth in SEQ ID NO: 49 or a sequence having a substitution, deletion or addition of one or several amino acids (for example, a substitution, deletion or addition of 1, 2 or 3 amino acids) as compared to SEQ ID NO: 49, CDR-L3 with a sequence as set forth in SEQ ID NO: 50 or a sequence having a substitution, deletion or addition of one or several amino acids (for example, a substitution, deletion or addition of 1, 2 or 3 amino acids) as compared to SEQ ID NO: 50;
(d) a heavy chain variable region (VH) comprising three CDRs as follows: CDR-H1 with a sequence as set forth in SEQ ID NO: 62 or a sequence having a substitution, deletion or addition of one or several amino acids (for example, a substitution, deletion or addition of 1, 2 or 3 amino acids) as compared to SEQ ID NO: 62, CDR-H2 with a sequence as set forth in SEQ ID NO: 63 or a sequence having a substitution, deletion or addition of one or several amino acids (for example, a substitution, deletion or addition of 1, 2 or 3 amino acids) as compared to SEQ ID NO: 63, CDR-H3 with a sequence as set forth in SEQ ID NO: 64 or a sequence having a substitution, deletion or addition of one or several amino acids (for example, a substitution, deletion or addition of 1, 2 or 3 amino acids) as compared to SEQ ID NO: 64; and/or,
   a light chain variable region (VL) comprising three CDRs as follows: CDR-L1 with a sequence as set forth in SEQ ID NO: 65 or a sequence having a substitution, deletion or addition of one or several amino acids (for example, a substitution, deletion or addition of 1, 2 or 3 amino acids) as compared to SEQ ID NO: 65, CDR-L2 with a sequence as set forth in SEQ ID NO: 66 or a sequence having a substitution, deletion or addition of one or several amino acids (for example, a substitution, deletion or addition of 1, 2 or 3 amino acids) as compared to SEQ ID NO: 66, CDR-L3 with a sequence as set forth in SEQ ID NO: 67 or a sequence having a substitution, deletion or addition of one or several amino acids (for example, a substitution, deletion or addition of 1, 2 or 3 amino acids) as compared to SEQ ID NO: 67;
   or
(e) a heavy chain variable region (VH) comprising three CDRs as follows: CDR-H1 with a sequence as set forth in SEQ ID NO: 76 or a sequence having a substitution, deletion or addition of one or several amino acids (for example, a substitution, deletion or addition of 1, 2 or 3 amino acids) as compared to SEQ ID NO: 76, CDR-H2 with a sequence as set forth in SEQ ID NO: 77 or a sequence having a substitution, deletion or addition of one or several amino acids (for example, a substitution, deletion or addition of 1, 2 or 3 amino acids) as compared to SEQ ID NO:77, CDR-H3 with a sequence as set forth in SEQ ID NO: 78 or a sequence having a substitution, deletion or addition of one or several amino acids (for example, a substitution, deletion or addition of 1, 2 or 3 amino acids) as compared to SEQ ID NO: 78; and/or,
   a light chain variable region (VL) comprising three CDRs as follows: CDR-L1 with a sequence as set forth in SEQ ID NO: 79 or a sequence having a substitution, deletion or addition of one or several amino acids (for example, a substitution, deletion or addition of 1, 2 or 3 amino acids) as compared to SEQ ID NO: 79, CDR-L2 with a sequence as set forth in SEQ ID NO: 80 or a sequence having a substitution, deletion or addition of one or several amino acids (for example, a substitution, deletion or addition of 1, 2 or 3 amino acids) as compared to SEQ ID NO: 80, CDR-L3 with a sequence as set forth in SEQ ID NO: 81 or a sequence having a substitution, deletion or addition of one or several amino acids (for example, a substitution, deletion or addition of 1, 2 or 3 amino acids) as compared to SEQ ID NO: 81.

In certain embodiments, the antibody or antigen-binding fragment thereof of the invention comprises a heavy chain variable region (VH) and/or a light chain variable region (VL) as follows, wherein the CDRs are defined by the Kabat numbering system:
(a) a heavy chain variable region (VH) comprising three CDRs as follows: CDR-H1 with a sequence as set forth in SEQ ID NO: 9, CDR-H2 with a sequence as set forth in SEQ ID NO: 10, CDR-H3 with a sequence as set forth in SEQ ID NO: 12; and/or,
   a light chain variable region (VL) comprising three CDRs as follows: CDR-L1 with a sequence as set forth in SEQ ID NO: 13, CDR-L2 with a sequence as set forth in SEQ ID NO: 14, CDR-L3 with a sequence as set forth in SEQ ID NO: 15;
(b) a heavy chain variable region (VH) comprising three CDRs as follows: CDR-H1 with a sequence as set forth in SEQ ID NO: 28, CDR-H2 with a sequence as set forth in SEQ ID NO: 29, CDR-H3 with a sequence as set forth in SEQ ID NO: 30; and/or,
   a light chain variable region (VL) comprising three CDRs as follows: CDR-L1 with a sequence as set forth in SEQ ID NO: 31, CDR-L2 with a sequence as set forth in SEQ ID NO: 32, CDR-L3 with a sequence as set forth in SEQ ID NO: 33;
(c) a heavy chain variable region (VH) comprising three CDRs as follows: CDR-H1 with a sequence as set forth in SEQ ID NO: 44, CDR-H2 with a sequence as set forth in SEQ ID NO: 45, CDR-H3 with a sequence as set forth in SEQ ID NO: 47; and/or,
   a light chain variable region (VL) comprising three CDRs as follows: CDR-L1 with a sequence as set forth in SEQ ID NO: 48, CDR-L2 with a sequence as set forth in SEQ ID NO: 49, CDR-L3 with a sequence as set forth in SEQ ID NO: 50;
(d) a heavy chain variable region (VH) comprising three CDRs as follows: CDR-H1 with a sequence as set forth in SEQ ID NO: 62, CDR-H2 with a sequence as set forth in SEQ ID NO: 63, CDR-H3 with a sequence as set forth in SEQ ID NO: 64; and/or,
   a light chain variable region (VL) comprising three CDRs as follows: CDR-L1 with a sequence as set forth in SEQ ID NO: 65, CDR-L2 with a sequence as set forth in SEQ ID NO: 66, CDR-L3 with a sequence as set forth in SEQ ID NO: 67;
   or
(e) a heavy chain variable region (VH) comprising three CDRs as follows: CDR-H1 with a sequence as set forth in SEQ ID NO: 76, CDR-H2 with a sequence as set forth in SEQ ID NO: 77, CDR-H3 with a sequence as set forth in SEQ ID NO: 78; and/or,
   a light chain variable region (VL) comprising three CDRs as follows: CDR-L1 with a sequence as set forth in SEQ ID NO: 79, CDR-L2 with a sequence as set forth in SEQ ID NO: 80, CDR-L3 with a sequence as set forth in SEQ ID NO: 81.

In certain embodiments, the antibody or antigen-binding fragment thereof of the invention comprises a heavy chain variable regions (VH) and/or a light chain variable regions (VL), wherein at least one CDR in the heavy chain variable region (VH) and/or the light chain variable region (VL) contains a mutation which is a substitution, deletion, addition of one or several amino acids or any combination thereof (for example, a substitution, deletion, addition of 1, 2 or 3 amino acids, or any combination thereof) as compared with the CDR defined by IMGT or Kabat as described above.

Preferably, the substitution of the invention is a conservative substitution.

In certain embodiments, the VH of the antibody or antigen-binding fragment thereof of the invention comprises framework regions (FRs) derived from a heavy chain variable region (VH) of a murine immunoglobulin, and/or the VL of the antibody or antigen-binding fragment thereof of the invention comprises framework regions (FRs) derived from a light chain variable region (VL) of a murine immunoglobulin. Therefore, in certain embodiments, the antibody or antigen-binding fragment thereof of the invention is murine.

In certain embodiments, the VH of the antibody or antigen-binding fragment thereof of the invention comprises framework regions (FRs) derived from a heavy chain variable region (VH) of a human immunoglobulin, and/or the VL of the antibody or antigen-binding fragment thereof of the invention comprises framework regions (FRs) derived from a light chain variable region (VL) of a human immunoglobulin. Therefore, in certain embodiments, the antibody or antigen-binding fragment thereof of the invention is humanized. In such embodiments, one or more FRs in the heavy chain variable region and/or one or more FRs in the light chain variable region of the antibody or antigen-binding fragment thereof of the invention may comprise one or more non-human (e.g. murine) amino acid residues, for example, one or more FRs in the heavy chain variable region and/or one or more FRs in the light chain variable region may comprise one or more amino acid reverse mutations in which corresponding murine amino acid residues are present.

In some embodiments, the antibody or antigen-binding fragment thereof of the invention comprises:
(a) a heavy chain framework region of a human immunoglobulin or a variant thereof, the variant comprising conservative substitutions of up to 20 amino acids (e.g., conservative substitutions of up to 20, up to 15, up to 10, or up to 5 amino acids; e.g., conservative substitutions of 1, 2, 3, 4, 5, 6, 7, 8, 9, or 10 amino acids) as compared to the germline antibody gene sequence from which it is derived; and/or
(b) a light chain framework region of a human immunoglobulin or a variant thereof, the variant comprising conservative substitutions of up to 20 amino acids (e.g., conservative substitutions of up to 20, up to 15, up to 10, or up to 5 amino acids; e.g., conservative substitutions of 1, 2, 3, 4, 5, 6, 7, 8, 9, or 10 amino acids) as compared to the germline antibody gene sequence from which it is derived.

In certain embodiments, the humanization degree of the antibody or antigen-binding fragment thereof of the invention is at least 75%, at least 80%, at least 85%, at least 90%, at least 91%, at least 92%, at least 93%, at least 94%, at least 95%, at least 96%, at least 97%, at least 98%, at least 99%, or 100%.

In certain embodiments, the antibody or antigen-binding fragment thereof of the invention comprises:
(a) a heavy chain variable region (VH) comprising an amino acid sequence selected from the group consisting of:
   (i) a sequence of any one set forth in SEQ ID NOS: 1, 20,36, 54, and 68;
   (ii) a sequence having a substitution, deletion or addition of one or several amino acids or any combination thereof (e.g., a substitution, deletion or addition of 1, 2, 3, 4, 5, 6, 7, 8, 9 or 10 amino acids, or any combination thereof) as compared to any one of the sequences set forth in SEQ ID NOs: 1, 20, 36, 54, and 68; or
   (iii) a sequence having at least 70%, at least 75%, at least 80%, at least 85%, at least 90%, at least 91%, at least 92%, at least 93%, at least 94%, at least 95%, at least 96%, at least 97%, at least 98%, or at least 99%, or 100% sequence identity to any one of the sequences set forth in SEQ ID NOs: 1, 20, 36, 54, and 68;
      and/or
(b) a light chain variable region (VL) comprising an amino acid sequence selected from the group consisting of:
   (iv) a sequence of any one set forth in SEQ ID NOs: 2, 21, 37, 55, and 69;
   (v) a sequence having a substitution, deletion or addition of one or several amino acids or any combination thereof (e.g., a substitution, deletion or addition of 1, 2, 3, 4, 5, 6, 7, 8, 9 or 10 amino acids, or any combination thereof) as compared to any one of the sequences set forth in SEQ ID NOs: 2, 21, 37, 55, and 69; or
   (vi) a sequence having at least 70%, at least 75%, at least 80%, at least 85%, at least 90%, at least 91%, at least 92%, at least 93%, at least 94%, at least 95%, at least 96%, at least 97%, at least 98%, or at least 99%, or 100% sequence identity to any one of the sequences set forth in SEQ ID NOS: 2, 21, 37, 55, and 69;

In some embodiments, the antibody or antigen-binding fragment thereof of the invention comprises:
(a) a heavy chain variable region (VH) comprising an amino acid sequence selected from the group consisting of:
   (i) a sequence of any one set forth in SEQ ID NOs: 16, 17, 34, 51, and 52;
   (ii) a sequence having a substitution, deletion or addition of one or several amino acids or any combination thereof (e.g., a substitution, deletion or addition of 1, 2, 3, 4, 5, 6, 7, 8, 9 or 10 amino acids, or any combination thereof) as compared to any one of the sequences set forth in SEQ ID NOs: 16, 17, 34, 51, and 52; or
   (iii) a sequence having at least 75%, at least 80%, at least 85%, at least 90%, at least 91%, at least 92%, at least 93%, at least 94%, at least 95%, at least 96%, at least 97%, at least 98%, at least 99%, or 100% sequence identity to any one of the sequences set forth in SEQ ID NOs: 16, 17, 34, 51, and 52; and/or
(b) a light chain variable region (VL) comprising an amino acid sequence selected from the group consisting of:
   (iv) a sequence of any one set forth in SEQ ID NOs: 18, 19, 35, and 53;
   (v) a sequence having a substitution, deletion or addition of one or several amino acids or any combination thereof (e.g., a substitution, deletion or addition of 1, 2, 3, 4, 5, 6, 7, 8, 9 or 10 amino acids, or any combination thereof) as compared to any one of the sequences set forth in SEQ ID NOs: 18, 19, 35, and 53; or
   (vi) a sequence having at least 70%, at least 75%, at least 80%, at least 85%, at least 90%, at least 91%, at least 92%, at least 93%, at least 94%, at least 95%, at least 96%, at least 97%, at least 98%, or at least 99%, or 100% sequence identity to any one of the sequences set forth in SEQ ID NOs: 18, 19, 35, and 53.

In certain embodiments, the antibody or antigen-binding fragment thereof of the invention comprises a heavy chain variable region (VH) set forth in any one of SEQ ID NOs: 1, 20, 36, 54, and 68, and/or a light chain variable region (VL) set forth in any one of SEQ ID NOs: 2, 21, 37, 55, and 69.

In certain embodiments, the antibody or antigen-binding fragment thereof of the invention comprises a heavy chain variable region (VH) set forth in any one of SEQ ID NOs: 16, 17, 34, 51, and 52, and/or a light chain variable region (VL) set forth in any one of SEQ ID NOs: 18, 19, 35, and 53.

In certain embodiments, the antibody or antigen-binding fragment thereof of the invention comprises a VH as set forth in SEQ ID NO: 1, and/or a VL as set forth in SEQ ID NO: 2.

In certain embodiments, the antibody or antigen-binding fragment thereof of the invention comprises a VH having at least 70%, at least 80%, at least 85%, at least 90%, at least 91%, at least 92%, at least 93%, at least 94%, at least 95%, at least 96%, at least 97%, at least 98%, at least 99%, or 100% sequence identity to the VH set forth in SEQ ID NO: 1, and/or a VL having at least 70%, at least 80%, at least 85%, at least 90%, at least 91%, at least 92%, at least 93%, at least 94%, at least 95%, at least 96%, at least 97%, at least 98%, at least 99%, or 100% sequence identity to the VL set forth in SEQ ID NO: 2.

In certain embodiments, the antibody or antigen-binding fragment thereof comprises a VH having a substitution, deletion or addition of one or several amino acids or any combination thereof (e.g. having a substitution, deletion or addition of 1, 2, 3, 4, 5, 6, 7, 8, 9 or 10 amino acids, or any combination thereof) compared to the VH set forth in SEQ ID NO: 1, and/or, a VL having a substitution, deletion or addition of one or several amino acids or any combination thereof (e.g. having a substitution, deletion or addition of 1, 2, 3, 4, 5, 6, 7, 8, 9 or 10 amino acids, or any combination thereof) compared to the VL set forth in SEQ ID NO: 2. In a preferred embodiment, the substitution is a conservative substitution.

In certain embodiments, the antibody or antigen-binding fragment thereof of the invention comprises a VH set forth in SEQ ID NO: 16, and/or a VL set forth in SEQ ID NO: 18.

In certain embodiments, the antibody or antigen-binding fragment thereof of the invention comprises a VH having at least 70%, at least 80%, at least 85%, at least 90%, at least 91%, at least 92%, at least 93%, at least 94%, at least 95%, at least 96%, at least 97%, at least 98%, at least 99%, or 100% sequence identity to the VH set forth in SEQ ID NO: 16, and/or a VL having at least 70%, at least 80%, at least 85%, at least 90%, at least 91%, at least 92%, at least 93%, at least 94%, at least 95%, at least 96%, at least 97%, at least 98%, at least 99%, or 100% sequence identity to the VL set forth in SEQ ID NO: 18.

In certain embodiments, the antibody or antigen-binding fragment thereof comprises a VH having a substitution, deletion or addition of one or several amino acids or any combination thereof (e.g. having a substitution, deletion or addition of 1, 2, 3, 4, 5, 6, 7, 8, 9 or 10 amino acids, or any combination thereof) compared to the VH set forth in SEQ ID NO: 16, and/or, a VL having a substitution, deletion or addition of one or several amino acids or any combination thereof (e.g. having a substitution, deletion or addition of 1, 2, 3, 4, 5, 6, 7, 8, 9 or 10 amino acids, or any combination thereof) compared to the VL set forth in SEQ ID NO: 18. In a preferred embodiment, the substitution is a conservative substitution.

In certain embodiments, the antibody or antigen-binding fragment thereof of the invention comprises a VH set forth in SEQ ID NO: 17, and/or a VL set forth in SEQ ID NO: 19.

In certain embodiments, the antibody or antigen-binding fragment thereof of the invention comprises a VH having at least 70%, at least 80%, at least 85%, at least 90%, at least 91%, at least 92%, at least 93%, at least 94%, at least 95%, at least 96%, at least 97%, at least 98%, at least 99%, or 100% sequence identity to the VH set forth in SEQ ID NO: 17, and/or a VL having at least 70%, at least 80%, at least 85%, at least 90%, at least 91%, at least 92%, at least 93%, at least 94%, at least 95%, at least 96%, at least 97%, at least 98%, at least 99%, or 100% sequence identity to the VL set forth in SEQ ID NO: 19.

In certain embodiments, the antibody or antigen-binding fragment thereof comprises a VH having a substitution, deletion or addition of one or several amino acids or any combination thereof (e.g. having a substitution, deletion or addition of 1, 2, 3, 4, 5, 6, 7, 8, 9 or 10 amino acids, or any combination thereof) compared to the VH set forth in SEQ ID NO: 17, and/or, a VL having a substitution, deletion or addition of one or several amino acids or any combination thereof (e.g. having a substitution, deletion or addition of 1, 2, 3, 4, 5, 6, 7, 8, 9 or 10 amino acids, or any combination thereof) compared to the VL set forth in SEQ ID NO: 19. In a preferred embodiment, the substitution is a conservative substitution.

In certain embodiments, the antibody or antigen-binding fragment thereof of the invention comprises a VH set forth in SEQ ID NO: 20, and/or a VL set forth in SEQ ID NO: 21.

In certain embodiments, the antibody or antigen-binding fragment thereof of the invention comprises a VH having at least 70%, at least 80%, at least 85%, at least 90%, at least 91%, at least 92%, at least 93%, at least 94%, at least 95%, at least 96%, at least 97%, at least 98%, at least 99%, or 100% sequence identity to the VH set forth in SEQ ID NO: 20, and/or a VL having at least 70%, at least 80%, at least 85%, at least 90%, at least 91%, at least 92%, at least 93%, at least 94%, at least 95%, at least 96%, at least 97%, at least 98%, at least 99%, or 100% sequence identity to the VL set forth in SEQ ID NO: 21.

In certain embodiments, the antibody or antigen-binding fragment thereof comprises a VH having a substitution, deletion or addition of one or several amino acids or any combination thereof (e.g. a substitution, deletion or addition of 1, 2, 3, 4, 5, 6, 7, 8, 9 or 10 amino acids, or any combination thereof) compared to the VH set forth in SEQ ID NO: 20, and/or, a VL having a substitution, deletion or addition of one or several amino acids or any combination thereof (e.g. a substitution, deletion or addition of 1, 2, 3, 4, 5, 6, 7, 8, 9 or 10 amino acids, or any combination thereof) compared to the VL set forth in SEQ ID NO: 21. In a preferred embodiment, the substitution is a conservative substitution.

In certain embodiments, the antibody or antigen-binding fragment thereof of the invention comprises a VH set forth in SEQ ID NO: 34, and/or a VL set forth in SEQ ID NO: 35.

In certain embodiments, the antibody or antigen-binding fragment thereof of the invention comprises a VH having at least 70%, at least 80%, at least 85%, at least 90%, at least 91%, at least 92%, at least 93%, at least 94%, at least 95%, at least 96%, at least 97%, at least 98%, at least 99%, or 100% sequence identity to the VH set forth in SEQ ID NO: 34, and/or a VL having at least 70%, at least 80%, at least 85%, at least 90%, at least 91%, at least 92%, at least 93%, at least 94%, at least 95%, at least 96%, at least 97%, at least 98%, at least 99%, or 100% sequence identity to the VL set forth in SEQ ID NO: 35.

In certain embodiments, the antibody or antigen-binding fragment thereof comprises a VH having a substitution, deletion or addition of one or several amino acids or any combination thereof (e.g. a substitution, deletion or addition of 1, 2, 3, 4, 5, 6, 7, 8, 9 or 10 amino acids, or any combination thereof) compared to the VH set forth in SEQ ID NO: 34, and/or, a VL having a substitution, deletion or addition of one or several amino acids or any combination thereof (e.g. a substitution, deletion or addition of 1, 2, 3, 4, 5, 6, 7, 8, 9 or 10 amino acids, or any combination thereof) compared to the VL set forth in SEQ ID NO: 35. In a preferred embodiment, the substitution is a conservative substitution.

In certain embodiments, the antibody or antigen-binding fragment thereof of the invention comprises a VH set forth in SEQ ID NO: 36, and/or a VL set forth in SEQ ID NO: 37.

In certain embodiments, the antibody or antigen-binding fragment thereof of the invention comprises a VH having at least 70%, at least 80%, at least 85%, at least 90%, at least 91%, at least 92%, at least 93%, at least 94%, at least 95%, at least 96%, at least 97%, at least 98%, at least 99%, or 100% sequence identity to the VH set forth in SEQ ID NO: 36, and/or a VL having at least 70%, at least 80%, at least 85%, at least 90%, at least 91%, at least 92%, at least 93%, at least 94%, at least 95%, at least 96%, at least 97%, at least 98%, at least 99%, or 100% sequence identity to the VL set forth in SEQ ID NO: 37.

In certain embodiments, the antibody or antigen-binding fragment thereof comprises a VH having a substitution, deletion or addition of one or several amino acids or any combination thereof (e.g. a substitution, deletion or addition of 1, 2, 3, 4, 5, 6, 7, 8, 9 or 10 amino acids, or any combination thereof) compared to the VH set forth in SEQ ID NO: 36, and/or, a VL having a substitution, deletion or addition of one or several amino acids or any combination thereof (e.g. a substitution, deletion or addition of 1, 2, 3, 4, 5, 6, 7, 8, 9 or 10 amino acids, or any combination thereof) compared to the VL set forth in SEQ ID NO: 37. In a preferred embodiment, the substitution is a conservative substitution.

In certain embodiments, the antibody or antigen-binding fragment thereof of the invention comprises a VH set forth in SEQ ID NO: 51 or 52, and/or a VL set forth in SEQ ID NO: 53.

In certain embodiments, the antibody or antigen-binding fragment thereof of the invention comprises a VH having at least 70%, at least 80%, at least 85%, at least 90%, at least 91%, at least 92%, at least 93%, at least 94%, at least 95%, at least 96%, at least 97%, at least 98%, at least 99%, or 100% sequence identity to the VH set forth in SEQ ID NO: 51 or 52, and/or a VL having at least 70%, at least 80%, at least 85%, at least 90%, at least 91%, at least 92%, at least 93%, at least 94%, at least 95%, at least 96%, at least 97%, at least 98%, at least 99%, or 100% sequence identity to the VL set forth in SEQ ID NO: 53.

In certain embodiments, the antibody or antigen-binding fragment thereof comprises a VH having a substitution, deletion or addition of one or several amino acids or any combination thereof (e.g. a substitution, deletion or addition of 1, 2, 3, 4, 5, 6, 7, 8, 9 or 10 amino acids, or any combination thereof) compared to the VH set forth in SEQ ID NO: 51 or 52, and/or, a VL having a substitution, deletion or addition of one or several amino acids or any combination thereof (e.g. a substitution, deletion or addition of 1, 2, 3, 4, 5, 6, 7, 8, 9 or 10 amino acids, or any combination thereof) compared to the VL set forth in SEQ ID NO: 53. In a preferred embodiment, the substitution is a conservative substitution.

In certain embodiments, the antibody or antigen-binding fragment thereof of the invention comprises a VH set forth in SEQ ID NO: 54, and/or a VL set forth in SEQ ID NO: 55.

In certain embodiments, the antibody or antigen-binding fragment thereof of the invention comprises a VH having at least 70%, at least 80%, at least 85%, at least 90%, at least 91%, at least 92%, at least 93%, at least 94%, at least 95%, at least 96%, at least 97%, at least 98%, at least 99%, or 100% sequence identity to the VH set forth in SEQ ID NO: 54, and/or a VL having at least 70%, at least 80%, at least 85%, at least 90%, at least 91%, at least 92%, at least 93%, at least 94%, at least 95%, at least 96%, at least 97%, at least 98%, at least 99%, or 100% sequence identity to the VL set forth in SEQ ID NO: 55.

In certain embodiments, the antibody or antigen-binding fragment thereof comprises a VH having a substitution, deletion or addition of one or several amino acids or any combination thereof (e.g. a substitution, deletion or addition of 1, 2, 3, 4, 5, 6, 7, 8, 9 or 10 amino acids, or any combination thereof) compared to the VH set forth in SEQ ID NO: 54, and/or, a VL having a substitution, deletion or addition of one or several amino acids or any combination thereof (e.g. a substitution, deletion or addition of 1, 2, 3, 4, 5, 6, 7, 8, 9 or 10 amino acids, or any combination thereof) compared to the VL set forth in SEQ ID NO: 55. In a preferred embodiment, the substitution is a conservative substitution.

In certain embodiments, the antibody or antigen-binding fragment thereof of the invention comprises a VH set forth in SEQ ID NO: 68, and/or a VL set forth in SEQ ID NO: 69.

In certain embodiments, the antibody or antigen-binding fragment thereof of the invention comprises a VH having at least 70%, at least 80%, at least 85%, at least 90%, at least 91%, at least 92%, at least 93%, at least 94%, at least 95%, at least 96%, at least 97%, at least 98%, at least 99%, or 100% sequence identity to the VH set forth in SEQ ID NO: 68, and/or a VL having at least 70%, at least 80%, at least 85%, at least 90%, at least 91%, at least 92%, at least 93%, at least 94%, at least 95%, at least 96%, at least 97%, at least 98%, at least 99%, or 100% sequence identity to the VL set forth in SEQ ID NO: 69.

In certain embodiments, the antibody or antigen-binding fragment thereof comprises a VH having a substitution, deletion or addition of one or several amino acids or any combination thereof (e.g. a substitution, deletion or addition of 1, 2, 3, 4, 5, 6, 7, 8, 9 or 10 amino acids, or any combination thereof) compared to the VH set forth in SEQ ID NO: 68, and/or, a VL having a substitution, deletion or addition of one or several amino acids or any combination thereof (e.g. a substitution, deletion or addition of 1, 2, 3, 4, 5, 6, 7, 8, 9 or 10 amino acids, or any combination thereof) compared to the VL set forth in SEQ ID NO: 69. In a preferred embodiment, the substitution is a conservative substitution.

In some embodiments, the antibody or antigen-binding fragment thereof of the invention comprises:
(a) a VH with a sequence as set forth in SEQ ID NO: 1 and a VL with a sequence as set forth in SEQ ID NO: 2;
(b) a VH with a sequence as set forth in SEQ ID NO: 16 and a VL with a sequence as set forth in SEQ ID NO: 18;
(c) a VH with a sequence as set forth in SEQ ID NO: 17 and a VL with a sequence as set forth in SEQ ID NO: 19;
(d) a VH with a sequence as set forth in SEQ ID NO: 20 and a VL with a sequence as set forth in SEQ ID NO: 21;
(e) a VH with a sequence as set forth in SEQ ID NO: 34 and a VL with a sequence as set forth in SEQ ID NO: 35;
(f) a VH with a sequence as set forth in SEQ ID NO: 36 and a VL with a sequence as set forth in SEQ ID NO: 37;
(g) a VH with a sequence as set forth in SEQ ID NO: 51 and a VL with a sequence as set forth in SEQ ID NO: 53;
(h) a VH with a sequence as set forth in SEQ ID NO: 52 and a VL with a sequence as set forth in SEQ ID NO: 53;
(i) a VH with a sequence as set forth in SEQ ID NO: 54 and a VL with a sequence as set forth in SEQ ID NO: 55;
(j) a VH with a sequence as set forth in SEQ ID NO: 68 and a VL with a sequence as set forth in SEQ ID NO: 69;
(k) a heavy chain variable region (VH) and a light chain variable region (VL) independently having at least 70%, at least 80%, at least 85%, at least 90%, at least 91%, at least 92%, at least 93%, at least 94%, at least 95%, at least 96%, at least 97%, at least 98%, at least 99%, or 100% sequence identity to the VH and the VL in any of (a) to (j), respectively; or
(l) a heavy chain variable region (VH) and a light chain variable region (VL) independently having a substitution, deletion or addition of one or several amino acids or any combination thereof (e.g., having a substitution, deletion or addition of 1, 2, 3, 4, 5, 6, 7, 8, 9 or 10 amino acids, or any combination thereof) as compared to the VH and the VL in any of (a) to (j), respectively. Preferably, the substitution is a conservative substitution.

In any one of the above aspects, the antibody or antigen-binding fragment thereof of the invention may further comprise a constant region sequence derived from a mammal (e.g., a mouse or a human) immunoglobulin or a variant thereof. In certain embodiments, the heavy chain of the antibody or antigen-binding fragment thereof of the invention comprises a heavy chain constant region (CH) of a human or murine immunoglobulin or a variant thereof, wherein the variant has a substitution, deletion or addition of one or more amino acids or any combination thereof (e.g., a substitution, deletion or addition of up to 50, up to 45, up to 40, up to 35, up to 30, up to 25, up to 20, up to 15, up to 10, or up to 5 amino acids, or any combination thereof; e.g., a substitution, deletion or addition of 1, 2, 3, 4, 5, 6, 7, 8, 9 or 10 amino acids, or any combination thereof) as compared to the wild-type sequence from which it is derived; and/or, the light chain of the antibody or antigen-binding fragment thereof of the invention comprises a light chain constant region (CL) of a human or murine immunoglobulin or a variant thereof, wherein the variant has a substitution, deletion or addition of one or more amino acids or any combination thereof (e.g., a substitution, deletion or addition of up to 50, up to 45, up to 40, up to 35, up to 30, up to 25, up to 20, up to 15, up to 10, or up to 5 amino acids, or any combination thereof; e.g., a substitution, deletion or addition of 1, 2, 3, 4, 5, 6, 7, 8, 9 or 10 amino acids, or any combination thereof) as compared to the wild-type sequence from which it is derived.

In certain embodiments, the heavy chain of the antibody or antigen-binding fragment thereof of the invention comprises a heavy chain constant region (CH) of a human immunoglobulin or a variant thereof, wherein the variant has conservative substitutions of up to 50 amino acids (e.g., conservative substitutions of up to 45, up to 40, up to 35, up to 30, up to 25, up to 20, up to 15, up to 10, or up to 5 amino acids; e.g., conservative substitutions of 1, 2, 3, 4, 5, 6, 7, 8, 9 or 10 amino acids) as compared to the wild-type sequence from which it is derived; and/or, the light chain of the antibody or antigen-binding fragment thereof of the invention comprises a light chain constant region (CL) of a human immunoglobulin or a variant thereof, wherein the variant has conservative substitutions of up to 50 amino acids (e.g., conservative substitutions of up to 45, up to 40, up to 35, up to 30, up to 25, up to 20, up to 15, up to 10, or up to 5 amino acids; e.g., conservative substitutions of 1, 2, 3, 4, 5, 6, 7, 8, 9 or 10 amino acids) as compared to the wild-type sequence from which it is derived.

In certain embodiments, the antibody or antigen-binding fragment thereof of the invention comprises a heavy chain constant region (CH) of a murine immunoglobulin or a variant thereof, wherein the variant has conservative substitutions of up to 50 amino acids (e.g., conservative substitutions of up to 45, up to 40, up to 35, up to 30, up to 25, up to 20, up to 15, up to 10, or up to 5 amino acids; e.g., conservative substitutions of 1, 2, 3, 4, 5, 6, 7, 8, 9 or 10 amino acids) as compared to the wild-type sequence from which it is derived. In certain embodiments, the antibody or antigen-binding fragment thereof of the invention comprises a light chain constant region (CL) of a murine immunoglobulin or a variant thereof, wherein the variant has conservative substitutions of up to 50 amino acids (e.g., conservative substitutions of up to 45, up to 40, up to 35, up to 30, up to 25, up to 20, up to 15, up to 10, or up to 5 amino acids; e.g., conservative substitutions of 1, 2, 3, 4, 5, 6, 7, 8, 9 or 10 amino acids) as compared to the wild-type sequence from which it is derived.

In some embodiments, the constant region is altered, e.g. mutated, to modify the properties of the anti-PD-1 antibody molecule (e.g., altering one or more of the following characteristics: Fc receptor binding, antibody glycosylation, the number of cysteine residues, effector cell function, or complement function). The functions can be altered by replacing at least one amino acid residue in the antibody constant region with a different residue, for example, to alter the affinity of the antibody for an effector ligand (e.g., FcR or complement C1q), thereby changing (e.g., decreasing) effector function. The Fc region of the antibody mediates several important effector functions, such as ADCC, phagocytosis, CDC and so on.

In certain embodiments, the antibody or antigen-binding fragment thereof of the invention comprises a heavy chain constant region (Fc) selected from the group consisting of heavy chain constant regions of, for example, IgG1, IgG2, IgG3, IgG4, IgM, IgA1, IgA2, IgD and IgE; particularly selected from the group consisting of heavy chain constant regions of, for example, IgG1, IgG2, IgG3 and IgG4, and more particularly selected from the group consisting of heavy chain constant regions of IgG1 and IgG4 (e.g., human IgG1 or IgG4). In some embodiments, the antibody or antigen-binding fragment thereof of the invention comprises a light chain constant region selected from, for example, a kappa or lambda light chain constant region, preferably a kappa light chain constant region (e.g., a human kappa light chain constant region).

In some embodiments, the antibody or antigen-binding fragment thereof comprises a heavy chain constant region selected from the group consisting of (1) a human IgG1 heavy chain constant region; (2) a human IgG4 heavy chain constant region; or (3) a mutant of human IgG4 heavy chain constant region (S228P).

In some embodiments, the antibody or antigen-binding fragment thereof comprises a human IgG1 constant region (uniprot ID P01857), or a human IgG4 constant region (uniprot ID P01861).

In certain embodiments, the antibody or antigen-binding fragment thereof comprises a heavy chain constant region (CH) as set forth in SEQ ID NO: 83 or a variant thereof, wherein the variant has conservative substitutions of up to 50 amino acids (e.g., conservative substitutions of up to 45, up to 40, up to 35, up to 30, up to 25, up to 20, up to 15, up to 10, or up to 5 amino acids; e.g., conservative substitutions of 1, 2, 3, 4, 5, 6, 7, 8, 9 or 10 amino acids) as compared to SEQ ID NO: 83; and/or, a light chain constant region (CL) as set forth in SEQ ID NO: 84 or a variant thereof, wherein the variant has conservative substitutions of up to 50 amino acids (e.g., conservative substitutions of up to 45, up to 40, up to 35, up to 30, up to 25, up to 20, up to 15, up to 10, or up to 5 amino acids; e.g., conservative substitutions of 1, 2, 3, 4, 5, 6, 7, 8, 9 or 10 amino acids) as compared to SEQ ID NO: 84.

In some embodiments, the antibody or antigen-binding fragment thereof of the invention comprises:
(a) a heavy chain constant region (CH) comprising an amino acid sequence selected from the group consisting of:
   (i) a sequence set forth in SEQ ID NO: 82 or 83;
   (ii) a sequence having a substitution, deletion or addition of one or several amino acids or any combination thereof (e.g., a substitution, deletion or addition of up to 50, up to 45, up to 40, up to 35, up to 30, up to 25, up to 20, up to 15, up to 10, or up to 5 amino acids, or any combination thereof; for example, a substitution, deletion or addition of 1, 2, 3, 4, 5, 6, 7, 8, 9 or 10 amino acids, or any combination thereof) as compared to the sequence set forth in SEQ ID NO: 82 or 83; or
   (iii) a sequence having at least 80%, at least 85%, at least 90%, at least 91%, at least 92%, at least 93%, at least 94%, at least 95%, at least 96%, at least 97%, at least 98%, at least 99%, or 100% sequence identity to the sequence set forth in SEQ ID NO: 82 or 83;
      and/or
(b) a light chain constant region (CL) comprising an amino acid sequence selected from the group consisting of:
   (iv) a sequence set forth in SEQ ID NO: 84;
   (v) a sequence having a substitution, deletion or addition of one or several amino acids or any combination thereof (e.g., a substitution, deletion or addition of up to 50, up to 45, up to 40, up to 35, up to 30, up to 25, up to 20, up to 15, up to 10, or up to 5 amino acids, or any combination thereof; for example, a substitution, deletion or addition of 1, 2, 3, 4, 5, 6, 7, 8, 9 or 10 amino acids, or any combination thereof) as compared to the sequence set forth in SEQ ID NO: 84; or
   (vi) a sequence having at least 80%, at least 85%, at least 90%, at least 91%, at least 92%, at least 93%, at least 94%, at least 95%, at least 96%, at least 97%, at least 98%, at least 99%, or 100% sequence identity to the sequence set forth in SEQ ID NO: 84.

In certain embodiments, the substitution in (ii) or (v) is a conservative substitution.

In certain embodiments, the antibody or antigen-binding fragment thereof of the invention comprises a heavy chain constant region (CH) set forth in SEQ ID NO: 82 or 83 and a light chain constant region (CL) set forth in SEQ ID NO: 84.

In some embodiments, the antibody or antigen-binding fragment thereof of the invention comprises:
(a) a heavy chain comprising an amino acid sequence selected from the group consisting of:
   (i) a sequence comprising the VH sequence set forth in SEQ ID NO: 1 and the CH sequence set forth in SEQ ID NO: 82 or 83;
   (ii) a sequence having a substitution, deletion or addition of one or several amino acids or any combination thereof (e.g., a substitution, deletion or addition of up to 50, up to 45, up to 40, up to 35, up to 30, up to 25, up to 20, up to 15, up to 10, or up to 5 amino acids, or any combination thereof; for example, a substitution, deletion or addition of 1, 2, 3, 4, 5, 6, 7, 8, 9 or 10 amino acids, or any combination thereof) as compared to the sequence in (i); or
   (iii) a sequence having at least 80%, at least 85%, at least 90%, at least 91%, at least 92%, at least 93%, at least 94%, at least 95%, at least 96%, at least 97%, at least 98%, at least 99%, or 100% sequence identity to the sequence in (i);
      and
(b) a light chain comprising an amino acid sequence selected from the group consisting of:
   (iv) a sequence comprising the VL sequence set forth in SEQ ID NO: 2 and the CL sequence set forth in SEQ ID NO: 84;
   (v) a sequence having a substitution, deletion or addition of one or several amino acids or any combination thereof (e.g., a substitution, deletion or addition of up to 50, up to 45, up to 40, up to 35, up to 30, up to 25, up to 20, up to 15, up to 10, or up to 5 amino acids, or any combination thereof; for example, a substitution, deletion or addition of 1, 2, 3, 4, 5, 6, 7, 8, 9 or 10 amino acids, or any combination thereof) as compared to the sequence in (iv); or
   (vi) a sequence having at least 80%, at least 85%, at least 90%, at least 91%, at least 92%, at least 93%, at least 94%, at least 95%, at least 96%, at least 97%, at least 98%, at least 99%, or 100% sequence identity to the sequence in (iv).

In certain embodiments, the substitution in (ii) or (v) is a conservative substitution.

In some embodiments, the antibody or antigen-binding fragment thereof of the invention comprises:
(a) a heavy chain comprising an amino acid sequence selected from the group consisting of:
   (i) a sequence comprising the VH sequence set forth in SEQ ID NO: 16 and the CH sequence set forth in SEQ ID NO: 82 or 83;
   (ii) a sequence having a substitution, deletion or addition of one or several amino acids or any combination thereof (e.g., a substitution, deletion or addition of up to 50, up to 45, up to 40, up to 35, up to 30, up to 25, up to 20, up to 15, up to 10, or up to 5 amino acids, or any combination thereof; for example, a substitution, deletion or addition of 1, 2, 3, 4, 5, 6, 7, 8, 9 or 10 amino acids, or any combination thereof) as compared to the sequence in (i); or
   (iii) a sequence having at least 80%, at least 85%, at least 90%, at least 91%, at least 92%, at least 93%, at least 94%, at least 95%, at least 96%, at least 97%, at least 98%, at least 99%, or 100% sequence identity to the sequence in (i);
   and
(b) a light chain comprising an amino acid sequence selected from the group consisting of:
   (iv) a sequence comprising the VL sequence set forth in SEQ ID NO: 18 and the CL sequence set forth in SEQ ID NO: 84;
   (v) a sequence having a substitution, deletion or addition of one or several amino acids or any combination thereof (e.g., a substitution, deletion or addition of up to 50, up to 45, up to 40, up to 35, up to 30, up to 25, up to 20, up to 15, up to 10, or up to 5 amino acids, or any combination thereof; for example, a substitution, deletion or addition of 1, 2, 3, 4, 5, 6, 7, 8, 9 or 10 amino acids, or any combination thereof) as compared to the sequence in (iv); or
   (vi) a sequence having at least 80%, at least 85%, at least 90%, at least 91%, at least 92%, at least 93%, at least 94%, at least 95%, at least 96%, at least 97%, at least 98%, at least 99%, or 100% sequence identity to the sequence in (iv).

In certain embodiments, the substitution in (ii) or (v) is a conservative substitution.

In some embodiments, the antibody or antigen-binding fragment thereof of the invention comprises:
(a) a heavy chain comprising an amino acid sequence selected from the group consisting of:
   (i) a sequence comprising the VH sequence set forth in SEQ ID NO: 17 and the CH sequence set forth in SEQ ID NO: 82 or 83;
   (ii) a sequence having a substitution, deletion or addition of one or several amino acids or any combination thereof (e.g., a substitution, deletion or addition of up to 50, up to 45, up to 40, up to 35, up to 30, up to 25, up to 20, up to 15, up to 10, or up to 5 amino acids, or any combination thereof; for example, a substitution, deletion or addition of 1, 2, 3, 4, 5, 6, 7, 8, 9 or 10 amino acids, or any combination thereof) as compared to the sequence in (i); or
   (iii) a sequence having at least 80%, at least 85%, at least 90%, at least 91%, at least 92%, at least 93%, at least 94%, at least 95%, at least 96%, at least 97%, at least 98%, at least 99%, or 100% sequence identity to the sequence in (i);
   and
(b) a light chain comprising an amino acid sequence selected from the group consisting of:
   (iv) a sequence comprising the VL sequence set forth in SEQ ID NO: 19 and the CL sequence set forth in SEQ ID NO: 84;
   (v) a sequence having a substitution, deletion or addition of one or several amino acids or any combination thereof (e.g., a substitution, deletion or addition of up to 50, up to 45, up to 40, up to 35, up to 30, up to 25, up to 20, up to 15, up to 10, or up to 5 amino acids, or any combination thereof; for example, a substitution, deletion or addition of 1, 2, 3, 4, 5, 6, 7, 8, 9 or 10 amino acids, or any combination thereof) as compared to the sequence in (iv); or
   (vi) a sequence having at least 80%, at least 85%, at least 90%, at least 91%, at least 92%, at least 93%, at least 94%, at least 95%, at least 96%, at least 97%, at least 98%, at least 99%, or 100% sequence identity to the sequence in (iv).

In certain embodiments, the substitution in (ii) or (v) is a conservative substitution.

In some embodiments, the antibody or antigen-binding fragment thereof of the invention comprises:
(a) a heavy chain comprising an amino acid sequence selected from the group consisting of:
   (i) a sequence comprising the VH sequence set forth in SEQ ID NO: 20 and the CH sequence set forth in SEQ ID NO: 82 or 83;
   (ii) a sequence having a substitution, deletion or addition of one or several amino acids or any combination thereof (e.g., a substitution, deletion or addition of up to 50, up to 45, up to 40, up to 35, up to 30, up to 25, up to 20, up to 15, up to 10, or up to 5 amino acids, or any combination thereof; for example, a substitution, deletion or addition of 1, 2, 3, 4, 5, 6, 7, 8, 9 or 10 amino acids, or any combination thereof) as compared to the sequence in (i); or
   (iii) a sequence having at least 80%, at least 85%, at least 90%, at least 91%, at least 92%, at least 93%, at least 94%, at least 95%, at least 96%, at least 97%, at least 98%, at least 99%, or 100% sequence identity to the sequence in (i);
   and
(b) a light chain comprising an amino acid sequence selected from the group consisting of:
   (iv) a sequence comprising the VL sequence set forth in SEQ ID NO: 21 and the CL sequence set forth in SEQ ID NO: 84;
   (v) a sequence having a substitution, deletion or addition of one or several amino acids or any combination thereof (e.g., a substitution, deletion or addition of up to 50, up to 45, up to 40, up to 35, up to 30, up to 25, up to 20, up to 15, up to 10, or up to 5 amino acids, or any combination thereof; for example, a substitution, deletion or addition of 1, 2, 3, 4, 5, 6, 7, 8, 9 or 10 amino acids, or any combination thereof) as compared to the sequence in (iv); or
   (vi) a sequence having at least 80%, at least 85%, at least 90%, at least 91%, at least 92%, at least 93%, at least 94%, at least 95%, at least 96%, at least 97%, at least 98%, at least 99%, or 100% sequence identity to the sequence in (iv).

In certain embodiments, the substitution in (ii) or (v) is a conservative substitution.

In some embodiments, the antibody or antigen-binding fragment thereof of the invention comprises:
(a) a heavy chain comprising an amino acid sequence selected from the group consisting of:
   (i) a sequence comprising the VH sequence set forth in SEQ ID NO: 34 and the CH sequence set forth in SEQ ID NO: 82 or 83;
   (ii) a sequence having a substitution, deletion or addition of one or several amino acids or any combination thereof (e.g., a substitution, deletion or addition of up to 50, up to 45, up to 40, up to 35, up to 30, up to 25, up to 20, up to 15, up to 10, or up to 5 amino acids, or any combination thereof; for example, a substitution, deletion or addition of 1, 2, 3, 4, 5, 6, 7, 8, 9 or 10 amino acids, or any combination thereof) as compared to the sequence in (i); or
   (iii) a sequence having at least 80%, at least 85%, at least 90%, at least 91%, at least 92%, at least 93%, at least 94%, at least 95%, at least 96%, at least 97%, at least 98%, at least 99%, or 100% sequence identity to the sequence in (i);
   and
(b) a light chain comprising an amino acid sequence selected from the group consisting of:
   (iv) a sequence comprising the VL sequence set forth in SEQ ID NO: 35 and the CL sequence set forth in SEQ ID NO: 84;
   (v) a sequence having a substitution, deletion or addition of one or several amino acids or any combination thereof (e.g., a substitution, deletion or addition of up to 50, up to 45, up to 40, up to 35, up to 30, up to 25, up to 20, up to 15, up to 10, or up to 5 amino acids, or any combination thereof; for example, a substitution, deletion or addition of 1, 2, 3, 4, 5, 6, 7, 8, 9 or 10 amino acids, or any combination thereof) as compared to the sequence in (iv); or
   (vi) a sequence having at least 80%, at least 85%, at least 90%, at least 91%, at least 92%, at least 93%, at least 94%, at least 95%, at least 96%, at least 97%, at least 98%, at least 99%, or 100% sequence identity to the sequence in (iv).

In certain embodiments, the substitution in (ii) or (v) is a conservative substitution.

In some embodiments, the antibody or antigen-binding fragment thereof of the invention comprises:
(a) a heavy chain comprising an amino acid sequence selected from the group consisting of:
   (i) a sequence comprising the VH sequence set forth in SEQ ID NO: 36 and the CH sequence set forth in SEQ ID NO: 82 or 83;
   (ii) a sequence having a substitution, deletion or addition of one or several amino acids or any combination thereof (e.g., a substitution, deletion or addition of up to 50, up to 45, up to 40, up to 35, up to 30, up to 25, up to 20, up to 15, up to 10, or up to 5 amino acids, or any combination thereof; for example, a substitution, deletion or addition of 1, 2, 3, 4, 5, 6, 7, 8, 9 or 10 amino acids, or any combination thereof) as compared to the sequence in (i); or
   (iii) a sequence having at least 80%, at least 85%, at least 90%, at least 91%, at least 92%, at least 93%, at least 94%, at least 95%, at least 96%, at least 97%, at least 98%, at least 99%, or 100% sequence identity to the sequence in (i);
   and
(b) a light chain comprising an amino acid sequence selected from the group consisting of:
   (iv) a sequence comprising the VL sequence set forth in SEQ ID NO: 37 and the CL sequence set forth in SEQ ID NO: 84;
   (v) a sequence having a substitution, deletion or addition of one or several amino acids or any combination thereof (e.g., a substitution, deletion or addition of up to 50, up to 45, up to 40, up to 35, up to 30, up to 25, up to 20, up to 15, up to 10, or up to 5 amino acids, or any combination thereof; for example, a substitution, deletion or addition of 1, 2, 3, 4, 5, 6, 7, 8, 9 or 10 amino acids, or any combination thereof) as compared to the sequence in (iv); or
   (vi) a sequence having at least 80%, at least 85%, at least 90%, at least 91%, at least 92%, at least 93%, at least 94%, at least 95%, at least 96%, at least 97%, at least 98%, at least 99%, or 100% sequence identity to the sequence in (iv).

In certain embodiments, the substitution in (ii) or (v) is a conservative substitution.

In some embodiments, the antibody or antigen-binding fragment thereof of the invention comprises:
(a) a heavy chain comprising an amino acid sequence selected from the group consisting of:
   (i) a sequence comprising the VH sequence set forth in SEQ ID NO: 51 and the CH sequence set forth in SEQ ID NO: 82 or 83;
   (ii) a sequence having a substitution, deletion or addition of one or several amino acids or any combination thereof (e.g., a substitution, deletion or addition of up to 50, up to 45, up to 40, up to 35, up to 30, up to 25, up to 20, up to 15, up to 10, or up to 5 amino acids, or any combination thereof; for example, a substitution, deletion or addition of 1, 2, 3, 4, 5, 6, 7, 8, 9 or 10 amino acids, or any combination thereof) as compared to the sequence in (i); or
   (iii) a sequence having at least 80%, at least 85%, at least 90%, at least 91%, at least 92%, at least 93%, at least 94%, at least 95%, at least 96%, at least 97%, at least 98%, at least 99%, or 100% sequence identity to the sequence in (i);
   and
(b) a light chain comprising an amino acid sequence selected from the group consisting of:
   (iv) a sequence comprising the VL sequence set forth in SEQ ID NO: 53 and the CL sequence set forth in SEQ ID NO: 84;
   (v) a sequence having a substitution, deletion or addition of one or several amino acids or any combination thereof (e.g., a substitution, deletion or addition of up to 50, up to 45, up to 40, up to 35, up to 30, up to 25, up to 20, up to 15, up to 10, or up to 5 amino acids, or any combination thereof; for example, a substitution, deletion or addition of 1, 2, 3, 4, 5, 6, 7, 8, 9 or 10 amino acids, or any combination thereof) as compared to the sequence in (iv); or
   (vi) a sequence having at least 80%, at least 85%, at least 90%, at least 91%, at least 92%, at least 93%, at least 94%, at least 95%, at least 96%, at least 97%, at least 98%, at least 99%, or 100% sequence identity to the sequence in (iv).

In certain embodiments, the substitution in (ii) or (v) is a conservative substitution.

In some embodiments, the antibody or antigen-binding fragment thereof of the invention comprises:
(a) a heavy chain comprising an amino acid sequence selected from the group consisting of:
   (i) a sequence comprising the VH sequence set forth in SEQ ID NO: 52 and the CH sequence set forth in SEQ ID NO: 82 or 83;
   (ii) a sequence having a substitution, deletion or addition of one or several amino acids or any combination thereof (e.g., a substitution, deletion or addition of up to 50, up to 45, up to 40, up to 35, up to 30, up to 25, up to 20, up to 15, up to 10, or up to 5 amino acids, or any combination thereof; for example, a substitution, deletion or addition of 1, 2, 3, 4, 5, 6, 7, 8, 9 or 10 amino acids, or any combination thereof) as compared to the sequence in (i); or
   (iii) a sequence having at least 80%, at least 85%, at least 90%, at least 91%, at least 92%, at least 93%, at least 94%, at least 95%, at least 96%, at least 97%, at least 98%, at least 99%, or 100% sequence identity to the sequence in (i);
   and
(b) a light chain comprising an amino acid sequence selected from the group consisting of:
   (iv) a sequence comprising the VL sequence set forth in SEQ ID NO: 53 and the CL sequence set forth in SEQ ID NO: 84;
   (v) a sequence having a substitution, deletion or addition of one or several amino acids or any combination thereof (e.g., a substitution, deletion or addition of up to 50, up to 45, up to 40, up to 35, up to 30, up to 25, up to 20, up to 15, up to 10, or up to 5 amino acids, or any combination thereof; for example, a substitution, deletion or addition of 1, 2, 3, 4, 5, 6, 7, 8, 9 or 10 amino acids, or any combination thereof) as compared to the sequence in (iv); or
   (vi) a sequence having at least 80%, at least 85%, at least 90%, at least 91%, at least 92%, at least 93%, at least 94%, at least 95%, at least 96%, at least 97%, at least 98%, at least 99%, or 100% sequence identity to the sequence in (iv).

In certain embodiments, the substitution in (ii) or (v) is a conservative substitution.

In some embodiments, the antibody or antigen-binding fragment thereof of the invention comprises:
(a) a heavy chain comprising an amino acid sequence selected from the group consisting of:
   (i) a sequence comprising the VH sequence set forth in SEQ ID NO: 54 and the CH sequence set forth in SEQ ID NO: 82 or 83;
   (ii) a sequence having a substitution, deletion or addition of one or several amino acids or any combination thereof (e.g., a substitution, deletion or addition of up to 50, up to 45, up to 40, up to 35, up to 30, up to 25, up to 20, up to 15, up to 10, or up to 5 amino acids, or any combination thereof; for example, a substitution, deletion or addition of 1, 2, 3, 4, 5, 6, 7, 8, 9 or 10 amino acids, or any combination thereof) as compared to the sequence in (i); or
   (iii) a sequence having at least 80%, at least 85%, at least 90%, at least 91%, at least 92%, at least 93%, at least 94%, at least 95%, at least 96%, at least 97%, at least 98%, at least 99%, or 100% sequence identity to the sequence in (i);
   and
(b) a light chain comprising an amino acid sequence selected from the group consisting of:
   (iv) a sequence comprising the VL sequence set forth in SEQ ID NO: 55 and the CL sequence set forth in SEQ ID NO: 84;
   (v) a sequence having a substitution, deletion or addition of one or several amino acids or any combination thereof (e.g., a substitution, deletion or addition of up to 50, up to 45, up to 40, up to 35, up to 30, up to 25, up to 20, up to 15, up to 10, or up to 5 amino acids, or any combination thereof; for example, a substitution, deletion or addition of 1, 2, 3, 4, 5, 6, 7, 8, 9 or 10 amino acids, or any combination thereof) as compared to the sequence in (iv); or
   (vi) a sequence having at least 80%, at least 85%, at least 90%, at least 91%, at least 92%, at least 93%, at least 94%, at least 95%, at least 96%, at least 97%, at least 98%, at least 99%, or 100% sequence identity to the sequence in (iv).

In certain embodiments, the substitution in (ii) or (v) is a conservative substitution.

In some embodiments, the antibody or antigen-binding fragment thereof of the invention comprises:
(a) a heavy chain comprising an amino acid sequence selected from the group consisting of:
   (i) a sequence comprising the VH sequence set forth in SEQ ID NO: 68 and the CH sequence set forth in SEQ ID NO: 82 or 83;
   (ii) a sequence having a substitution, deletion or addition of one or several amino acids or any combination thereof (e.g., a substitution, deletion or addition of up to 50, up to 45, up to 40, up to 35, up to 30, up to 25, up to 20, up to 15, up to 10, or up to 5 amino acids, or any combination thereof; for example, a substitution, deletion or addition of 1, 2, 3, 4, 5, 6, 7, 8, 9 or 10 amino acids, or any combination thereof) as compared to the sequence in (i); or
   (iii) a sequence having at least 80%, at least 85%, at least 90%, at least 91%, at least 92%, at least 93%, at least 94%, at least 95%, at least 96%, at least 97%, at least 98%, at least 99%, or 100% sequence identity to the sequence in (i);
   and
(b) a light chain comprising an amino acid sequence selected from the group consisting of:
   (iv) a sequence comprising the VL sequence set forth in SEQ ID NO: 69 and the CL sequence set forth in SEQ ID NO: 84;
   (v) a sequence having a substitution, deletion or addition of one or several amino acids or any combination thereof (e.g., a substitution, deletion or addition of up to 50, up to 45, up to 40, up to 35, up to 30, up to 25, up to 20, up to 15, up to 10, or up to 5 amino acids, or any combination thereof; for example, a substitution, deletion or addition of 1, 2, 3, 4, 5, 6, 7, 8, 9 or 10 amino acids, or any combination thereof) as compared to the sequence in (iv); or
   (vi) a sequence having at least 80%, at least 85%, at least 90%, at least 91%, at least 92%, at least 93%, at least 94%, at least 95%, at least 96%, at least 97%, at least 98%, at least 99%, or 100% sequence identity to the sequence in (iv).

In certain embodiments, the substitution in (ii) or (v) is a conservative substitution.

In certain embodiments, the antibody of the invention comprises: a heavy chain comprising a VH set forth in SEQ ID NO: 1 and a heavy chain constant region (CH) set forth in SEQ ID NO: 83, and a light chain comprising a VL set forth in SEQ ID NO: 2 and a light chain constant region (CL) set forth in SEQ ID NO: 84.

In certain embodiments, the antibody of the invention comprises: a heavy chain comprising a VH set forth in SEQ ID NO: 16 and a heavy chain constant region (CH) set forth in SEQ ID NO: 83, and a light chain comprising a VL set forth in SEQ ID NO: 18 and a light chain constant region (CL) set forth in SEQ ID NO: 84.

In certain embodiments, the antibody of the invention comprises: a heavy chain comprising a VH set forth in SEQ ID NO: 17 and a heavy chain constant region (CH) set forth in SEQ ID NO: 83, and a light chain comprising a VL set forth in SEQ ID NO: 19 and a light chain constant region (CL) set forth in SEQ ID NO: 84.

In certain embodiments, the antibody of the invention comprises: a heavy chain comprising a VH set forth in SEQ ID NO: 20 and a heavy chain constant region (CH) set forth in SEQ ID NO: 83, and a light chain comprising a VL set forth in SEQ ID NO: 21 and a light chain constant region (CL) set forth in SEQ ID NO: 84.

In certain embodiments, the antibody of the invention comprises: a heavy chain comprising a VH set forth in SEQ ID NO: 34 and a heavy chain constant region (CH) set forth in SEQ ID NO: 83, and a light chain comprising a VL set forth in SEQ ID NO: 35 and a light chain constant region (CL) set forth in SEQ ID NO: 84.

In certain embodiments, the antibody of the invention comprises: a heavy chain comprising a VH set forth in SEQ ID NO: 36 and a heavy chain constant region (CH) set forth in SEQ ID NO: 83, and a light chain comprising a VL set forth in SEQ ID NO: 37 and a light chain constant region (CL) set forth in SEQ ID NO: 84.

In certain embodiments, the antibody of the invention comprises: a heavy chain comprising a VH set forth in SEQ ID NO: 51 and a heavy chain constant region (CH) set forth in SEQ ID NO: 83, and a light chain comprising a VL set forth in SEQ ID NO: 53 and a light chain constant region (CL) set forth in SEQ ID NO: 84.

In certain embodiments, the antibody of the invention comprises: a heavy chain comprising a VH set forth in SEQ ID NO: 52 and a heavy chain constant region (CH) set forth in SEQ ID NO: 83, and a light chain comprising a VL set forth in SEQ ID NO: 53 and a light chain constant region (CL) set forth in SEQ ID NO: 84.

In certain embodiments, the antibody of the invention comprises: a heavy chain comprising a VH set forth in SEQ ID NO: 54 and a heavy chain constant region (CH) set forth in SEQ ID NO: 83, and a light chain comprising a VL set forth in SEQ ID NO: 55 and a light chain constant region (CL) set forth in SEQ ID NO: 84.

In certain embodiments, the antibody of the invention comprises: a heavy chain comprising a VH set forth in SEQ ID NO: 68 and a heavy chain constant region (CH) set forth in SEQ ID NO: 83, and a light chain comprising a VL set forth in SEQ ID NO: 69 and a light chain constant region (CL) set forth in SEQ ID NO: 84.

In certain embodiments, the antibody of the invention is a chimeric or humanized antibody. In certain embodiments, the antibody or antigen-binding fragment thereof of the invention is selected from the group consisting of scFv, Fab, Fab', (Fab')₂, Fv fragments, disulfide linked Fv (dsFv), a diabody, a bispecific antibody, and a multispecific antibody.

In certain embodiments, the antibody or antigen-binding fragment thereof of the invention may exhibit at least one of the following properties:
(a) binding to PD-1 (e.g., human PD-1) with a KD of less than 50 nM, e.g., less than 40 nM, 30 nM, 20 nM, 10 nM, 1 nM, 0.1 nM, 1 pM, 0.1 pM or less, wherein the KD can be measured by techniques well known in the art, such as Bio-Layer Interferometry (BLI) (e.g., ForteBio Octet ^{®}) or ELISA;
(b) binding to PD-1 (e.g., human PD-1) with an EC₅₀ of less than 50 nM, e.g., less than 40 nM, 30 nM, 20 nM, 10 nM, 1 nM, 0.9 nM, 0.8 nM, 0.7 nM, 0.6 nM, 0.5 nM, 0.4 nM, 0.3 nM, 0.2 nM, 0.1 nM, 0.01 nM, 1 pM, 0.1 pM or less, wherein the EC₅₀ can be measured by techniques well known in the art, such as flow cytometry or cell competitive ELISA;
(c) competing with PD-1 for binding to PD-L1 or PD-L2 with an IC₅₀ of from about 0.1 ng/mL to about 1000 ng/mL; preferably, the IC₅₀ is measured by ELISA;
(d) increasing T cell activation, e.g., increasing IFN-γ and/or IL-2 expression in T lymphocytes;
(e) not binding to BTLA, PD-L1 or ICOS;
(g) killing or inhibiting the growth of tumor cells;
(g) having good thermal stability; or
(h) having no or reduced activity of at least one of ADCP, ADCC and CDC.

In certain embodiments, the antibody or antigen-binding fragment thereof of the invention has reduced ADCP activity.

In certain embodiments, the antibody or antigen-binding fragment thereof of the invention has reduced ADCC activity.

In certain embodiments, the antibody or antigen-binding fragment thereof of the invention has reduced CDC activity.

In certain embodiments, the antibody or antigen-binding fragment thereof of the invention does not have CDC activity.

In certain embodiments, the antibody or antigen-binding fragment thereof of the invention does not have ADCP activity.

In certain embodiments, the antibody or antigen-binding fragment thereof of the invention does not have ADCC activity.

In certain embodiments, the antibody or antigen-binding fragment thereof of the invention has reduced activity of one, two, or all of ADCP, ADCC, and CDC activities.

In certain embodiments, the antibody or antigen-binding fragment thereof of the invention does not have one, both, or all of ADCP, ADCC, and CDC activities.

### Derivatives of the antibody

The antibody or antigen-binding fragment thereof of the invention may be derivatized, for example, by linked to an additional molecule (e.g., an additional polypeptide or protein). Typically, derivatization (e.g., labeling) of an antibody or antigen-binding fragment thereof does not adversely affect its binding to PD-1 (particularly human PD-1). Thus, the antibody or antigen-binding fragment thereof of the invention also intends to include such derivative forms. For example, the antibody or antigen-binding fragment thereof of the invention may be linked to (by chemical coupling, gene fusion, non-covalently or other manners) one or more other molecular groups, e.g., another antibody (e.g. forming a bispecific antibody), a detection agent, a pharmaceutical reagent, and/or a protein or polypeptide capable of mediating the binding of the antibody or the antigen-binding fragment to another molecule (e.g. avidin or polyhistidine tag).

One type of derivative antibody (e.g., a bispecific antibody) is generated by cross-linking two or more antibodies (of same or different types). A method for obtaining a bispecific antibody is well known in the art and exemplary methods include, but not limited to, chemical cross-linking, cell engineering (hybridoma) or genetic engineering.

Another type of derivative antibody is a labeled antibody. For example, the antibody or antigen-binding fragment thereof of the invention may be linked to a detectable marker. The detectable marker of the invention may be any substance to be detected by fluorescence, spectroscopy, photochemistry, biochemistry, immunology, electricity, optics or chemistry. Such markers are well known in the art and examples thereof include, but not limited to, enzymes (e.g. horseradish peroxidase, alkaline phosphatase, beta-galactosidase, urease, glucose oxidase, etc.), radionuclides (e.g.,³H, ¹²⁵I, ³⁵S, ¹⁴C or ³²P), fluorescent dyes (e.g., fluorescein isothiocyanate (FITC), fluorescein, tetramethyl rhodamine isothiocyanate (TRITC), phycoerythrin (PE), Texas Red, rhodamine, quantum dots or cyanine dye derivatives (e.g., Cy7, Alexa 750)), acridine ester compounds, magnetic beads (e.g., Dynabeads^{®}), calorimetric markers such as colloidal gold or colored glass or plastic (e.g. polystyrene, polypropylene, latex, etc.) beads, and biotin for binding to avidin (e.g. streptavidin) modified by the above markers. Such markers are disclosed in patents including, but not limited to, U.S. Pat. 3,817,837; 3,850,752; 3,939,350; 3,996,345; 4,277,437; 4,275,149; and 4,366,241, all of which are incorporated herein by reference. The detectable marker described above can be detected by a method known in the art. For example, a radioactive marker may be detected using a photographic film or a scintillation calculator, and a fluorescent marker may be detected using a photodetector to detect the emitted light. An enzyme marker is generally detected by providing a substrate to the enzyme and detecting the reaction product produced by the effect of enzyme on the substrate, and a calorimetric marker is detected by a simple visual coloring marker. In certain embodiments, such markers are useful in immunoassays (e.g., enzyme-linked immunoassay, radioimmunoassay, fluorescence immunoassay, chemiluminescence immunoassay, etc.). In some embodiments, the detectable markers described above may be linked to the antibody or antigen-binding fragment thereof of the invention through linkers of different lengths to reduce potential steric hindrance.

In addition, the antibody or antigen-binding fragment thereof of the invention may also be derivatized with a chemical group, such as polyethylene glycol (PEG), methyl or ethyl, or glycosyl. These groups can be used to improve the biological characteristics of the antibody, such as increase the serum half-life.

Therefore, in one aspect, the invention provides a conjugate comprising the monoclonal antibody or antigen-binding fragment thereof of the invention and a coupling moiety which may be a detectable marker as described above, such as a radioisotope, a fluorescent substance, a luminescent substance, a colored substance, or an enzyme. The coupling moiety may also be an agent, such as a chemotherapeutics, a radionuclide or toxin, such as paclitaxel, cyclophosphamide, fluorouracil, 89Sr, duocarmycin.

As one of the derivatives of the antibody, the invention provides a multispecific antibody comprising a first antibody or fragment thereof, and an additional antibody or fragment thereof, or an antibody mimetic, wherein the first antibody or fragment thereof, the additional antibody or fragment thereof, or the antibody mimetic retains its original binding specificity. The first antibody or fragment thereof is any (monoclonal) antibody or antigen-binding fragment thereof of the invention which specifically binds to PD-1. As used herein, "antibody mimetic" refers to specifically binds to an antigen as an antibody, but without an antibody structure. They are usually artificial peptides or proteins having a molar mass of about 3 to 20 kDa, e.g. a designed ankyrin repeat protein (DARPin) and fynomer. The designed ankyrin repeat protein (DARPin) can be linked to an IgG antibody, a scFv-Fc antibody fragment or combination thereof, as described in CN104341529A. An anti-IL-17a fynomer binds to an anti-IL-6R antibody, as described in WO2015141862A1.

In certain embodiments, the multispecific antibody is formed by coupling the first antibody or antigen-binding fragment thereof with an additional antibody or antigen-binding fragment thereof or an antibody mimetic, wherein each antibody or antigen-binding fragment thereof or antibody analog retains its original binding specificity, and wherein the first antibody or antigen-binding fragment thereof is the antibody or antigen-binding fragment thereof of the invention. In certain embodiments, the multispecific antibody is a bispecific antibody or a trispecific antibody or a tetraspecific antibody.

### Antibody preparation

The antibody of the invention can be prepared by various methods known in the art, e.g., genetic engineering recombinant technology. For example, obtaining a DNA molecule encoding the heavy and light chain genes of the antibody of the invention by chemical synthesis or PCR amplification, inserting the obtained DNA molecule into an expression vector and then transfecting a host cell; then, culturing the transfected host cell under particular conditions to express the antibody of the invention.

The antigen-binding fragments of the invention can be obtained by hydrolyzing an intact antibody molecule (See Morimoto et al., J. Biochem. Biophys. Methods 24: 107-117 (1992) and Brennan et al., Science 229: 81 (1985)). In addition, these antigen-binding fragments can also be produced directly by recombinant host cells (reviewed in Hudson, Curr. Opin. Immunol. 11: 548-557 (1999); Little et al., Immunol. Today, 21: 364-370 (2000)). For example, Fab' fragments can be obtained directly from host cells; Fab' fragments can be chemically coupled to form F (ab')₂ fragments (Carter et al., Bio/Technology, 10: 163-167 (1992)). Furthermore, Fv, Fab or F (ab')₂ fragments can also be isolated directly from the recombinant host cell culture medium. Other technologies for preparing these antigen-binding fragments are well known to those of ordinary skill in the art.

Thus, in another aspect, the invention provides an isolated nucleic acid molecule comprising a nucleotide sequence encoding the antibody or antigen-binding fragment thereof of the invention, or a heavy chain variable region and/or a light chain variable region thereof, or one or more CDRs thereof. In some embodiments, the nucleotide sequence may be substituted in accordance with the codon degeneracy which is known in the art. In certain embodiments, the nucleotide sequence is codon optimized.

In certain embodiments, the isolated nucleic acid molecule of the invention comprises: (i) a first nucleic acid and a second nucleic acid encoding a heavy chain variable region and a light chain variable region of the antibody or antigen-binding fragment thereof of the invention, respectively, or (ii) a first nucleic acid encoding a heavy chain variable region and a heavy chain constant region, and a second nucleic acid encoding a light chain variable region and a light chain constant region, respectively, of the antibody or antigen-binding fragment thereof of the invention, or (iii) a first nucleic acid and a second nucleic acid encoding a heavy chain and a light chain of the antibody or antigen-binding fragment thereof of the invention, respectively. In certain embodiments, the first and second nucleic acids comprise a nucleic acid of a degenerate sequence of, or a substantially identical sequence to, any one of the first and second nucleic acids in the above (i)-(iii). The degenerate sequence or substantially identical sequence refers to a sequence having at least about 85%, 90%, 95%, 99% or greater sequence identity or having one or more nucleotide substitutions or differing by not more than 3, 6, 15, 30 or 45 nucleotides, as compared to the nucleic acid molecules in (i)-(iii). For example, the isolated nucleic acid molecule may comprise a nucleotide sequence set forth in SEQ ID NO: 85, SEQ ID NO: 87, SEQ ID NO: 89, SEQ ID NO: 46 or a degenerate sequence thereof or a sequence substantially identical thereto.

In certain embodiments, the invention provides an isolated nucleic acid molecule, comprising a nucleic acid molecule encoding a heavy chain of antibody, and/or a nucleic acid molecule encoding a light chain of antibody, wherein the nucleic acid molecule encoding the heavy chain of antibody has a sequence selected from the group consisting of: (a) a nucleotide sequence set forth in SEQ ID NO: 85 or a degenerate sequence thereof, or (b) a sequence substantially identical to the nucleotide sequence of (a) (e.g., a sequence having at least about 85%, 90%, 95%, 99% or greater sequence identity or having one or more nucleotide substitutions compared to the nucleotide sequence of (a)), or (c) a sequence which differs by not more than 3, 6, 15, 30 or 45 nucleotides from the nucleotide sequence of (a); and/or, the nucleic acid molecule encoding the light chain of antibody has a sequence selected from the group consisting of: (d) a nucleotide sequence set forth in SEQ ID NO: 87 or a degenerate sequence thereof, or (e) a sequence substantially identical to the nucleotide sequence of (d) (e.g. a sequence having at least about 85%, 90%, 95%, 99% or greater sequence identity or having one or more nucleotide substitutions compared to the nucleotide sequence of (d)), or (f) a sequence which differs by not more than 3, 6, 15, 30 or 45 nucleotides from the nucleotide sequence of (d).

In certain embodiments, the isolated nucleic acid molecule of the invention comprises a nucleic acid molecule encoding a heavy chain of antibody set forth in SEQ ID NO: 85, and/or a nucleic acid molecule encoding a light chain of antibody set forth in SEQ ID NO: 87.

In certain embodiments, the invention provides an isolated nucleic acid molecule, comprising a nucleic acid molecule encoding a heavy chain of antibody, and/or a nucleic acid molecule encoding a light chain of antibody, wherein the nucleic acid molecule encoding a heavy chain of antibody has a sequence selected from the group consisting of: (a) a nucleotide sequence set forth in SEQ ID NO: 89 or a degenerate sequence thereof, or (b) a sequence substantially identical to the nucleotide sequence of (a) (e.g., a sequence having at least about 85%, 90%, 95%, 99% or greater sequence identity or having one or more nucleotide substitutions compared to the nucleotide sequence of (a)), or (c) a sequence which differs by not more than 3, 6, 15, 30 or 45 nucleotides from the nucleotide sequence of (a); and/or, the nucleic acid molecule encoding a light chain of antibody has a sequence selected from the group consisting of: (d) a nucleotide sequence set forth in SEQ ID NO: 46 or a degenerate sequence thereof, or (e) a sequence substantially identical to the nucleotide sequence of (d) (e.g. a sequence having at least about 85%, 90%, 95%, 99% or greater sequence identity or having one or more nucleotide substitutions compared to the nucleotide sequence of (d)), or (f) a sequence which differs by not more than 3, 6, 15, 30 or 45 nucleotides from the nucleotide sequence of (d).

In certain embodiments, the isolated nucleic acid molecule of the invention comprises a nucleic acid molecule encoding a heavy chain of antibody set forth in SEQ ID NO: 89, and/or a nucleic acid molecule encoding a light chain of antibody set forth in SEQ ID NO: 46.

In another aspect, a vector (e.g., a cloning vector or an expression vector) comprising the isolated nucleic acid molecule of the invention is provided. In certain embodiments, the vector of the invention is, for example, a plasmid, a cosmid, a phage, a lentivirus, or the like. In certain embodiments, the vector is capable of expressing the antibody or antigen-binding fragment thereof of the invention in vivo in a subject (e.g., a mammal, e.g., a human).

In another aspect, a host cell comprising the isolated nucleic acid molecule of the invention or a vector of the invention is provided. The host cell may be a eukaryotic cell (e.g., a mammalian cell, an insect cell, a yeast cell) or a prokaryotic cell (e.g., Escherichia coli). Suitable eukaryotic cells include, but not limited to, NS0 cells, Vero cells, Hela cells, COS cells, CHO cells, HEK293 cells, BHK cells, and MDCKII cells. Suitable insect cells include, but not limited to, Sf9 cells. In certain embodiments, the host cell of the invention is a mammalian cell, such as CHO (e.g., CHO-K1, CHO-S, CHO DXB11, CHO DG44).

In another aspect, a method for preparing the antibody or antigen-binding fragment thereof of the invention is provided, comprising culturing the host cell of the invention under conditions allowing for expression of the antibody or antigen-binding fragment thereof, and recovering the antibody or antigen-binding fragment thereof from the cultured host cell culture.

In another aspect, one or more chimeric antigen receptors (CAR) and cells carrying or expressing the CAR are provided, the cells carrying or expressing the CAR comprising or expressing any antibody or antigen-binding fragment (e.g., scFv), nucleic acid or vector of the invention.

The cell receiving CAR is called as host cell. A host cell carrying or expressing CAR is called as CAR cell. In certain embodiments, the host cell is selected from an immune cell, preferably, the immune cell is selected from the group consisting of T lymphocytes, NK cells, monocytes, macrophages or dendritic cells, and any combination thereof.

Preparation of CAR cells can be referred to the technologies or conditions described in the literatures in the art, for example, see Sambrook et al., Molecular Cloning: A Laboratory Manual, Third Edition (translated by Huang Peitang et al.), or can be carried out according to the product instructions.

In another aspect, an oncolytic virus comprising the isolated nucleic acid molecule of the invention or a vector of the invention is provided.

It is characterized in that a nucleic acid molecule encoding one or several antibodies or antigen-binding fragments thereof of the invention is inserted or carried into the oncolytic virus. The oncolytic virus carrying the nucleic acid molecule of the invention expresses one or more anti-PD-1 antibodies or antigen-binding fragments thereof.

Preparation of a modified oncolytic virus can be referred to the technologies or conditions described in the literatures in the art, for example, see Sambrook et al., Molecular Cloning: A Laboratory Manual, Third Edition (translated by Huang Peitang et al.), or can be carried out according to the product instructions.

The oncolytic virus carrying the nucleic acid molecule of the invention can be effective in delivering an anti-PD-1 antibody or antigen-binding fragment thereof, realizing the function of expressing the anti-PD-1 antibody or antigen-binding fragment thereof in vivo while realizing the virus function of lysing tumor cells, thus playing dual roles in both lysing tumor cells and enhancing anti-tumor immunity. Therefore, the oncolytic virus modified by the invention can be used to prepare a medicament for preventing and/or treating tumors; the oncolytic virus modified by the invention can be used to prevent and/or treat tumors.

In another aspect, a fusion protein comprising the monoclonal antibody or antigen-binding fragment thereof of the invention and a non-immunoglobulin portion which is a polypeptide such as a cytokine (e.g., IL-2, IL-15, IL-7, IL-12, IL-18, IL-21, TNF-α, or IFNγ) is provided.

### Use, therapeutic method, and pharmaceutical composition

In another aspect, the invention provides a pharmaceutical composition comprising the antibody or antigen-binding fragment thereof, the nucleic acid, the vector, the host cell, the CAR cell, the multispecific antibody, the conjugate, the oncolytic virus or the fusion protein of the invention, and a pharmaceutically acceptable carrier and/or excipient.

In certain embodiments, the pharmaceutical composition of the invention comprises the antibody or antigen-binding fragment thereof of the invention, and a pharmaceutically acceptable carrier and/or excipient.

In certain embodiments, the pharmaceutical composition of the invention comprises the host cell of the invention, which comprises an isolated nucleic acid molecule or vector described above, and a pharmaceutically acceptable carrier and/or excipient.

In certain embodiments, the pharmaceutical composition of the invention comprises the multispecific antibody of the invention, and a pharmaceutically acceptable carrier and/or excipient.

In certain embodiments, the pharmaceutical composition of the invention comprises the conjugate of the invention, and a pharmaceutically acceptable carrier and/or excipient.

In certain embodiments, the pharmaceutical composition of the invention comprises the CAR cell of the invention, and a pharmaceutically acceptable carrier and/or excipient.

In certain embodiments, the pharmaceutical composition of the invention comprises the oncolytic virus of the invention, and a pharmaceutically acceptable carrier and/or excipient.

In certain embodiments, the pharmaceutical composition of the invention comprises the fusion protein of the invention, and a pharmaceutically acceptable carrier and/or excipient.

In certain embodiments, the pharmaceutical composition may also comprise an additional pharmaceutical active agent. In certain embodiments, the additional pharmaceutically active agent is a chemotherapeutic, an anti-tumor agent, an anti-infection agent, or an anti-autoimmune disease agent, an anti-angiogenesis agent, or a tyrosine kinase inhibitor.

In certain embodiments, the antibody or antigen-binding fragment thereof of the invention and the additional pharmaceutically active agent are provided as separate components or as components in the same composition. Thus, the antibody or antigen-binding fragment thereof of the invention can be administered in combination or separately, simultaneously, or sequentially with the additional pharmaceutically active agent.

In certain embodiments, the pharmaceutical composition may also comprise an additional pharmaceutical active agent. The additional pharmaceutically active agent is selected from the group consisting of an anti-tumor agent, a radiopharmaceutical, an agent associated with an immune-regulatory effect, or an agent associated with an autoimmune disease. The anti-tumor agent comprises a chemotherapeutic agent, an antibody associated with an immune-regulatory effect, or an anti-tumor biomacromolecule agent. The chemotherapeutic agent comprises an anti-angiogenesis agent or a tyrosine kinase inhibitor. In certain embodiments, the chemotherapeutic agent includes, but not limited to: for example, 5-fluorouracil, actinomycin-D, daunorubicin, mitomycin, vincristine and so on, or any combination thereof.

In certain embodiments, the radiopharmaceutical includes, but not limited to, carbon-11, carbon-14, chromium-51, cobalt-57, cobalt-58, erbium-169, fluorine-18, gallium-67, gold-198, indium-111, indium-113m, iodine-123, iodine-125, iodine-131, and the like.

In certain embodiments, the antibody associated with an immune-regulatory effect refers to an antibody useful in preventing, inhibiting, and/or treating a tumor, such as an anti-CTLA-4 antibody, an anti-HER2 antibody, an anti-EGFR antibody, an anti-LAG-3 antibody, or a TIM-3 antibody, and the like; preferably, the anti-LAG-3 antibody is AB12T8 (Sichuan Kelun Biotech Biopharmaceutical Co., Ltd., WO2019141092); and the TIM-3 antibody is AB12S3 (Sichuan Kelun Biotech Biopharmaceutical Co., Ltd., WO2019206095).

In certain embodiments, the agent associated with an immune-regulatory effect refers to an agent useful in preventing, inhibiting and/or treating inflammation or infection, or having an immune-regulatory effect, such as an anti-IL-1 antibody, an anti-IL-6 antibody, an anti-TNF-a antibody, and the like.

In certain embodiments, the agent associated with an autoimmune disease refers to an agent useful in preventing, inhibiting and/or treating an autoimmune disease, such as an anti-IL-1 antibody, an anti-IL-6 antibody, an anti-TNF-a antibody, an anti-CD20 antibody, an anti-β-lymphocyte stimulator (BLyS) antibody, and the like.

In certain embodiments, the tyrosine kinase inhibitor includes, but not limited to, imatinib, sorafenib, sunitinib, lapatinib, dasatinib.

In certain embodiments, the pharmaceutical composition is administered concurrently, separately, or sequentially with other treatments or therapeutic agents, such as before, simultaneously with, or after tumor treatment.

In another aspect, the antibody or antigen-binding fragment thereof, nucleic acid, vector, host cell, CAR cell, multispecific antibody, oncolytic virus, fusion protein or conjugate in the pharmaceutical composition of the invention is sufficient to produce at least one of the following biological activities in a subject:
(1) inhibiting or blocking the binding of PD-1 to PD-L1 or PD-L2,
(2) down-regulating or eliminating the activity of PD-1,
(3) reducing or relieving the immunosuppression induced by PD-1 or PD-L1 or PD-L2,
(4) increasing the production of IFNy or IL-2 in T lymphocytes,
(5) enhancing the ability of T lymphocytes to kill tumor cells.

In another aspect, the invention provides the use of the antibody or antigen-binding fragment thereof, the nucleic acid, the vector, the host cell, the CAR cell, the multispecific antibody, the conjugate, the oncolytic virus, or the fusion protein of the invention in the preparation of a medicament for:
(1) inhibiting or blocking the binding of PD-1 to PD-L1 or PD-L2,
(2) down-regulating or eliminating the activity of PD-1,
(3) reducing or relieving the immunosuppression induced by PD-1 or PD-L1 or PD-L2,
(4) increasing the production of IFNy or IL-2 in T lymphocytes, and/or
(5) enhancing the ability of T lymphocytes to kill tumor cells.

In certain embodiments, when used for preparing a medicament, the host cell of the invention comprises the isolated nucleic acid molecule or the vector described above.

In certain embodiments, when the antibody or antigen-binding fragment thereof, the nucleic acid, the vector, the host cell, the CAR cell, the multispecific antibody, the oncolytic virus, the fusion protein, the conjugate, the pharmaceutical composition, or the pharmaceutical composition of the invention in combination with an additional pharmaceutically active agent (e.g., an anti-tumor agent, a radiopharmaceutical, an agent associated with an immune-regulatory effect, or an agent associated with an autoimmune disease) is used for preparing a medicament, the medicament is used for preventing and/or treating a tumor, an infection, or an autoimmune disease in a subject (e.g., a human).

In certain embodiments, the subject is a mammal. In certain embodiments, the subject is a human.

In certain embodiments, the antibody or antigen-binding fragment thereof, the nucleic acid, the vector, the cell, the multispecific antibody, the oncolytic virus, the conjugate, the fusion protein, the agent, the pharmaceutical composition, or the pharmaceutical composition of the invention in combination with an additional pharmaceutically active agent (e.g., an anti-tumor agent, a radiopharmaceutical, an agent associated with an immune-regulatory effect, or an agent associated with an autoimmune disease) is used for preventing and/or treating a tumor. In certain embodiments, the tumor is selected from one or more in the tissues from the group consisting of: skin, bone marrow, blood, lymph, head and neck, brain, lung, breast, stomach, liver, pancreas, gallbladder, intestine, ovary, prostate, adrenal gland, kidney, bladder, uterus, cervix, testis, penis, and soft tissue.

In certain embodiments, the tumor is selected from one or more in the group consisting of lung cancer, liver cancer, ovarian cancer, cervical cancer, skin cancer, colon cancer, rectal cancer, glioma, bladder cancer, breast cancer, kidney cancer, esophageal cancer, gastric cancer, oral squamous cell cancer, urethral epithelial cell cancer and pancreatic cancer, and head and neck tumors.

In certain embodiments, the antibody or antigen-binding fragment thereof, the nucleic acid, the vector, the host cell, the CAR cell, the multispecific antibody, the oncolytic virus, the fusion protein, the conjugate, the pharmaceutical composition, or the pharmaceutical composition of the invention together with an additional pharmaceutically active agent (e.g., an anti-tumor agent, a radiopharmaceutical, an agent associated with an immune-regulatory effect, or an agent associated with an autoimmune disease) is used for preventing and/or treating a disease or condition associated with an infection. In certain embodiments the infection includes, but not limited to, a bacterial infection, a pathogen infection, a fungal infection, and a viral infection. In certain embodiments, the antibody or antigen-binding fragment thereof, the vector, the host cell, the multispecific antibody, the conjugate is used for preventing and/or treating a viral infection, such as a chronic viral infection, an infection of hepatitis B virus, hepatitis C virus, or HIV, etc., or preventing and/or treating other chronic infections, such as tuberculosis.

In certain embodiments, the antibody or antigen-binding fragment thereof, the nucleic acid, the vector, the host cell, the CAR cell, the multispecific antibody, the oncolytic virus, the fusion protein, the conjugate, the pharmaceutical composition, or the pharmaceutical composition of the invention together with an additional pharmaceutically active agent (e.g., an anti-tumor agent, a radiopharmaceutical, an agent associated with an immune-regulatory effect, or an agent associated with an autoimmune disease) is used for preventing and/or treating a disease associated with autoimmunity. In certain embodiments, the disease includes, but not limited to, hyperthyroidism, diabetes, myasthenia gravis, ulcerative colitis, gastritis, pemphigus, primary biliary cirrhosis, multiple sclerosis, lupus erythematosus, rheumatoid arthritis, and the like.

In another aspect, the invention provides a method for at least one of in vivo/in vitro (1) inhibiting or blocking the binding of PD-1 to PD-L1 or PD-L2, (2) down-regulating or eliminating the activity of PD-1, (3) reducing or relieving the immunosuppression of PD-1 or PD-L1 or PD-L2, (4) increasing the production of IFNγ or IL-2 in T lymphocytes and (5) enhancing the ability of T lymphocytes to kill tumor cells, comprising: administrating to a cell or a subject any one of the described antibody or antigen-binding fragment thereof, the nucleic acid, the vector, the host cell, the multispecific antibody, the conjugate, the pharmaceutical composition, or the pharmaceutical composition together with an additional pharmaceutically active agent (e.g., an anti-tumor agent, a radiopharmaceutical, an agent associated with an immune-regulatory effect, or an agent associated with an autoimmune disease).

Optionally, a chemotherapeutic, an anti-tumor agent, an anti-angiogenesis agent, a tyrosine kinase inhibitor, or a PD-1 pathway inhibitor is administered concurrently with, before or after administration of the antibody or antigen-binding fragment thereof, the nucleic acid, the vector, the host cell, the multispecific antibody, the conjugate, or the pharmaceutical composition.

In certain embodiments, the subject is a mammal; preferably, the subject is a human.

Thus, in another aspect, the invention provides a method for preventing and/or treating a tumor, an infection or an autoimmune disease in a subject, comprising administering to the subject in need thereof an effective amount of the antibody or antigen-binding fragment thereof, the nucleic acid, the vector, the host cell, the CAR cell, the multispecific antibody, the oncolytic virus, the conjugate, the fusion protein, the pharmaceutical composition, or the pharmaceutical composition of the invention together with an additional pharmaceutically active agent (e.g., an anti-tumor agent, a radiopharmaceutical, an agent associated with an immune-regulatory effect, or an agent associated with an autoimmune disease).

The antibody or antigen-binding fragment thereof, the nucleic acid, the vector, the host cell, the CAR cell, the multispecific antibody, the oncolytic virus, the conjugate, the fusion protein or the pharmaceutical composition of the invention can be formulated as any dosage form known in the medicines, such as tablet, pill, suspension, emulsion, solution, gel, capsule, powder, granule, elixir, lozenge, suppository, injection (including injectable solution, sterile injectable powder and concentrated injectable solution), inhalant, spray, etc. The preferred dosage form depends on the intended administration route and therapeutic use. The pharmaceutical composition of the invention should be sterile and stable under production and storage conditions, and can be prepared into an injection.

In addition, the antibody or antigen-binding fragment thereof of the invention may be present in a unit dose form in the pharmaceutical composition for convenient administration.

The pharmaceutical composition of the invention may include a "therapeutically effective amount" or a "prophylactically effective amount" of the antibody or antigen-binding fragment thereof, the nucleic acid, the vector, the host cell, the CAR cell, the multispecific antibody, the oncolytic virus, the fusion protein, or the conjugate of the invention. A "prophylactically effective amount" means an amount sufficient to prevent, stop, or delay the occurrence of a disease. A "therapeutically effective amount" means an amount sufficient to cure or at least partially prevent a disease and its complications in a patient who has suffered from the disease, for example 0.1 mg/mL to 5000 mg/mL.

In the invention, the subject may be a mammal, such as a human.

### Detection methods and kits

The antibody or antigen-binding fragment thereof of the invention is capable of specifically binding PD-1, and thus can be used to detect the presence or level of PD-1 in a sample.

Thus, in another aspect, the invention provides a kit comprising an antibody or antigen-binding fragment thereof of the invention. In certain embodiments, the antibody or antigen-binding fragment thereof of the invention carries a detectable marker. In a preferred embodiment, the kit further comprises a second antibody that specifically recognizes the antibody or antigen-binding fragment thereof of the invention. Preferably, the second antibody further comprises a detectable marker.

In the invention, the detectable marker may be any substance detected by fluorescence, spectroscopy, photochemistry, biochemistry, immunology, electricity, optics, or chemistry. Especially preferred is that such markers are suitable for immunoassays (e.g., enzyme-linked immunoassay, radioimmunoassay, fluorescence immunoassay, chemiluminescence immunoassay, etc.). Such markers are well known in the art and include, but not limited to, enzymes (e.g. horseradish peroxidase, alkaline phosphatase, β-galactosidase, urease, glucose oxidase, etc.), radionuclides (e.g., ³H, ¹²⁵I, ³⁵S, ¹⁴C or ³²P), fluorescent dyes (e.g., fluorescein isothiocyanate (FITC), fluorescein, tetramethyl rhodamine isothiocyanate (TRITC), phycoerythrin (PE), Texas Red, rhodamine, quantum dots or cyanine dye derivatives (e.g., Cy7, Alexa 750)), acridine ester compounds, magnetic beads (e.g., Dynabeads^{®}), calorimetric markers such as colloidal gold or colored glass or plastic (e.g. polystyrene, polypropylene, latex, etc.) beads, and biotin used to bind avidin (e.g. streptavidin) modified by the above markers . The use of such markers was taught in the patents including, but not limited to, U.S. Pat. 3,817,837; 3,850,752; 3,939,350; 3,996,345; 4,277,437; 4,275,149; and 4,366,241, all of which are incorporated herein by reference. Markers encompassed by the invention can be detected by a method known in the art. For example, a radioactive marker may be detected using a photographic film or a scintillation calculator, and a fluorescent marker may be detected using a photodetector to detect the emitted light. An enzyme marker may be generally detected by providing a substrate for the enzyme and detecting the reaction product produced by the effect of the enzyme on the substrate, and a calorimetric marker may be detected by a simple visual coloring marker. In some embodiments, the detectable markers described above may be linked to the recombinant protein of the invention via linkers of different lengths to reduce the potential steric hindrance.

In another aspect, the invention provides a method for detecting the presence or level of PD-1 in a sample, comprising the step of utilizing the antibody or antigen-binding fragment thereof of the invention. In a preferred embodiment, the antibody or antigen-binding fragment thereof of the invention still carries a detectable marker. In another preferred embodiment, the method further comprises detecting an antibody or antigen-binding fragment thereof of the invention using an agent with a detectable marker. The method may be used for a diagnostic purpose or a non-diagnostic purpose (e.g., PD-1/PD-L1 pathway studies, drug screening, histochemical analysis, etc.). In certain embodiments, the sample for a non-diagnostic purpose is a cell sample, such as a cell line or an ex vivo cell culture.

In another aspect, the invention provides a method for detecting the presence or level of PD-1 in a sample, comprising contacting the sample with the antibody or antigen-binding fragment thereof of the invention under conditions allowing for the formation of a complex between the antibody or antigen-binding fragment thereof and PD-1, and detecting the formation of the complex.

In another aspect, use of the antibody or antigen-binding fragment thereof of the invention for preparing a kit for detecting the presence or level of PD-1 in a sample is provided. In another aspect, the invention provides a diagnostic or therapeutic kit comprising one or more of the antibody or antigen-binding fragment thereof, the nucleic acid, the vector, the host cell, the multispecific antibody, the conjugate, the fusion protein or the pharmaceutical composition of the invention. Optionally, the diagnostic or therapeutic kit further includes instructions for using the same.

The antibody or antigen-binding fragment of the invention has a high binding affinity to PD-1 and has a strong specificity. Therefore, the antibody or antigen-binding fragment of the invention is suitable for preventing and/or treating a tumor, an infection, or an autoimmune disease. The humanized and IgG1-1D mutant anti-PD-1 humanized antibody of the invention not only retains the functions and properties of the parent murine antibody, but also has a higher humanization degree, so it can be administered safely to a human subject without triggering an immunogenic reaction. Therefore, the antibody or antigen-binding fragment of the invention is of great clinical value.

### Definitions of terms

In the invention, unless defined otherwise, all scientific and technical terms used herein have the same meaning as those commonly understood by one of ordinary skill in the art to which this invention belongs. In addition, the cell culture, biochemistry, nucleic acid chemistry, immunology laboratory and other operation steps used herein are conventional steps widely used in corresponding fields. Meanwhile, in order to better understand the invention, definitions and explanations of related terms are provided below.

There is no rule of singular and plural in Chinese grammar, the nouns used herein refer to both singular and plural, which can be particularly determined according to the context, therefore, when the nouns used herein are translated into English, "one or more" can be preceded.

As used herein, the term "antibody" refers to an immunoglobulin molecule that typically consists of two pairs of polypeptide chains, each having a light chain (LC) and a heavy chain (HC). Light chains are classified as either kappa (κ) or lambda (λ) light chains. Heavy chains can be classified as µ, δ, γ, α, or ε, and the isotypes of antibody can be defined as IgM, IgD, IgG, IgA and IgE, respectively. Within light and heavy chains, variable and constant regions are joined by a "J" segment of about 12 or more amino acids, the heavy chain also comprising a "D" segment of about 3 or more amino acids. Each heavy chain consists of a heavy chain variable region (VH) and a heavy chain constant region (CH). The heavy chain constant region consists of three domains (CHI, CH2 and CH3). Each light chain consists of a light chain variable region (VL) and a light chain constant region (CL). The light chain constant region consists of one domain CL. The constant domain is not directly involved in the binding of an antibody to an antigen, but exhibits various effector functions, such as mediating the binding of an immunoglobulin to a host tissue or factor, including various cells of the immune system (e.g., effector cells) and a first component (C1q) of the classical complement system. The VH and VL regions can also be subdivided into hypervariable regions (called complementary determining region (CDR)) interspersed with relatively conservative regions called framework regions (FR). Each VH and VL consists of three CDRs and four FRs arranged from amino terminal to carboxyl terminal in the following order: FR1, CDR1, FR2, CDR2, FR3, CDR3, FR4. The variable regions (VH and VL) of each heavy chain/light chain pair form antigen-binding sites, respectively. The distribution of amino acids in various regions or domains may follow the definitions described in Kabat, Sequences of Proteins of Immunological Interest (National Institutions of Health, Bethesda, Md. (1987 and 1991)), or Chothia & Lesk (1987) J. Mol. Biol. 196: 901-917; Chothia et al. (1989) Nature 342: 878-883.

As used herein, the term "complementary determining region" or "CDR" refers to amino acid residues in the variable region of an antibody which are responsible for antigen binding. The precise boundaries of these amino acid residues may be defined according to various numbering systems known in the art, such as the definitions in the Kabat numbering system (Kabat et al., Sequences of Proteins of Immunological Interest, 5th Ed. Public Health Service, National Institutions of Health, Bethesda, Md., 1991), the Chothia numbering system (Chothia & Lesk (1987)) J. Mol. Biol. 196: 901-917; Chothia et al. (1989) Nature 342: 878-883) or the IMGT numbering system (Lefranc et al., Dev. Comparat. Immunol. 27: 55-77, 2003). For a given antibody, a person skilled in the art will readily identify CDRs defined by each numbering system. Also, the correspondence between different numbering systems is well known to those skilled in the art (see, for example, Lefranc et al., Dev. Comparat. Immunol. 27: 55-77, 2003).

In the invention, the CDRs contained in the antibody or antigen-binding fragment thereof of the invention may be determined according to various numbering systems known in the art. In certain embodiments, the CDRs contained in the antibody or antigen-binding fragment thereof of the invention are preferably determined by a Kabat or IMGT numbering system.

As used herein, the term "framework region" or "FR" residues refer to those amino acid residues in the variable region of an antibody other than the CDR residues as defined above.

As used herein, the term "germline antibody gene" is an immunoglobulin sequence encoded by a non-lymphocyte that does not undergo maturation processes of genetic rearrangement and maturation leading to the expression of a specific immunoglobulin. An advantage provided by various embodiments of the invention is derived from a consensus that germline antibody genes retain more important amino acid sequence structures showing characteristics of individual animal species than mature antibody genes. Therefore, when applied therapeutically to this species, it is less recognized as an exogenous substance by this species.

The term "antibody" is not limited by any particular method for producing the same. For example, it includes recombinant antibodies, monoclonal antibodies, and polyclonal antibodies. The antibody may be an antibody of different isotypes, such as an IgG (e.g., an IgG1, IgG2, IgG3, or IgG4 subtype), IgA1, IgA2, IgD, IgE, or IgM antibody.

As used herein, the term "antigen-binding fragment" of an antibody refers to a polypeptide fragment of an antibody, such as a polypeptide fragment of a full-length antibody, which retains the ability to specifically bind to the same antigen as bound by the full-length antibody and/or competes with the full-length antibody for specific binding to the antigen, which is also known as an "antigen-binding part". In general, see Fundamental Immunology, Ch. 7 (Paul, W., ed., 2nd Edition, Raven Press, N.Y. (1989), which is incorporated herein by reference in its entirety for all purposes. Antigen-binding fragments of an antibody can be produced by recombinant DNA technologies or by enzymatic or chemical cleavage of an intact antibody. Non-limiting examples of antigen-binding fragments include Fab, Fab', F (ab')₂, Fd, Fv, dAb and complementary determining region (CDR) fragments, single chain antibody (e.g. scFv), chimeric antibody, diabody, linear antibody, nanobody (e.g. by technology from Ablynx), domain antibody (e.g. by technology from Domantis), and a polypeptide comprising at least a portion of an antibody sufficient to confer the specific antigen-binding ability to the polypeptide. Engineering modified antibody variants are reviewed in Holliger et al., 2005; Nat Biotechnol, 23: 1126-1136.

As used herein, the term "full-length antibody" means an antibody consisting of two "full-length heavy chains" and two "full-length light chains". Wherein, "full-length heavy chain" refers to a polypeptide chain which, from N-terminal to C-terminal, consists of a heavy chain variable region (VH), a heavy chain constant region CH1 domain, a hinge region (HR), a heavy chain constant region CH2 domain and a heavy chain constant region CH3 domain; and, when the full-length antibody is an IgE isotype, it optionally further includes a heavy chain constant region CH4 domain. Preferably, a "full-length heavy chain" is a polypeptide chain consisting of VH, CHI, HR, CH2, and CH3 from N-terminal to C-terminal. A "full-length light chain" is a polypeptide chain consisting of a light chain variable region (VL) and a light chain constant region (CL) from N-terminal to C-terminal. Two pairs of full-length antibody chains are linked by a disulfide bridge between CL and CH1 and a disulfide bridge between HRs of two full-length heavy chains. The full-length antibody of the invention may be derived from a single species, such as a human; and can also be a chimeric antibody or a humanized antibody. The full-length antibody of the invention comprises two antigen-binding sites formed by VH and VL pairs, respectively, which sites specifically recognize/bind the same antigen.

As used herein, the term "Fd fragment" means an antibody fragment consisting of VH and CH1 domains; the term "dAb fragment" means an antibody fragment consisting of a VH domain (Ward et al., Nature 341: 544 546 (1989)); the term "Fab fragment" means an antibody fragment consisting of VL, VH, CL and CH1 domains; the term "F(ab')₂ fragment" means an antibody fragment comprising two Fab fragments linked by a disulfide bridge on the hinge region; the term "Fab' fragment" means a fragment obtained by reducing the disulfide bridge linking two heavy chain fragments in the F(ab')₂ fragment, which consists of a complete light chain and a Fd fragment (composed of VH and CH1 domains) of heavy chain.

As used herein, the term "Fv fragment" means an antibody fragment consisting of VL and VH domains of a single-arm of antibody. The Fv fragment is generally considered to be the smallest antibody fragment that can form a complete antigen-binding site. It is generally thought that six CDRs confer antigen-binding specificity to an antibody. However, even one variable region (such as a Fd fragment, which contains only three antigen-specific CDRs) can recognize and bind antigen, although possibly having a lower affinity than a complete binding site.

As used herein, the term "Fc fragment" means an antibody fragment formed by linking the second and third constant regions of the first heavy chain of an antibody to the second and third constant regions of the second heavy chain of the antibody via a disulfide bridge. Fc fragment of an antibody has many different functions, but does not involve in antigen binding.

As used herein, the term "scFv" refers to a single polypeptide chain comprising VL and VH domains, wherein the VL and VH are linked by a linker (see, for example, Bird et al., Science 242: 423-426 (1988); Huston et al., Proc. Natl. Acad. Sci. USA 85: 5879-5883 (1988); and Pluckthun, The Pharmacology of Monoclonal Antibodies, vol. 113, Ed. Roseburg and Moore, Springer-Verlag, New York, pp. 269-315 (1994)). Such scFv molecules may have a general structure: NH₂-VL-Linker-VH-COOH or NHz-VH-Linker-VL-COOH. A suitable linker in the art consists of repeated GGGGS amino acid sequences or variants thereof. For example, a linker of amino acid sequence (GGGGS)₄ or a variant thereof can be used (Holliger et al. (1993), Proc. Natl. Acad. Sci. USA 90: 6444-6448). Other linkers that may be used in the invention are described in Alfthan et al. (1995), Protein Eng. 8: 725-731; Choi et al. (2001), Eur. J. Immunol. 31: 94-106; Hu et al. (1996), Cancer Res. 56: 3055-3061; Kipriyanov et al. (1999), J. Mol. Biol. 293: 41-56 and Roovers et al. (2001), Cancer Immunol. In some cases, a disulfide bridge may also be present between VH and VL of scFv. As used herein, the term "di-scFv" refers to an antibody fragment formed by linking two scFvs.

As used herein, the term "diabody" means that the VH and VL domains are expressed on a single polypeptide chain but the used linker is too short to allow for pairing between two domains of the same chain, thus forcing the domain to pair with a complementary domain of the other chain and producing two antigen-binding sites (see, for example, Holliger P. et al., Proc. Natl. Acad. Sci. USA 90: 6444-6448 (1993), and Poljak R.J. et al., Structure 2:1121-1123 (1994)).

As used herein, "antibody mimetic" refers to specifically binds to an antigen as an antibody, but without antibody structure. They are usually artificial peptides or proteins having a molar mass of about 3 to 20 kDa. Examples are designed ankyrin repeat protein (DARPin) and fynomer. The designed ankyrin repeat protein (DARPin) can be linked to an IgG antibody, a scFv-Fc antibody fragment or combination thereof, as described in CN104341529A. An anti-IL-17a fynomer binds to an anti-IL-6R antibody, as described in WO2015141862A1.

Each of the above antibody fragments retains the ability to specifically bind to the same antigen as bound by the full-length antibody, and/or competes with the full-length antibody for specific binding to an antigen.

An antigen-binding fragment (e.g., the antibody fragment described above) may be obtained from a given antibody (e.g., the antibody provided by the invention) using conventional technologies known to those skilled in the art (e.g., recombinant DNA technologies or enzymatic or chemical cleavage methods), and the antigen-binding fragment of the antibody is specifically screened in the same manner as for an intact antibody.

Herein, unless clearly indicated otherwise in the context, reference to the term "antibody" includes not only an intact antibody but also an antigen-binding fragment thereof.

As used herein, the terms "monoclonal antibody", "McAb" and "mAb" have the same meaning and are used interchangeably, which refers to an antibody or a fragment thereof derived from a population of highly homologous antibody molecules, that is, a population of identical antibody molecules except for natural mutations that may occur spontaneously. A monoclonal antibody has a high specificity for a single epitope on an antigen. Compared to a monoclonal antibody, polyclonal antibodies usually contain at least two or more different antibodies, which usually recognize different epitopes on an antigen. Furthermore, the modifier "monoclonal" simply indicates that the features of an antibody are obtained from a population of highly homologous antibodies and cannot be understood as requiring any particular method to prepare the antibody.

A monoclonal antibody of the invention can be prepared by various technologies, such as hybridoma technology (see, for example, Kohler et al. Nature, 256: 495, 1975), recombinant DNA technology (see, for example, U.S. Patent Application 4,816,567), or phage antibody library technology (see, for example, Clackson et al. Nature 352: 624-628, 1991, or Marks et al. J. Mol. Biol. 222: 581-597, 1991).

For example, a monoclonal antibody can be prepared as follows. Firstly, a mouse or other suitable host animal is immunized with an immunogen (added with an adjuvant if necessary). After immunized, the animal will produce lymphocytes that secrete antibodies that specifically bind to the immunogen in vivo. In addition, lymphocytes can also be obtained by immunization in vitro. The lymphocytes of interest are collected and fused with myeloma cells using a suitable fusion reagent, such as PEG, to obtain hybridoma cells (Goding, Monoclonal Antibodies: Principles and Practice, pp.59-103, Academic Press, 1996). The hybridoma cells prepared as above can be inoculated into a suitable culture medium for growth, which then can be used for detecting the production of monoclonal antibodies against a specific antigen. Methods for determining the binding specificity of monoclonal antibodies produced by hybridoma cells include, for example, immunoprecipitation or in vitro binding assays, such as radioimmunoassay (RIA), enzyme-linked immunosorbent assay (ELISA). For example, the affinity of monoclonal antibodies can be determined by Scatchard analysis described in Munson et al., Anal. Biochem. 107: 220 (1980). After the specificity, affinity and reactivity of the antibodies produced by hybridoma are determined, the cell lines of interest can be subcloned by the standard limited dilution method described in Goding, Monoclonal Antibodies: Principles and Practice, pp. 59-103, Academic Press, 1996. The suitable culture medium can be DMEM or RPMI-1640. In addition, hybridoma cells can also be grown in vivo in animals in the form of ascites tumors. Monoclonal antibodies secreted by subcloned cells can be isolated from the cell culture medium, ascites, or serum by using traditional immunoglobulin purification methods, such as protein A agarose gel, hydroxyapatite chromatography, gel electrophoresis, dialysis or affinity chromatography, or the like.

Monoclonal antibodies can also be obtained by genetic engineering recombination technologies. DNA molecules encoding heavy chain and light chain genes of a monoclonal antibody can be isolated from hybridoma cells by PCR amplification using nucleic acid primers specifically binding to the heavy chain and light chain genes of the monoclonal antibody. The obtained DNA molecules are inserted into an expression vector with which host cells (such as E. coli cells, COS cells, CHO cells, or other myeloma cells that do not produce immunoglobulins) are then transfected, and are cultured under suitable conditions to obtain the recombinantly expressed antibody of interest.

Antibodies can be purified by well-known technologies such as affinity chromatography using protein A or protein G. Subsequently or alternatively, a specific antigen (a target molecule recognized by an antibody) or its antigenic epitope can be immobilized on a column, and an immunologically specific antibody can be purified by immune-affinity chromatography. For purification of immunoglobulins, reference may be made to, for example, D. Wilkinson (The Scientist, published by The Scientist, Inc., Philadelphia Pa., Vol. 14, No. 8 (Apr. 17, 2000), pp. 25-28).

As used herein, "% identity" between two sequences is a function of the number of identical positions shared by the two sequences (i.e., % homology = the number of identical positions/the total number of positions x 100), while considering the number of gaps and the length of each gap which needs to be introduced when the two sequences are optimally aligned. Sequence alignment and determination of % identity between two sequences can be accomplished by a mathematical algorithm. For example, the % identity between two amino acid sequences can be determined by the algorithm of E. Meyers and W. Miller (Computer. App. Biosci., 4:11-17 (1988)).

As used herein, a sequence having "% identity" retains important biological activities, such as antibody-binding specificity, of the sequence which it is aligned with or it is derived from. A sequence having a substitution, deletion or addition of one or more amino acids or any combination thereof retains important biological activities, such as antibody-binding specificity, of the sequence which it is aligned with or it is derived from. A nucleotide sequence having "% identity" or a nucleotide sequence differing by not more than 3, 6, 15, 30 or 45 nucleotides can achieve similar functions as the sequence which it is aligned with or it is derived from, e.g. all the expressed proteins can specifically bind to the same antigen or molecule.

As used herein, the term "chimeric antibody" means such an antibody where one part of its light chain or/and heavy chain is derived from an antibody (which can be derived from a specific species or belong to a specific antibody class or subclass), and the other part of the light chain or/and heavy chain is derived from another antibody (which may be derived from the same or different species or belong to the same or different antibody class or subclass), which in any case retains binding activity to a target antigen (U.S. P 4,816, 567 to Cabilly et al.; Morrison et al., Proc. Natl. Acad. Sci. USA, 81: 6851 6855 (1984)).

As used herein, the term "humanized antibody" refers to a genetically engineered non-human antibody whose amino acid sequence is modified to increase the homology with the sequence of a human antibody. Generally, all or part of the CDR regions of a humanized antibody are derived from a non-human antibody (donor antibody), and all or part of the non-CDR regions (e.g., FR in the variable region and/or the constant region) are derived from a human immunoglobulin (acceptor antibody). Humanized antibody usually retain the expected properties of the donor antibody, including but not limited to antigen specificity, affinity, reactivity, ability to improve immune cell activity, ability to enhance immune response, etc. Donor antibody may be mouse, rat, rabbit, or non-human primate (e.g., cynomolgus monkey) antibody having desired properties (e.g., antigen specificity, affinity, reactivity, ability to enhance immune cell activity and/or ability to enhance immune response).

Humanized antibodies are particularly advantageous, because they not only retain the expected properties of non-human donor antibodies (e.g., murine antibodies), but also effectively reduce the immunogenicity of non-human donor antibodies (e.g., murine antibodies) in a human subject. However, due to the matching problem between the CDRs of the donor antibody and the FRs of the acceptor antibody, the expected properties of humanized antibodies (e.g., antigen specificity, affinity, reactivity, ability to improve immune cell activity and/or ability to enhance immune response) are generally lower than those of non-human donor antibodies (e.g. mouse antibodies).

Thus, although researchers in the art have developed a deep research and made some progress on the humanization of antibodies (see, for example, Jones et al., Nature, 321: 522 525 (1986); Reichmann et al., Nature, 332: 323 329 (1988); Presta, Curr. Op. Struct. Biol., 2: 59 3 596 (1992); And Clark, Immunol. Today 21: 397 402 (2000)), nevertheless, the detailed guidance is not provided in the prior art on how to humanize a given donor antibody sufficiently so that the produced humanized antibody not only has a humanization degree as high as possible, but also retain the expected properties of the donor antibody as much as possible. To obtain a humanized antibody not only having a high humanization degree (for example, at least 75%, at least 80%, at least 85%, at least 90%, at least 91%, at least 92%, at least 93%, at least 94%, at least 95%, at least 96%, at least 97%, at least 98%, at least 99% humanization), but also retaining the expected properties of a particular donor antibody, those skilled in the art have to exploit, investigate and modify the particular donor antibody by expensing a lot of creative works.

In the invention, in order to make a humanized antibody retain the properties of a donor antibody as much as possible (including, for example, antigen specificity, affinity, reactivity, ability to improve immune cell activity and/or ability to enhance immune response), the framework region (FR) of the humanized antibody of the invention comprises both amino acid residues of a human acceptor antibody and corresponding amino acid residues of a non-human donor antibody.

In the present application, the expected properties of the antibody of the invention include: (1) specific recognizing/binding PD-1 (in particular human PD-1); (2) inhibiting and/or blocking the binding of PD-1 to PDL-1; (3) inhibiting and/or blocking the intracellular signaling mediated by the binding of PD-1 to PDL-1; (4) improving the activity of immune cells; (5) enhancing immune response; (6) reducing or eliminating antibody-dependent cell-mediated phagocytosis (ADCP) and/or antibody-dependent cell-mediated cytotoxicity (ADCC) and/or complement-dependent cytotoxicity (CDC). The humanized antibody of the invention retains one or more of the above-mentioned expected properties of the parent antibody (a murine antibody or mouse-human chimeric antibody).

The chimeric antibody or humanized antibody of the invention can be prepared according to the sequence of the murine monoclonal antibody prepared as above. The DNA encoding heavy and light chains can be obtained from a target mouse hybridoma and engineered using standard molecular biology technologies to include a non-mouse (e.g., human) immunoglobulin sequence.

For preparing a chimeric antibody, a mouse immunoglobulin variable region can be linked to a human immunoglobulin constant region using methods known in the art (see, for example, U.S. Pat. No. 4,816,567 to Cabilly et al.). For example, the DNA encoding a VH is operably linked to another DNA molecule encoding a heavy chain constant region to obtain a full-length heavy chain gene, or the DNA encoding a VL is operably linked to another DNA molecule encoding a light chain constant region CL to obtain a full-length light chain gene (as well as a Fab light chain gene). Sequences of human heavy chain constant region genes and light chain constant region genes are known in the art (see, for example, Kabat, E.A. et al. (1991) Sequences of Proteins of Immunological Interest, Fifth Edition, U.S. Department of Health and Human Services, NIH Publication No. 91-3242), and DNA fragments containing these regions can be obtained by standard PCR amplification. The heavy chain constant region may be an IgG1 (e.g., uniprot ID P01857), IgG2 (e.g., uniprot ID P01859), IgG3 (e.g., uniprot ID P01860), IgG4 (e.g., uniprot ID P01861), IgA, IgE, IgM, or IgD constant region, generally preferably an IgG1 or IgG4 constant region. Light chain constant regions can be κ or λ constant regions, generally preferably a κ constant region.

For preparing a humanized antibody, a mouse CDR region can be inserted into a human framework sequence using methods known in the art (see U.S. Pat. No. 5,225,539 to Winter; U.S. Pat. Nos. 5,530,101; 5,585,089; 5,693,762 and 6,180, 370 to Queen et al.; and Lo, Benny, K.C., editor, in Antibody Engineering: Methods and Protocols, volume 248, Humana Press, New Jersey, 2004). Alternatively, transgenic animals can also be utilized, which are incapable of producing endogenous immunoglobulins after immunization but capable of producing an intact human antibody library (see, for example, Jakobovits et al., 1993, Proc. Natl. Acad. Sci. USA 90: 2551; Jakobovits et al., 1993, Nature 362: 255-258; Bruggermann et al., 1993, Year in Immunology 7:33; and Duchosal et al., 1992, Nature 355: 258; Lonberg et al. (1994) Nature 368 (6474): 856-859; WO02/43478). Other methods of antibody humanization include phage display technology (Hoogenboom et al., 1991, J.Mol. Biol. 227: 381; Marks et al., J.Mol. Biol. 1991, 222: 581-597; Vaughan et al., 1996, Nature Biotech 14: 309).

As used herein, the term "humanization degree" is an indicator for evaluating the number of non-human amino acid residues in a humanized antibody. The humanization degree of humanized antibodies can be predicted, for example, by DomainGapAlign from the IMGT website for the homology of variable region sequences with human V domains.

As used herein, the term "specifically binding" refers to non-random binding reaction between two molecules, such as reaction between an antibody and an antigen against which it is. The strength or affinity of the specific binding interaction can be indicated by the equilibrium dissociation constant (K_{D}) of the interaction. In the invention, the term "K_{D}" refers to the dissociation equilibrium constant of specific antibody-antigen interaction, which is used to describe the binding affinity between an antibody and an antigen. The smaller the equilibrium dissociation constant, the tighter the antibody-antigen binding, and the higher the affinity between the antibody and the antigen. In certain embodiments, an antibody specifically binding to an antigen (or an antibody specific for an antigen) means that the antibody binds to the antigen with an affinity (K_{D}) of less than about 10⁻⁹ M, for example less than about 10⁻⁹ M, 10⁻¹⁰ M, 10⁻¹¹ M or 10⁻¹² M or less. In some embodiments, when K_{D}≤ 10 × 10⁻⁸ M (preferably K_{D}≤ 5 × 10⁻⁹ M), the antibody or antigen-binding fragment thereof of the invention is considered as specifically binding to PD-1.

The specific binding properties between two molecules can be measured using methods well known in the art. One of the methods involves measuring the rate of formation and dissociation of antigen-binding site/antigen complexes. Both "binding rate constant" (ka or kon) and "dissociation rate constant" (kdis or koff) can be calculated from the concentration and the actual rates of association and dissociation (see Malmqvist M, Nature, 1993, 361: 186-187). The ratio of kdis/kon is equal to the dissociation constant K_{D} (see Davies et al., Annual Rev Biochem, 1990; 59: 439-473). K_{D}, kon and kdis values can be measured by any effective method. In certain embodiments, the dissociation constant can be measured by bioluminescence interferometry (e.g., ForteBio Octet assay). In addition, the dissociation constant can also be measured by surface plasmon resonance technology (such as Biacore) or Kinexa.

As used herein, the term "vector" refers to a nucleic acid vehicle into which a polynucleotide can be inserted. When enabling the expression of a protein encoded by the inserted polynucleotide, the vector is called as expression vector. Vectors can be introduced into host cells through transformation, transduction, or transfection, so that the carried genetic material elements can be expressed in host cells. Vectors are well known to those skilled in the art, including but not limited to: plasmids; phagmids; cosmids; artificial chromosomes, such as yeast artificial chromosomes (YAC), bacterial artificial chromosomes (BAC), or P1-derived artificial chromosomes (PAC); phages, such as λ phage or M13 phage, and animal viruses. Animal viruses that can be used as vectors include, but not limited to, retroviruses (including lentivirus), adenoviruses, adeno-associated viruses, herpesviruses (e.g., herpes simplex virus), poxviruses, baculoviruses, papillomaviruses, papovaviruses (e.g., SV40). A vector may contain elements controlling expression, including but not limited to a promoter sequence, a transcription initiation sequence, an enhancer sequence, a selection element, and a reporter gene. In addition, the vector may also contain an origin of replication.

As used herein, the term "host cell" refers to a cell into which a vector can be introduced, including, but not limited to, prokaryotic cells such as Escherichia coli or Bacillus subtilis, fungal cells such as yeast cells or Aspergillus, insect cells such as S2 drosophila cells or Sf9, or animal cells such as fibroblasts, CHO cells, COS cells, NSO cells, HeLa cells, BHK cells, HEK 293 cells or human cells.

As used herein, the term "identity" is used to refer to the sequence matching degree between two polypeptides or two nucleic acids. When a position in two sequences for comparison is occupied by the same base or amino acid monomer subunit (for example, a position in each of two DNA molecules is occupied by adenine, or a position in each of two polypeptides is occupied by lysine), then the molecules are identical at that position. "Percentage identity" between two sequences is a function of the number of matching positions shared by the two sequences divided by the number of positions to be compared and ×100. For example, if six positions of the ten positions in two sequences match, then the two sequences are 60% identical. For example, the DNA sequences CTGACT and CAGGTT have 50% identity (3 positions of the total 6 positions match). Typically, the two sequences are compared and aligned for maximum identity. Such an alignment can be realized by using, for example, the method of Needleman et al. (1970) J. Mol. Biol. 48: 443-453, which can be conveniently performed by a computer program such as Align program (DNAstar, Inc). Additionally, the percentage identity between two amino acid sequences can be determined by the algorithm of Needleman and Wunsch (J Mol Biol. 48: 444-453 (1970)) in the GAP program incorporated in the GCG software package (available in www.gcg.com), using either a Blossum Matrix 62 or PAM250, and a gap weight of 16, 14, 12, 10, 8, 6 or 4 and a length weight of 1, 2, 3, 4, 5 or 6.

As used herein, the term "conservative substitution" means an amino acid substitution that does not adversely affect or alter the expected properties of a protein/polypeptide comprising an amino acid sequence. For example, a conservative substitution may be introduced by standard technologies known in the art, such as site-directed mutagenesis and PCR-mediated mutagenesis. A conservative amino acid substitution includes a substitution of an amino acid residue with an amino acid residue having a similar side chain, e.g., with a residue that is physically or functionally similar to the corresponding amino acid residue (e.g., having similar size, shape, charge, chemical property, including the ability to form covalent or hydrogen bonds, etc.). Families of amino acid residues having similar side chains have been defined in the art. These families include amino acids having basic side chains (e.g., lysine, arginine and histidine), acidic side chains (e.g. aspartic acid, glutamic acid), uncharged polar side chains (e.g. glycine, asparagine, glutamine, serine, threonine, tyrosine, cysteine, tryptophan), nonpolar side chains (e.g. alanine, valine, leucine, isoleucine, proline, phenylalanine, methionine), beta branched side chains (e.g., threonine, valine, isoleucine) and aromatic side chains (e.g. tyrosine, phenylalanine, tryptophan, histidine). Therefore, it is preferred to replace a corresponding amino acid residue with another amino acid residue from the same side chain family. Methods for identifying conservative substitution of amino acids are well known in the art (see, for example, Brummell et al., Biochem. 32: 1180-1187 (1993); Kobayashi et al., Protein Eng. 12 (10): 879-884 (1999); and Burks et al., Proc. Natl Acad. Set USA 94: 412-417 (1997), which are incorporated herein by reference).

The compilation of twenty conventional amino acids involved herein follows the conventional usage. See, for example, Immunology-A Synthesis (2nd Edition, E. S. Golub and D. R. Gren, Eds., Sinauer Associates, Sunderland, Mass. (1991)), which is incorporated herein by reference. In the invention, the terms "polypeptide" and "protein" have the same meaning and are used interchangeably. Also, in the invention, amino acids are generally expressed by single-letter and three-letter abbreviations well known in the art. For example, alanine can be expressed as A or Ala.

As used herein, the term "pharmaceutically acceptable carrier and/or excipient" means a carrier and/or excipient that is pharmacologically and/or physiologically compatible with the subject and active ingredient, which are well known in the art (see, for example, Remington's Pharmaceutical Sciences. Edited by Gennaro AR, 19th ed. Pennsylvania: Mack Publishing Company, 1995) and include, but not limited to, pH modulators, surfactants, adjuvants, ionic strength enhancers, diluents, osmotic pressure maintaining reagents, delayed absorption reagents, preservatives. For example, the pH modulators include, but not limited to, phosphate buffers. The surfactants include but not limited to cationic, anionic, or nonionic surfactants, such as Tween-80. The ionic strength enhancers include, but not limited to, sodium chloride. The preservatives include, but not limited to, various antibacterial and antifungal agents, such as p-hydroxybenzoate, trichloro-tert-butyl alcohol, phenol, sorbic acid, etc. The osmotic pressure maintaining reagents include, but not limited to, sugars, NaCl and the like. The delayed absorption reagents include, but not limited to, monostearates and gelatin. Diluents include, but not limited to, water, aqueous buffers (e.g., buffer saline), alcohols and polyols (e.g., glycerol), etc. The preservatives include, but not limited to, various antibacterial and antifungal agents, such as thiomersalate, 2-phenoxyethanol, p-hydroxybenzoate, trichloro-tert-butyl alcohol, phenol, sorbic acid, etc. The stabilizers have a meaning commonly understood by those skilled in the art, which can stabilize the desired activity of the active ingredient in drug, including but limited to, sodium glutamate, gelatin, SPGA, sugars (such as sorbitol, mannitol, starch, sucrose, lactose, dextran, or glucose), amino acids (such as glutamic acid, glycine), proteins (such as dried whey, albumin, or casein) or degradation products thereof (such as lactalbumin hydrolysate).

As used herein, the term "prevention" refers to a process for preventing or delaying the onset of a disease or condition or symptom (e.g., a tumor, an infection, or an autoimmune disease) in vivo in a subject. As used herein, the term "treatment" refers to a process for achieving a beneficial or desired clinical result. For purposes of the invention, beneficial or desired clinical result includes, but not limited to, alleviation of a symptom, diminishment of extent of disease, stabilizing (i.e., not worsening) state of a disease, delay or slowing of disease progression, amelioration or palliation of disease state, and remission (whether partial or all) of symptoms, no matter detectable or undetectable. In addition, "treatment" can also mean prolonging survival as compared to the expected survival (without treatment).

As used herein, the term "subject" refers to a mammal, such as a primate mammal, such as a human. In some embodiments, the subject (e.g., human) has a tumor, an infection, or an autoimmune disease, or is at a risk of suffering from such disease.

As used herein, the term "effective amount" refers to an amount sufficient to achieve or at least partially achieve a desired effect. For example, an effective amount for preventing a disease (e.g., a tumor, an infection, or an autoimmune disease) is an amount sufficient to prevent, stop, or delay the onset of the disease (e.g., a tumor, an infection, or an autoimmune disease); a therapeutic effective amount refers to an amount sufficient to cure or at least partially prevent a disease and its complications of a patient who have already suffered from the disease. To determine such effective amount is within the scope of capability of those skilled in the art. For example, the amount effective for a therapeutic use will depend on the severity of the disease to be treated, the general state of the patient's own immune system, the general condition of the patient such as age, weight and gender, route of administration, and other treatments administered concurrently, etc.

As used herein, the term "immune cell" includes cells that have hematopoietic origins and play a role in an immune response, for example, lymphocytes, such as B cells and T cells; natural killer cells; myeloid cells, such as monocytes, macrophages, eosinophils, mast cells, basophils, and granulocytes.

As used herein, the term "immune response" means the action of immune cells (such as lymphocytes, antigen presenting cells, phagocytes or granulocytes) and soluble macromolecules (including antibodies, cytokines and complements) produced by immune cells or liver, which leads to selective damage, destruction or removal from human body of invasive pathogens, pathogen-infected cells or tissues, cancer cells, or normal human cells or tissues in the case of an autoimmune or pathological inflammation. In the invention, the term "antigen-specific T cell response" refers to an immune response produced by T cells when the T cells are stimulated by an antigen specific to the T cells. Non-limiting examples of responses produced by T cells in response to antigen-specific stimulation include proliferation of T cells and production of cytokines (e.g., IL-2).

As used herein, the term "effector function" refers to those biological activities which contribute to the Fc region of an antibody (Fc region of a natural sequence or Fc region of an amino acid sequence variant) and vary with antibody isotypes. Examples of effector functions of an antibody include, but not limited to: Fc receptor binding affinity, antibody-dependent cell-mediated cytotoxicity (ADCC), complement-dependent cytotoxicity (CDC), antibody-dependent cell phagocytosis (ADCP), down-regulation of cell surface receptors (such as B cell receptors), B cell activation, cytokine secretion, half-lives/clearance rates of antibodies and antigen-antibody complexes, etc. Methods for altering effector functions of an antibody are known in the art, for example by introducing mutations in the Fc region.

As used herein, the term "antibody-dependent cell-mediated cytotoxicity (ADCC)" means a type of cytotoxicity, wherein Ig binds to the Fc receptor (FcR) present on cytotoxic cells (e.g., natural killer (NK) cells, neutrophils, or macrophages), enabling these cytotoxic effector cells to specifically bind target cells to which antigens are attached, and then killing the target cells by secreting cytotoxins. Methods for detecting the ADCC activity of an antibody are known in the art, for example, evaluated by determining the binding activity between the antibody to be tested and the Fc receptor (e.g., CD16a).

As used herein, the term "complement-dependent cytotoxicity (CDC)" refers to a type of cytotoxicity that activates a complement cascade by binding the complement component C1q to the antibody Fc. Methods for detecting the CDC activity of an antibody are known in the art, for example, evaluated by determining the binding activity between the antibody to be tested and the Fc receptor (e.g., C1q).

The terms "cancer" and "tumor" are used interchangeably and refer to a large class of diseases characterized by uncontrolled growth of abnormal cells in vivo. Unregulated cell division may lead to the formation of malignant tumors or cells that invade neighboring tissues, which may metastasize to distant sites of the body through the lymphatic system or bloodstream. Cancer includes benign and malignant cancers and dormant tumors or micrometastases. Cancer also includes hematological malignancies.

Exemplary tumors include, but not limited to, solid tumors, hematological tumors (e.g., leukemia, lymphoma, myeloma, such as multiple myeloma), and metastatic, refractory, or recurrent lesions of cancer; for example, including but not limited to, esophageal cancer, gastrointestinal cancer, pancreatic cancer, thyroid cancer, colorectal cancer, kidney cancer, lung cancer (e.g., non-small cell lung cancer), liver cancer, stomach cancer, head and neck cancer, bladder cancer, breast cancer, uterine cancer, cervical cancer, ovarian cancer, prostate cancer, testicular cancer, germ cell cancer, bone cancer, skin cancer, thoracic adenocarcinoma, cholangiocarcinoma, gallbladder cancer, melanoma, mesothelioma, lymphoma, myeloma (e.g., multiple myeloma), sarcoma, glioblastoma, neuroglioblastoma, and leukemia.

The term "hematological malignancy" includes lymphoma, leukemia, myeloma or lymphoid malignant tumor, and splenic cancer and lymph node tumor. Exemplary lymphoma includes B-cell lymphoma and T-cell lymphoma. B-cell lymphoma includes, for example, Hodgkin's lymphoma. T-cell lymphoma includes, for example, cutaneous T-cell lymphoma. Hematological malignancy also includes leukemia, such as secondary leukemia or acute lymphoblastic leukemia. Hematological malignancy also includes myeloma (e.g., multiple myeloma) and other hematological and/or B-cell or T-cell related cancers.

The term "pharmaceutically acceptable" means, when a molecule entity, a fragment of a molecule or a composition is properly administered to an animal or human, they will not produce unfavorable, allergic, or other adverse reactions. Specific examples of materials that may be used as pharmaceutically acceptable carrier or component thereof include sugars (e.g., lactose), starch, cellulose and derivatives thereof, vegetable oils, gelatin, polyols (e.g., propylene glycol), alginic acid, and the like.

### Beneficial effects of the invention

Compared with the prior art, the technical solutions of the invention have the following beneficial effects.
(1) The antibody of the invention not only specifically recognizes/binds PD-1 and blocks the binding of PD-1 to PDL-1 or PD-L2, but also enhances the activity of immune cells and stimulates immune response in vitro/in vivo. Therefore, the antibody of the invention has a potential to prevent and/or treat a tumor, an infection, or an autoimmune disease.
(2) The antibody (especially humanized antibody) of the invention not only retains the functions and properties of the parental murine antibody, thereby having a potential to prevent and treat a tumor, an infection or an autoimmune disease; but also has an extremely high humanization degree, so it can be safely administered to a human subject without triggering an immunogenic reaction. Therefore, the antibody (especially humanized antibody) of the invention is of great clinical value.

### Brief Description of the figures

Fig. 1: Detection of affinity of anti-PD-1 murine antibodies to CHO-hPD-1.
Fig. 2: Detection of in vivo drug efficacy of anti-PD-1 murine antibodies in inhibiting MC38 tumor growth in hPD-1 transgenic mice.
Fig. 3: Detection of activity of anti-PD-1 chimeric antibodies to block the interactions between CHO-hPD-1 and PD-L1.
Fig. 4: Detection of Tm of anti-PD-1 chimeric antibodies.
Fig. 5: Verification of in vivo drug efficacy of anti-PD-1 chimeric antibodies in inhibiting MC38 tumor growth in hPD-1 transgenic mice.
Fig. 6: Verification of the function of anti-PD-1 humanized antibodies in PD-1/PD-L1 luciferase reporter system.
Fig. 7-1: Detection of anti-PD-1 humanized antibodies in promoting SEB to stimulate PBMC to express and secrete IL-2 (Donor A). The final concentrations of the control antibody 1, 91D5-HZ2 and 29C6-HZ2 started at 1µM from left to right and were 3-fold diluted, and the final concentration of negative control antibody IgG was 1µM.
Fig. 7-2: Detection of anti-PD-1 humanized antibodies in promoting SEB to stimulate PBMC to express and secret IL-2 (Donor B). The final concentrations of control antibody 2, 91D5-HZ2 and 29C6-HZ2 started at 1µM from left to right and were 3-fold diluted, and the final concentration of negative control antibody IgG was 1µM.
Fig. 8: Detection of affinity of IgG1-1D mutant anti-PD-1 humanized antibodies to CHO-hPD-1.
Fig. 9: Detection of activity of IgG1-1D mutant anti-PD-1 humanized antibodies to block the interactions between CHO-hPD-1 and PD-L1.
Fig. 10: Nonspecific verification of IgG1-1D mutant anti-PD-1 humanized antibodies to BTLA-his and PD-L1-his.
Fig. 11: Detection of Tm of IgG1-1D mutant anti-PD-1 humanized antibodies.
Fig. 12: Verification of the function of IgG1-1D mutant anti-PD-1 humanized antibodies in PD-1/PD-L1 luciferase reporter system.
Fig. 13-1: Detection of dynamic affinity of 91D5-HZ2-IgG1-1D humanized antibody to CD64.
Fig. 13-2: Detection of dynamic affinity of 35E6-HZ1-IgG1-1D humanized antibody to CD64.
Fig. 13-3: Detection of dynamic affinity of the control antibody 1 to CD64.
Fig. 13-4: Detection of dynamic affinity of the control antibody 3 to CD64.
Fig. 14: Verification of the function of IgG1-1D mutant anti-PD-1 humanized antibodies in ADCP luciferase reporter system.
Fig. 15: Verification of the function of IgG1-1D mutant anti-PD-1 humanized antibodies in ADCC luciferase reporter system. The concentrations of 91D5-HZ2-IgG1-1D, 35E6-HZ1-IgG1-1D, the control antibody 1 and the control antibody 3 were 450 µg/mL, 45 µg/mL, 4.5 µg/mL, and 450 ng/mL from left (near Y axis) to right, and the final concentration of negative control antibody IgG was 150 µg/mL.
Fig. 16: Changes of tumor volume in each group of mice in human immune system reconstituted B-NDG mouse model 1 in the presence of IgG1-1D mutant anti-PD-1 humanized antibody and the control antibody, respectively.
Fig. 17: Changes of body weight in each group of mice in human immune system reconstituted B-NDG mouse model 1 in the presence of IgG1-1D mutant anti-PD-1 humanized antibody and the control antibody, respectively.
Fig. 18: Changes of tumor volume in each group of mice in human immune system reconstituted B-NDG mouse model 2 in the presence of IgG1-1D mutant anti-PD-1 humanized antibody combined with AB12T8, and the control antibody, respectively.
Fig. 19: Changes of body weight in each group of mice in Human immune system reconstituted B-NDG mouse model 2 in the presence of IgG1-1D mutant anti-PD-1 humanized antibody combined with AB12T8, and the control antibody, respectively.
Fig. 20: Changes of tumor volume in each group of mice in hPD-1/hTIM-3 transgenic mice model in the presence of IgG1-1D mutant anti-PD-1 humanized antibodies.
Fig. 21: Changes of body weight in each group of mice in hPD-1/hTIM-3 transgenic mice model in the presence of IgG1-1D mutant anti-PD-1 humanized antibodies.

### Sequence Information

The sequence information related to the invention is described in the following table, and the specific sequences are shown in the sequence listing.

| SEQ ID NO: | Description | SEQ ID NO: | Description |
|---|---|---|---|
| 1 | Heavy chain variable region of murine antibody 29C6 | 46 | 35E6-HZl-IgGl-lD Light chain nucleic acid sequence |
| 2 | Light chain variable region of murine antibody 29C6 | 47 | Kabat 91D5 CDR-H3 |
| 3 | IMGT 29C6 CDR-H1 | 48 | Kabat 91D5 CDR-L1 |
| 4 | IMGT 29C6 CDR-H2 | 49 | Kabat 91D5 CDR-L2 |
| 5 | IMGT 29C6 CDR-H3 | 50 | Kabat 91D5 CDR-L3 |
| 6 | IMGT 29C6 CDR-L1 | 51 | Heavy chain variable region of humanized antibody 91D5-HZ1 |
| 7 | IMGT 29C6 CDR-L2 | 52 | Heavy chain variable region of humanized antibody 91D5-HZ2 |
| 8 | IMGT 29C6 CDR-L3 | 53 | Light chain variable region of humanized antibody 91D5-HZ1, 91D5-HZ2 |
| 9 | Kabat 29C6 CDR-H1 | 54 | Heavy chain variable region of murine antibody 14A4 |
| 10 | Kabat 29C6 CDR-H2 | 55 | Light chain variable region of murine antibody 14A4 |
| 11 | 35E6-HZ1-IgG1-1D Light chain amino acid sequence | 56 | IMGT 14A4 CDR-H1 |
| 12 | Kabat 29C6 CDR-H3 | 57 | IMGT 14A4 CDR-H2 |
| 13 | Kabat 29C6 CDR-L1 | 58 | IMGT 14A4 CDR-H3 |
| 14 | Kabat 29C6 CDR-L2 | 59 | IMGT 14A4 CDR-L1 |
| 15 | Kabat 29C6 CDR-L3 | 60 | IMGT 14A4 CDR-L2 |
| 16 | Heavy chain variable region of | 61 | IMGT |
| | humanized antibody 29C6-HZ1 | | 14A4 CDR-L3 |
| 17 | Heavy chain variable region of humanized antibody 29C6-HZ2 | 62 | Kabat 14A4 CDR-H1 |
| 18 | Light chain variable region of humanized antibody 29C6-HZ1 | 63 | Kabat 14A4 CDR-H2 |
| 19 | Light chain variable region of humanized antibody 29C6-HZ2 | 64 | Kabat 14A4 CDR-H3 |
| 20 | Heavy chain variable region of murine antibody 35E6 | 65 | Kabat 14A4 CDR-L1 |
| 21 | Light chain variable region of murine antibody 35E6 | 66 | Kabat 14A4 CDR-L2 |
| 22 | IMGT 35E6 CDR-H1 | 67 | Kabat 14A4 CDR-L3 |
| 23 | IMGT 35E6 CDR-H2 | 68 | Heavy chain variable region of murine antibody 42G1 |
| 24 | IMGT 35E6 CDR-H3 | 69 | Light chain variable region of murine antibody 42G1 |
| 25 | IMGT 35E6 CDR-L1 | 70 | IMGT 42G1 CDR-H1 |
| 26 | IMGT 35E6 CDR-L2 | 71 | IMGT 42G1 CDR-H2 |
| 27 | IMGT 35E6 CDR-L3 | 72 | IMGT 42G1 CDR-H3 |
| 28 | Kabat 35E6 CDR-H1 | 73 | IMGT 42G1 CDR-L1 |
| 29 | Kabat 35E6 CDR-H2 | 74 | IMGT 42G1 CDR-L2 |
| 30 | Kabat 35E6 CDR-H3 | 75 | IMGT 42G1 CDR-L3 |
| 31 | Kabat 35E6 CDR-L1 | 76 | Kabat 42G1 CDR-H1 |
| 32 | Kabat 35E6 CDR-L2 | 77 | Kabat 42G1 CDR-H2 |
| 33 | Kabat 35E6 CDR-L3 | 78 | Kabat 42G1 CDR-H3 |
| 34 | Heavy chain variable region of humanized antibody 35E6-HZ1 | 79 | Kabat 42G1 CDR-L1 |
| 35 | Light chain variable region of humanized antibody 35E6-HZ1 | 80 | Kabat 42G1 CDR-L2 |
| 36 | Heavy chain variable region of murine antibody 91D5 | 81 | Kabat 42G1 CDR-L3 |
| 37 | Light chain variable region of murine antibody 91D5 | 82 | Heavy chain constant region of IgG4 (S228P) Mutant |
| 38 | IMGT 91D5 CDR-H1 | 83 | Heavy chain constant region of human IgG1 |
| | | | (mutant type) |
| 39 | IMGT 91D5 CDR-H2 | 84 | Human kappa light chain constant region |
| 40 | IMGT 91D5 CDR-H3 | 85 | 91D5-HZ2-IgG1-1D Heavy chain nucleic acid sequence |
| 41 | IMGT 91D5 CDR-L1 | 86 | 91D5-HZ2-IgG1-1D Heavy chain amino acid sequence |
| 42 | IMGT 91D5 CDR-L2 | 87 | 91D5-HZ2-IgG1-1D Light chain nucleic acid sequence |
| 43 | IMGT 91D5 CDR-L3 | 88 | 91D5-HZ2-IgG1-1D Light chain amino acid sequence |
| 44 | Kabat 91D5 CDR-H1 | 89 | 35E6-HZ1-IgG1-1D Heavy chain nucleic acid sequence |
| 45 | Kabat 91D5 CDR-H2 | 90 | 35E6-HZ1-IgG1-1D Heavy chain amino acid sequence |

### Abbreviations

- CDR: Complementary determining region in the variable region of an immunoglobulin
- FR: Antibody framework region: amino acid residues in the variable region of an antibody except for CDR residues
- VH: Heavy chain variable region of an antibody
- VL: Light chain variable region of an antibody
- IgG: Immunoglobulin G
- Kabat: Immunoglobulin alignment and numbering system proposed by Elvin A. Kabat (see, for example, Kabat et al., Sequences of Proteins of Immunological Interest, 5th Ed. Public Health Service, National Institutions of Health, Bethesda, Md., 1991).
- IMGT: A numbering system based on the international ImmunoGenetics information system^{®} (IMGT) initiated by Lefranc et al., see Lefranc et al., Dev. Comparat. Immunol. 27:55-77, 2003.
- HLA-DR: Specific receptor on lymphocytes
- TGI: Tumor Growth Inhibition
- EC₅₀: A concentration that produces 50% efficacy or binding
- IC₅₀: A concentration that produces 50% inhibition
- ELISA: Enzyme-linked immunosorbent assay
- PCR: Polymerase chain reaction
- HRP: Horseradish peroxidase
- IL-2: Interleukin-2
- KD: Equilibrium dissociation constant
- Ka: Binding rate constant
- Kd: Dissociation rate constant
- ADCC: Antibody-dependent cytotoxicity
- ADCP: Antibody-dependent cell-mediated phagocytosis
- Kon: Binding rate
- Kdif: Dissociation rate
- FACS: Flow cytometry fluorescence sorting
- CDR-H1: Complementary determining region 1 in heavy variable region of immunoglobulin
- CDR-H2: Complementary determining region 2 in heavy variable region of immunoglobulin
- CDR-H3: Complementary determining region 3 in heavy variable region of immunoglobulin
- CDR-L1: Complementary determining region 1 in light variable region of immunoglobulin
- CDR-L2: Complementary determining region 2 in light variable region of immunoglobulin
- CDR-L3: Complementary determining region 3 in light variable region of immunoglobulin

Embodiments of the invention will be described in detail below in combination with the accompanying drawings and examples, but those skilled in the art will understand that the following drawings and examples are only intended to illustrate the invention and are not intended to limit the scope of the invention. The various objects and advantageous aspects of the invention will become implementable to those skilled in the art from the following detailed description of the drawings and preferred embodiments. The examples without specific technologies or conditions illustrated therein can be referred to the technologies or conditions described in the literatures in the art, for example, see Sambrook et al., Molecular Cloning: A Laboratory Manual, Third Edition (translated by Huang Peitang et al.), or can be carried out according to the product instructions. The reagents or instruments employed without denoting manufacturers are conventional products that can be purchased from the market.

### EXAMPLE 1: Preparation of anti-PD-1 monoclonal antibody

### 1.1 Preparation of antigen and control antibody

The following three materials were used as antigens for immunization: (i) a plasmid DNA containing a nucleic acid sequence coding for an intact human PD-1. The nucleotide sequence encoding the intact human PD-1 of the amino acid sequence of NP_005009.2 was obtained by codon optimization and synthesis (Shanghai Sangon Biotech), which was then cloned into a pcDNA3.1 vector, the plasmid was extracted by an endotoxin-free plasmid extraction kit (purchased from OMEGA) and dissolved in a normal saline injection. (ii) A recombinant fusion protein of the extracellular domain of human PD-1. The human PD-1-ECD-mFc protein were obtained by fusing the extracellular domain (ECD) of PD-1 with a mouse Fc (mFc) amino acids (AB097847.1), codon optimizing and synthesizing the nucleotide sequence by Nanjing Genscript, molecular cloning and lentivirus infecting the HEK293F cells, the supernatant was then purified by a ProA column, the purity of the recombinant protein was verified by SDS-PAGE and SEC, and the activity of the recombinant protein human PD-1-mFc was verified by ELISA. (iii) CHO-S cells expressing the full-length human PD-1 on membrane. The CHO-hPD-1 cell line was obtained by molecular cloning, infecting CHO-S cells with lentivirus and screening under pressure followed by FACS assay.

Preparation of the control antibody for the experiments: the amino acid sequences of pembrolizumab (IMGT ID: 472), sintilimab (IMGT ID: 859) and tirelizumab (IMGT ID: 757) were from the IMGT website, each of which was codon optimized and synthesized into an expression vector pcCHO1.0 by Nanjing Genscrip, and transiently transfected into CHO-S cells by PEI, and the obtained cells expressed the antibodies, the supernatant were then purified, and denominated as the control antibody 1, the control antibody 2 and the control antibody 3, respectively.

### 1.2 Mouse immunization

The plasmid DNA, the recombinant fusion protein of human PD-1 extracellular domain and the CHO-hPD-1 cells were used as antigens to immunize various strains of mice such as B6C57 mice, Balb/c mice, and the like (all purchased from Beijing Vital River) aged 7-8 weeks. During immunization, serum titer of the anti-PD-1 antibody was detected by ELISA every two weeks. After the single-cell suspension of mice spleen and lymph node was mixed, SP2/0 myeloma cells were then added in a quantity ratio of 1:1 and mixed evenly. The cell mixed solution was washed and resuspended with electrofusion buffer (Zhuhai Qiwen Biotechnology) wherein the cell density in the suspension is 1×10⁶ cells/mL. After electrofusion by a BTX 2000 electrofusion device, the cell suspension was immediately transferred from the fusion chamber to a sterile centrifuge tube containing complete culture medium and incubated in an incubator at 37°C for at least 1 hour. The cell suspension was then mixed and plated into 96-well plates at a density of 2×10⁴ cells per well, for a total of about 300 plates. The fused cells were cultured at 37°C and 5% CO₂. After hybridoma clones were cultured for 5 days, the hybridoma clones in all the 96-well plates were thoroughly exchanged for complete culture medium, and when growing for 7 days, the hybridoma clones were screened.

### 1.3 Hybridoma screening

Hybridoma screening was performed by ELISA and competitive FACS. The specific experimental steps of ELISA were as follows: the extracellular domain of human PD-1 and that of monkey PD-1 (NP_001107830) were fused to corresponding tags, respectively, antigens such as human PD-1-His, human PD-1-hFc and monkey PD-1-hFc were constructed by molecular cloning and homologous recombination, expressed and purified, which were then diluted to 1 µg/mL with CBS solution, and coated on a 96-well ELISA plate (purchased from Guangzhou Jiete Bio) at 4°C overnight. After washing, 100µL of phosphate buffer (PBS) containing 2% BSA was used for blocking at 37°C for 1 hour, followed by adding 20µL of hybridoma clone supernatant and incubating at 37°C for 2 hours. After washing, a goat anti-mouse secondary antibody (FC) labeled with horseradish peroxidase diluted in phosphate buffer (PBS) containing 2% BSA in a ratio of 1:15000 was added, followed by incubating at 37°C for 1 hour. After washing, the TMB chromogen solution was used for developing, and the absorbance value was read using a microplate reader at 450nm.

The specific experimental steps of competitive FACS were as follows: competitive FACS screening were performed on human and monkey binding positive hybridoma clones after ELISA screening. CHO-hPD-1 cells expressing the full-length human PD-1 were centrifugated and washed with phosphate buffer (PBS). The cell density was adjusted to 1×10⁷ cells/mL with phosphate buffer (PBS), the resultant solution was then added into a V-bottomed 96-well plate at 50µL/well, and the supernatant was removed by centrifugation. 50µL of the supernatant of positive hybridoma clones was taken to be added to a V-bottomed 96-well plate together with 50µL of FITC-labeled human PD-L1-mFc recombinant fusion protein which was diluted to 300nM/mL with phosphate buffer (PBS) containing 0.5% BSA, followed by thoroughly mixing, and incubating at 4°C in dark for 40min. After centrifugation and washing with phosphate buffer (PBS) twice, the results were analyzed by flow cytometry (Beckman) and FlowJo software.

### 1.4 Anti-PD-1 Hybridoma Subcloning

Through the screening described in 1.3, the hybridoma clones 42G1, 29C6, 91D5, 14A4 and 35E6 were selected for subcloning, the anti-PD-1 antibodies produced by which were denominated as 42G1, 29C6, 91D5, 14A4 and 35E6, respectively. Specifically, after serial dilution, hybridoma cells in wells were subjected to cell counting, individual wells having 60-70 hybridoma cells were selected for resuspending and plating in a 96-well plate, and the plated cells were cultured at 37°C, 5% CO₂. After culturing for 7 days, ELISA and FACS assays were performed using the same method as in Example 1.3, 1-2 positive single clones were selected from each plate for expansion culture in serum-free medium, then murine antibodies were collected for the next functional verification.

### EXAMPLE 2: Evaluation of anti-PD-1 murine antibody

### 2.1 Determination of binding affinity of anti-PD-1 murine antibody to human PD-1-hFc and monkey PD-1-hFc

In order to verify the binding affinity of murine anti-PD-1 antibody to human PD-1-hFc and monkey PD-1-hFc, ELISA was used. The specific experimental steps are as follows: human PD-1-hFc and monkey PD-1-hFc were diluted to 1µg/mL with CBS solution, and then coated on a 96-well ELISA plate (purchased from Guangzhou Jiete Bio) at 4°C overnight. After washing, 100µL of phosphate buffer (PBS) containing 2% BSA was used for blocking at 37°C for 1 hour, and then 100µL of murine anti-PD-1 antibodies 42G1, 29C6 and 91D5 were added (starting at 10µg/mL, 4-fold serial dilution, 2 replicates), then allowed for incubating at 37°C for 2 hours. After washing, a goat anti-mouse secondary antibody (FC) labeled with horseradish peroxidase diluted in phosphate buffer (PBS) containing 2% BSA in a ratio of 1:15000 was added, followed by incubating at 37°C for 1 hour. After washing, the TMB chromogen solution was used for developing, and the absorbance value was read using a microplate reader at 450nm. The data were imported into Graphprism to calculate the EC₅₀ value.

The results were shown in Table 1, EC₅₀ of each of murine antibodies 29C6, 42G1 and 91D5 for human PD-1-hFc and monkey PD-1-hFc were 30-70pM, showing a good binding affinity of murine antibodies to human or monkey PD-1.

### 2.2 Determination of the activity of anti-PD-1 murine antibody for blocking the binding of human PD-1/PD-L1

Competitive ELISA was used to verify blocking of PD-1/PD-L1 by murine anti-PD-1 antibody. The specific experimental steps were as follows: human PD-1-hFc antigens were diluted to 1µg/mL with CBS solution, then coated on a 96-well ELISA plate (purchased from Guangzhou Jiete Bio) at 4°C overnight. After washing, 100µL of phosphate buffer (PBS) containing 2% BSA was used for blocking at 37°C for 1 hour, and then 100µL of murine anti-PD-1 antibodies 42G1, 29C6 and 91D5 containing 0.8µg/mL biotinylated PD-L1-mFc were added (starting at 10µg/mL, 2-fold serial dilution, 2 replicates), and allowed for incubating at room temperature for 2 hours. After washing, streptavidin labeled with horseradish peroxidase diluted in phosphate buffer (PBS) containing 2% BSA in a ratio of 1:15000 was added, followed by incubating at room temperature for 1 hour. After washing, the TMB chromogen solution was used for developing, and the absorbance value was read using a microplate reader at 450nm, and the data were imported into Graphprism to calculate the IC₅₀ value.

The results were shown in Table 1, IC₅₀ of each of murine antibodies 29C6, 42G1 and 91D5 for blocking the binding of PD-1-hFc to PD-L1-mFc were at the level of nM, showing a good activity of blocking PD-1/PD-L1 binding.

**Table 1 Affinity and blocking activity of murine antibody**

| Name of murine antibody | Human PD-1-hFc EC₅₀ (pM) | Monkey PD-1-hFc EC₅₀ (pM) | IC₅₀ (nM) of blocking the binding of PD-1-hFc to PD-L1-mFc |
|---|---|---|---|
| 42G1 | 51.3 | 59.5 | 4.50 |
| 29C6 | 35.5 | 34.4 | 2.93 |
| 91D5 | 44.0 | 68.5 | 4.25 |

### 2.3 Determination of binding affinity of anti-PD-1 murine antibody to PD-1 on the cell membrane surface of CHO-hPD-1

In order to verify the binding of anti-human PD-1 murine antibody to PD-1 on the cell membrane surface, FACS technology was used to detect the affinity between murine antibodies and CHO-hPD-1 cells.

The specific experimental steps were as follows: CHO-hPD-1 cells were centrifuged at 500g for 3 minutes, and then resuspended with PBS (containing 1% BSA) to 6×10⁶ cells/mL, and 50µL was taken to be added to corresponding 96-well plates; murine antibodies 42G1, 29C6 and the control antibody 1 were diluted with PBS (containing 1% BSA) (starting at 1µM, 3-fold serial dilution, 11 concentration points), and then 50µL was added to corresponding 96-well plates, and there was no antibody in the blank control well; the cells and antibodies were gently mixed, and left at 4°C for 1 hour, washed with PBS twice, and resuspended with 50µL of PBS (containing 1% BSA); wherein, 2.5µL of fluorescent secondary antibody Alexa Fluor 488 anti-human IgG Fc was added to the sample of the control antibody 1, 2.5µL of FITC anti-mouse IgG was added to the samples of the 42G1 and 29C6 murine antibodies, respectively, and left at 4°C for 1 hour, washed with PBS for 3 times, and then detected by flow cytometry (manufactured by Beckman, Model: CytExpert), and the data were imported into Graphprism to calculate EC₅₀ values.

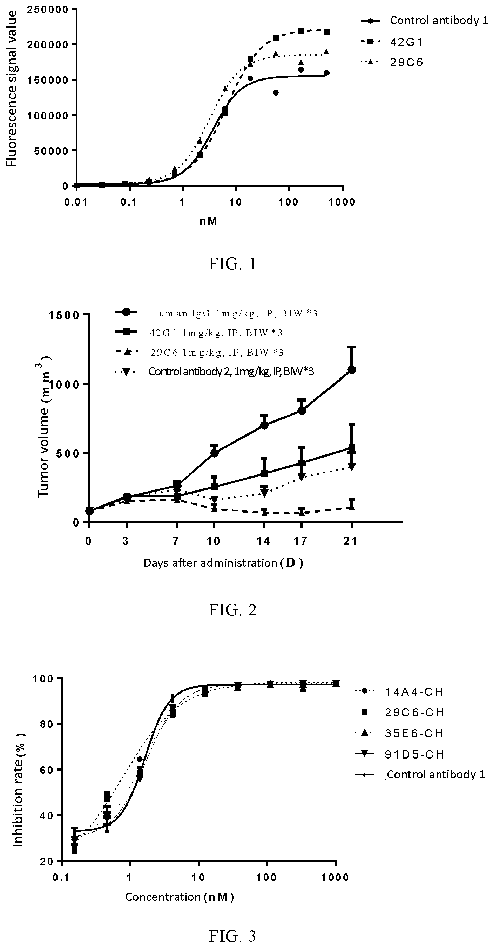

The experimental results were shown in Fig. 1, wherein, EC₅₀ of murine antibody 29C6 was 3.12nM, EC₅₀ of 42G1 was 6.62nM, and EC₅₀ of control antibody 1 was 3.57nM. Therefore, murine antibodies have a good binding affinity to PD-1 on the cell membrane surface.

### 2.4 Determination of in vivo drug efficacy of anti-PD-1 murine antibody in hPD-1 transgenic mice

In order to verify the anti-tumor effect of anti-human PD-1 murine antibody in vivo, a subcutaneous tumorigenesis model of hPD-1 (Beijing Vitalstar) transgenic mice was used. The assay was as follows: MC38 tumor cells (purchased from ATCC) were cultured using RPMI1640 culture medium containing 10% fetal bovine serum at 37°C, 5% CO₂. Mouse PD-L1 can bind to human PD-1 normally, and anti-human PD-1 antibody can inhibit the growth of tumor cells after blocking the binding of mouse PD-L1 to human PD-1. MC38 cells in exponential growth phase were collected, and resuspended to a suitable concentration with PBS and inoculated subcutaneously to female hPD-1 mice to establish a colon cancer model. When reaching an average volume of about 100 mm³, the mice were randomly divided into the following groups according to tumor size: the group of Human IgG (negative control), the group of murine antibody 42G1, the group of murine antibody 29C6 and the group of the control antibody 2, respectively, wherein administered dosage is 1 mg/kg and injected intraperitoneally twice a week for 3 weeks. After administration, the tumor volume and body weight of mice were observed and measured regularly. The experimental index was to investigate the effect of drugs on tumor growth, and the specific index was T/C% or tumor growth inhibition% (TGI%). The tumor diameter was measured by a vernier caliper twice a week, and the formula of calculating the tumor volume (V) was: V = 1/2×a×b², wherein, a and b denote the length and the width, respectively. T/C% = T/C×100, wherein C and T represent the tumor volume or the tumor weight of the solvent group and the treatment group, respectively. Tumor growth inhibition (TGI%) = (C-T)/C×100, wherein C and T represent the tumor volume or the tumor weight of the solvent group and the treatment group, respectively.

The results were shown in Figure 2: compared with the negative control group, the efficacy of the murine antibody 29C6 group was significant, TGI = 97.23%, wherein complete tumor regression occurred in 3 of 6 mice; the efficacy of the murine antibody 42G1 was significant, TGI = 55.12%, wherein complete tumor regression occurred in 1 of 6 mice; the efficacy of the control antibody 2 was significant, TGI = 68.99%, wherein partial tumor regression occurred in 1 of 6 mice.

### EXAMPLE 3 Acquisition of the anti-PD-1 antibody sequence

The murine antibody sequence was acquired according to the method of Gary C. Howard et al. (Basic Methods in Antibody Production and Character, CRC press, 2000), the candidate hybridoma RNAs were extracted and amplified by RT-PCR to obtain the light and heavy variable regions of the murine antibodies, which were then constructed to T vectors for sequencing. Hybridoma RNAs were extracted according to the instructions of the TRIzol RNA Isolation Reagents kit, which was then dissolved in 50µL of DEPC treated water, and the RNA concentration was detected. According to the instructions of the RevertAid First Strand cDNA Synthesis Kit, the total cDNA was obtained by reverse transcription using an oligo(dT)18 primer. Referring to the method of Gary C. Howard et al., as well as the sequence analysis of all murine antibodies with IMGT, the upstream primers of multiple pairs for variable regions were designed by selecting the regions of high homology, and the downstream primers were designed by CH1 homologous sequences, the light and heavy chain variable regions of the antibodies were obtained by PCR amplification by using primer pool, and the PCR product was constructed to pUCm-T vector, after identification and sequencing analysis, the variable regions and CDR sequences of the antibodies were shown in Table 2.

**Table 2 Variable regions and CDR amino acid sequences of anti-human PD-1 murine antibody**

| Name of murine antibody | Heavy chain variable region (SEQ ID NO:) | Light chain variable region (SEQ ID NO:) | Defined by | Heavy chain CDR1 (SEQ ID NO:) | Heavy chain CDR2 (SEQ ID NO:) | Heavy chain CDR3 (SEQ ID NO:) | Light chain CDR1 (SEQ ID NO:) | Light chain CDR2 (SEQ ID NO:) | Light chain CDR3 (SEQ ID NO:) |
|---|---|---|---|---|---|---|---|---|---|
| 14A4 | 54 | 55 | IMGT | 56 | 57 | 58 | 59 | 60 | 61 |
| | | | Kabat | 62 | 63 | 64 | 65 | 66 | 67 |
| 29C6 | 1 | 2 | IMGT | 3 | 4 | 5 | 6 | 7 | 8 |
| | | | Kabat | 9 | 10 | 12 | 13 | 14 | 15 |
| 35E6 | 20 | 21 | IMGT | 22 | 23 | 24 | 25 | 26 | 27 |
| | | | Kabat | 28 | 29 | 30 | 31 | 32 | 33 |
| 91D5 | 36 | 37 | IMGT | 38 | 39 | 40 | 41 | 42 | 43 |
| | | | Kabat | 44 | 45 | 47 | 48 | 49 | 50 |
| 42G1 | 68 | 69 | IMGT | 70 | 71 | 72 | 73 | 74 | 75 |
| | | | Kabat | 76 | 77 | 78 | 79 | 80 | 81 |

### EXAMPLE 4: Preparation and determination of anti-PD-1 chimeric antibody

### 4.1 Expression of anti-PD-1 chimeric antibody

The chimeric antibodies were constructed by fusion the amino acid sequences of the light chain variable regions of 14A4, 29C6, 35E6 and 91D5 to the amino acid sequence of the light chain kappa constant region (SEQ ID NO: 84), respectively, and fusion the amino acid sequences of the heavy chain variable regions of 14A4, 29C6, 35E6 and 91D5 to the amino acid sequence of the heavy chain constant region of IgG4 (SEQ ID NO: 82), respectively. The cDNA was synthesized after codon optimization and ligated to the plasmid PPT5 (Shanghai sangon Co., Ltd.). PPT5 vectors carrying the heavy and light chains of each chimeric antibody were simultaneously transfected into CHO-S-E cells (prepared by Sichuan Kelun Biotech Biopharmaceutical Co., Ltd.), and the culture supernatant was purified by a protein A column (MabSelect SuRe, GE) to obtain chimeric antibodies, which were denominated as 14A4-CH, 29C6-CH, 35E6-CH, and 91D5-CH, respectively.

### 4.2 Determination of binding affinity of anti-PD-1 chimeric antibodies to human PD-1-hFc and monkey PD-1-hFc by ELISA

The specific experimental steps were as follows: human PD-1-hFc and monkey PD-1-hFc antigens were diluted to 1µg/mL with CBS solution, then coated on a 96-well ELISA plate (purchased from Guangzhou Jiete Bio) at 4°C overnight. After washing, 100µL of phosphate buffer (PBS) containing 2% BSA was used for blocking at 37°C for 1 hour, followed by adding 100µL of chimeric antibodies 14A4-CH, 91D5-CH, 35E6-CH, 29C6-CH and the control antibody 1 (starting at 10µg/mL, 4-fold serial dilution), respectively, and incubating at 37°C for 2 hours. After washing, a goat anti-human secondary antibody (FC) labeled with horseradish peroxidase diluted in phosphate buffer (PBS) containing 2% BSA in a ratio of 1:15000 was added, followed by incubating at 37°C for 1 hour. After washing, the TMB chromogen solution was used for developing, and the absorbance value was read using a microplate reader at 450nm, and the data were imported into Graphprism to calculate EC₅₀ values.

The results were shown in Table 3 below, the binding affinity EC₅₀ of each of chimeric antibodies 14A4-CH, 91D5-CH, 35E6-CH, 29C6-CH, and the control antibody 1 to human PD-1-hFc and monkey PD-1-hFc were in a range from 0.05 to 0.3 nM.

### 4.3 Determination of the activity of anti-PD-1 chimeric antibody for blocking PD-1/PD-L1 binding and PD-1/PD-L2 binding by ELISA

The specific experimental steps were as follows: human PD-1-hFc was diluted to 1µg/mL with CBS solution, coated on a 96-well ELISA plate (purchased from Guangzhou Jiete Bio) at 4°C overnight. After washing, 100µL of phosphate buffer (PBS) containing 2% BSA was used for blocking at 37°C for 1 hour. 50µL of chimeric antibodies 14A4-CH, 91D5-CH, 35E6-CH, 29C6-CH and the positive control antibody 1 was added (starting at 20µg/mL, 2-fold dilution), respectively, simultaneously 50µL (1.6µg/mL) of hPD-L1-mFc (hPD-L1 amino acid sequence NP_054862.1) or hPD-L2-mFc (hPD-L2 amino acid sequence AAI13679) was added per well, followed by incubating at room temperature for 2 hours. After washing, a goat anti-mouse secondary antibody labeled with HRP diluted in phosphate buffer (PBS) containing 2% BSA in a ratio of 1:15000 was added, followed by incubating at room temperature for 1 hour. After washing, the TMB chromogen solution was used for developing, and the absorbance value was read using a microplate reader at 450nm, and the data were imported into Graphprism to calculate IC₅₀ values.

The results were shown in Table 3 below. It can be seen from IC₅₀ that, the activity of each of the chimeric antibodies 14A4-CH, 91D5-CH, 35E6-CH, 29C6-CH for blocking PD-1/PD-L1 binding was comparable to that of the control antibody 1, and the activity of each of the chimeric antibodies for blocking PD-1/PD-L2 binding was comparable to that of the control antibody 1.

### 4.4 Detection of the activity of anti-PD-1 chimeric antibody for blocking PD-L1/CHO-hPD-1 binding

A competitive FACS technology was used to detect the activity of anti-PD-1 chimeric antibody for blocking PD-L1/CHO-hPD-1 binding. The specific experimental steps were as follows: CHO-hPD-1 cells were centrifuged at 500g for 3 minutes, and resuspended with PBS (containing 1% BSA) to 6×10⁶ cells/mL, 50µL of which was added to corresponding 96-well plates; chimeric antibodies 14A4-CH, 91D5-CH, 35E6-CH, 29C6-CH and the control antibody 1 were diluted with PBS (containing 1% BSA), and diluted to 1µM with PBS containing 0.4µg/mL PD-L1-mFc-Bio, then 3-fold diluted to obtain 9 concentration points, and then 50µL was added to corresponding 96-well plates, and there was no antibody in the blank control well; the cells and the antibodies were gently mixed, left at 4°C for 1 hour, washed with PBS twice, resuspended with 50µL of PBS (containing 1% BSA); 2.5µL of APC streptavidin fluorescent secondary antibody was added to each of the samples, left at 4°C for 1 hour; followed by washing with PBS for 3 times, and each sample was detected by flow cytometry (manufactured by Beckman, Model: CytExpert). The data were imported into Graphprism to calculate IC₅₀ values.

The experimental results were shown in Table 3 below and Fig. 3, it can be seen from IC₅₀ that chimeric antibodies 14A4-CH and 91D5-CH were more active in blocking the binding of CHO-hPD-1 to PD-L1 than the control antibody 1; the activities of chimeric antibodies 35E6-CH and 29C6-CH for blocking the binding of CHO-hPD-1 to PD-L1 were comparable to that of the positive control antibody 1.

**Table 3 Affinity and blocking activity of anti-human PD-1 chimeric antibody**

| Antibody name | Human PD-1-mFc EC₅₀ (nM) | Monkey PD-1-hFc EC₅₀ (nM) | Blocking PD-1/PD-L1 IC₅₀ (nM) | Blocking PD-1/PD-L2 IC₅₀ (nM) | Blocking CHO-hPD-1/PD-L1 IC₅₀ (ng)/mL |
|---|---|---|---|---|---|
| 14A4-CH | 0.082 | 0.171 | 1.12^{∗} | 1.17^{∗} | 0.75 |
| 91D5-CH | 0.090 | 0.235 | 1.23 | 1.39 | 0.96 |
| 35E6-CH | 0.103 | 0.198 | 1.60 | 1.78 | 1.58 |
| 29C6-CH | 0.115 | 0.171 | 1.73 | 1.97 | 1.70 |
| Control antibody 1 | 0.116 | 0.131 | 1.42 | 1.44 | 1.64 |

| | | | | | |
|---|---|---|---|---|---|
| ^{∗} denotes calibration conversion. | | | | | |

### 4.5 Measurement of dynamic affinity of anti-PD-1 chimeric antibody

The dynamic affinity of candidate chimeric antibodies 14A4-CH, 91D5-CH, 35E6-CH, 29C6-CH and the control antibody 1 to human PD-1-His were measured by Octet ForteBio^{®} for detecting the dynamic affinity of an antibody to an antigen. The specific experimental steps were as follows: a ProA biosensor was firstly allowed for binding to the antibody to be tested until the response signal value reached 0.5 nm, then binding to human PD-1-His protein (500, 250, 125, 62, 31, 16 and 0 nM) for 80 seconds, and then dissociating for 120 seconds, the measured results were analyzed by 1:1 model and global fitting. As shown in Table 4, the association rates of each of the chimeric antibodies 14A4-CH, 91D5-CH, 35E6-CH and 29C6-CH were at the same level compared to that of the control antibody 1 (as shown by kon values), while the dissociation rates of each of the chimeric antibodies were slower than that of the control antibody 1 by one order of magnitude (as shown by kdis values), the affinity of each of the chimeric antibodies were stronger than that of the control antibody 1 by 4.3, 11.7, 15.4 and 7.3 times, respectively.

**Table 4 Determination of dynamic affinity of anti-PD-1 chimeric antibody to PD-1-his**

| Antibody name | KD (M) | kon (1/Ms) | kdis (1/s) | Full R^2 |
|---|---|---|---|---|
| 14A4-CH | 1.49E-09 | 1.38E+05 | 2.06E-04 | 0.997 |
| 91D5-CH | 5.49E-10 | 2.61E+05 | 1.43E-04 | 0.997 |
| 35E6-CH | 4.16E-10 | 2.96E+05 | 1.23E-04 | 0.9937 |
| 29C6-CH | 8.80E-10 | 2.26E+05 | 1.98E-04 | 0.9951 |
| Control antibody 1 | 6.44E -09 | 4.67E +05 | 3.01E-03 | 0.9913 |

### 4.6 Cell activity of anti-PD-1 chimeric antibody for blocking PD-1/PD-L1 binding

The cell bioactivity of blocking PD-1/PD-L1 binding was detected by the reporter gene assay. The CHO-PD-L1-CD3 cell line stably expresses PD-L1 and anti-CD3-scFv, used as target cell; the Jurkat-PD-1-NFAT cell line stably expresses PD-1 and luciferase, used as effector cell, wherein the luciferase gene is regulated by a NFAT element (transcription factor) (IL-2 promoter). The binding of PD-1 to PD-L1 can block the transduction of CD3 downstream signaling, thus inhibiting the expression of luciferase, when PD-1 antibody or PDL-1 antibody is added, this blocking effect is reversed, and luciferase can be expressed, so fluorescence signal can be detected (see Lan Wang, Chuanfei Yu et. al., Development of a robust reporter gene assay to measure the bioactivity of anti-PD-1/anti-PD-L1 therapeutic antibodies. J Pharm Biomed Anal 2017 Oct 25; 145: 447-453).

Specific experimental steps were as follows: Jurkat-PD-1-NFAT cells (purchased from Promega) and CHO-S-OKT3-PD-L1 cells (purchased from Promega) were centrifuged at 200g for 5 min, and resuspended with the detection buffer RPMI1640+1% FBS. CHO-S-OKT3-PD-L1 cells were plated at 5×10⁵ cells/40µL/well. Jurkat-PD-1-NFAT cells were added at 1.25×10⁶/40µL/well to the wells previously plated with CHO-S-OKT3-PDL1 cells, followed by adding 20µL of chimeric antibodies 14A4-CH, 91D5-CH, 35E6-CH, 29C6-CH and the control antibody 1 (starting at 100µM, 2-fold serial dilution), respectively. After incubation for 5 hours, they were taken out and placed at room temperature for 20 min, added with 20µL of detection reagent Bright-Glo Luciferase (purchased from Promega Company), oscillated at room temperature for 5 min, and measured using a microplate reader (BMG Company, PHEARstar FS). The experimental data were curve-fitted by Graphprism to calculate EC₅₀. The results were shown in Table 5. The cell activities of each of chimeric antibodies 14A4-CH, 91D5-CH, 35E6-CH, 29C6-CH and the control antibody 1 for blocking PD-1/PD-L1 binding were at the level of nM.

**Table 5 Cell activity of anti-PD-1 chimeric antibody for blocking PD-1/PD-L1 binding**

| Antibody name | EC₅₀ (nM) |
|---|---|
| Control antibody 1 | 5.24±2.22 (n=3) |
| 14A4-CH | 8.40±2.70 (n=2) |
| 91D5-CH | 4.41±2.03 (n=2) |
| 35E6-CH | 5.53±2.09 (n=2) |
| 29C6-CH | 10.67 (n=1) |

| | |
|---|---|
| n: denoting the number of experiments | |

### 4.7 Determination of Tm of anti-human PD-1 chimeric antibody

The Tm value of anti-PD-1 chimeric antibody was determined by DSF (Differential Scanning Fluorimetry). The specific experimental steps were as follows: each of anti-PD-1 chimeric antibodies 14A4-CH, 91D5-CH, 35E6-CH and 29C6-CH were diluted to 1mg/mL with PBS, and 12.5µL of each was taken and added with 2.5µL of 40×SYPRO Orange dye (Life Technologies Co., Ltd., Cat. No. 4306737) and 5µL of ddH₂O. Then each sample was added to a Q-PCR system (AB Applied Biosystems ABI, 7500) for reaction, the Q-PCR parameter setting: Target (ROX), the program (25°C, 2min; 1% rate, 95%°C; 95°C, 2min).

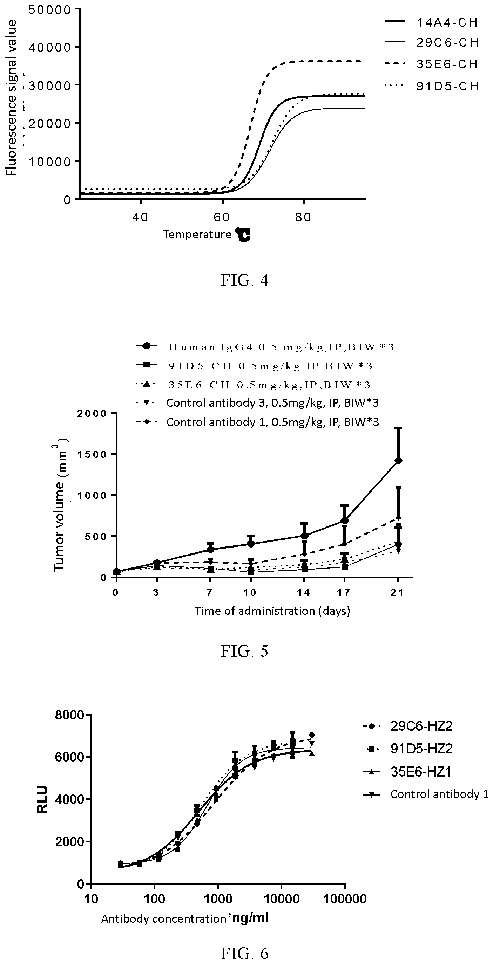

The results were shown in Fig. 4, wherein, the Tm value of anti-PD-1 chimeric antibody 91D5-CH was 72.28°C, the Tm value of 29C6-CH was 71.46°C, the Tm value of 14A4-CH was 68.99°C, the Tm value of 35E6-CH was 66.77°C, these antibodies all had a good thermal stability.

### 4.8 Determination of in vivo drug efficacy of anti-human PD-1 chimeric antibody in hPD-1 transgenic mice

The model was established using the same method as in Example 2.4. When reaching the average volume of about 100 mm³, the mice were randomly divided into the following groups according to tumor size: the group of Human IgG4 (negative control), the group of chimeric antibody 91D5-CH, the group of 35E6-CH antibody, the group of the positive control antibody 1 and the group of the control antibody 3, respectively, each group was injected intraperitoneally with a dosage of 0.5 mg/kg, twice a week for 3 weeks. After administration, the tumor volume and body weight of mice were observed and measured regularly, the specific results were shown in Figure 5. Compared to the negative control, the efficacy of chimeric antibody 91D5-CH group was significant (p<0.05), TGI = 75.46%, wherein, complete tumor regression occurred in 3 of 6 mice; the efficacy of 35E6-CH group was significant (p<0.05), TGI = 73.08%, wherein, complete tumor regression occurred in 3 of 6 mice; the efficacy of the control antibody 3 was significant (p<0.05), TGI = 81.79%, wherein, partial tumor regression occurred only in 1 of 6 mice; the efficacy of the control antibody 1 was significant (p<0.05), TGI = 51.80%, wherein, no tumor regression occurred in any of 6 mice.

### EXAMPLE 5: Humanization of anti-PD-1 antibody

The murine antibodies 29C6, 35E6 and 91D5 were humanized using antibody CDR-grafted methods. In brief, the humanization modification involves the following steps: aligning the amino acid sequence of a murine monoclonal antibody with that of a human germline antibody to find out the sequence with high homology and better physical and chemical properties for serving as the human germline framework sequence; analyzing and investigating the affinity of HLA-DR to select the human germline framework sequence with low affinity; and grafting six CDRs of the murine antibody to the selected heavy chain and light chain framework sequences, respectively.

Specifically, the heavy chain and light chain CDR regions of murine antibodies 29C6, 35E6 and 91D5 were transplanted into the FR frameworks of corresponding humanization templates. The heavy chain humanization template of 29C6 was the human germline gene sequence IGHV1-18^{∗}01 (see IMGT accession number M99641), and the light chain humanization template of 29C6 was the human germline gene sequence IGKV3-11^{∗}01 (see IMGT accession number X01668); the heavy chain humanization template of 35E6 was the human germline gene sequence IGHV4-59^{∗}01 (see IMGT accession number AB019438), and the light chain humanization template of 35E6 was the human germline gene sequence IGKV3-11^{∗}01 (see IMGT accession number X01668); the heavy chain humanization template of 91D5 was the human germline gene sequence IGHV1-18^{∗}01 (see IMGT accession number M99641), and the light chain humanization template of 91D5 was the human germline gene sequence IGKV1-39^{∗}01 (see IMGT accession number X59315).

Furthermore, the framework amino acid sequences of variable regions and neighboring regions thereof were analyzed by molecular docking using computer simulation techniques, to investigate the stereoscopic combination mode. By calculating electrostatic forces, van der Waals forces, hydrophilicity and hydrophobicity as well as entropy values, the key amino acids in the amino acid sequence of the murine antibody that can interact with PD-1 and maintain the spatial conformation were analyzed, and retained in the transplanted antibody. Namely, a series of back mutations were carried out on the amino acid residues in the FR regions of the humanization template, so that the humanized antibody could retain the antigen-binding ability of the murine antibody as much as possible.

According to the above methods, based on the CDRs of murine antibody 29C6, two humanized antibodies were constructed, denominated as 29C6-HZ1 and 29C6-HZ2, respectively; based on the CDRs of murine antibody 35E6, one humanized antibody was constructed, denominated as 35E6-HZ1; based on the CDRs of murine antibody 91D5, two humanized antibodies were constructed, denominated as 91D5-HZ1 and 91D5-HZ2, respectively. The heavy chain constant region of each humanized antibody was human IgG4 heavy chain constant region (SEQ ID NO: 82), and the light chain constant region thereof was human light chain kappa constant region (SEQ ID NO: 84).

The amino acid sequences of the variable regions and constant regions of the humanized antibodies above were shown in Table 6.

**Table 6 Amino acid sequences of variable regions and constant regions of humanized antibodies**

| Name | Heavy chain variable region (SEQ ID NO:) | Light chain variable region (SEQ ID NO:) | Heavy chain constant region (SEQ ID NO:) | Light chain constant region (SEQ ID NO:) |
|---|---|---|---|---|
| 29C6-HZ1 | 16 | 18 | 82 | 84 |
| 29C6-HZ2 | 17 | 19 | | |
| 35E6-HZ1 | 34 | 35 | | |
| 91D5-HZ1 | 51 | 53 | | |
| 91D5-HZ2 | 52 | 53 | | |

### EXAMPLE 6: Evaluation of anti-PD-1 humanized antibody

### 6.1 Determination of binding affinity of humanized antibody to human PD-1-His by ELISA

To verify the affinity of the humanized PD-1 antibody to PD-1-His antigen, ELISA was employed. The specific experimental steps were as follows: human PD-1-his was diluted to 1µg/mL with CBS solution, then coated on a 96-well ELISA plate (purchased from Guangzhou Jiete Bio) at 4°C overnight. After washing, 100µL of phosphate buffer (PBS) containing 2% BSA was used for blocking at 37°C for 1 hour, followed by adding 100µL of chimeric antibodies 29C6-CH, 35E6-CH, 91D5-CH and humanized antibodies 29C6-HZ1, 29C6-HZ2, 35E6-HZ1, 91D5-HZ1, 91D5-HZ2 (starting at 10µg/mL, 3-fold gradient dilution, 2 replicates), and incubating at 37°C for 2 hours. After washing, a goat anti-mouse secondary antibody (FC) labeled with horseradish peroxidase diluted in phosphate buffer (PBS) containing 2% BSA in a ratio of 1:15000 was added, followed by incubating at 37°C for 1 hour. After washing, the TMB chromogen solution was used for developing, and the absorbance value was read using a microplate reader at 450nm. The data were imported into Graphprism to calculate EC₅₀ values.

The results were shown in Table 7, in whole, the humanization had little effect on affinity, and retained a good binding affinity to human PD-1-His.

**Table 7 ELISA affinitiy of anti-PD-1 chimeric antibody, anti-PD-1 humanized antibody for PD-1-His**

| Plate number | Antibody name | EC₅₀ (nM) |
|---|---|---|
| 1 | 29C6-CH | 0.17 |
| | 29C6-HZ1 | 0.42 |
| | 29C6-HZ2 | 0.16 |
| 2 | 35E6-CH | 0.20 |
| | 35E6-HZ1 | 0.30 |
| | 91D5-CH | 0.29 |
| | 91D5-HZ1 | 0.39 |
| | 91D5-HZ2 | 0.42 |

### 6.2 Determination of dynamic affinity of anti-human PD-1 humanized antibody to PD-1-His

The dynamic affinity of anti-human PD-1 humanized antibodies to PD-1-His was determined using Octet ForteBio^{®}, including chimeric antibodies 29C6-CH and 91D5-CH, humanized antibodies 29C6-HZ2, 35E6-HZ1, 91D5-HZ1, 91D5-HZ2 and the control antibody 1, and specific experimental steps refer to Example 4.5. The experimental results were shown in Table 8, and the KD values of each of chimeric antibodies and humanized antibodies were at the level of nM, and stronger than that of the control antibody 1.

**Table 8 Dynamic affinity of anti-PD-1 chimeric antibody, anti-PD-1 humanized antibody to PD-1-His**

| Antibody name | KD (M) | kon (1/Ms) | kdis (1/s) | Full R^2 |
|---|---|---|---|---|
| 29C6-CH | 5.16E-10 | 3.22E+05 | 1.66E-04 | 0.9982 |
| 29C6-HZ2 | 1.65E-09 | 2.75E+05 | 4.54E-04 | 0.9983 |
| 35E6-HZ1 | 1.31E-09 | 3.95E+05 | 5.17E-04 | 0.998 |
| 91D5-CH | 5.47E-10 | 3.24E+05 | 1.77E-04 | 0.9989 |
| 91D5-HZ1 | 1.89E-09 | 2.81E+05 | 5.30E-04 | 0.9986 |
| 91D5-HZ2 | 1.50E-09 | 3.15E+05 | 4.72E-04 | 0.9989 |
| Control antibody 1 | 5.23E-09 | 5.20E+05 | 2.72E-03 | 0.9963 |

### 6.3 Determination of the activity of anti-human PD-1 humanized antibody for blocking human PD-1/human PD-L1 binding and human PD-1/human PD-L2 binding

The activity of anti-human PD-1 humanized antibodies for blocking human PD-1/human PD-L1 binding and human PD-1/human PD-L2 binding was detected by competitive ELISA, including chimeric antibodies 29C6-CH, 91D5-CH and 35E6-CH, as well as humanized antibodies 29C6-HZ1, 29C6-HZ2, 35E6-HZ1, 91D5-HZ1 and 91D5-HZ2, and the control antibody 1, and see Example 4.3 for specific experimental steps and the result analysis. The results were shown in Table 9. All the antibodies had a good activity of blocking the binding of human PD-1/human PD-L1 or human PD-1/human PD-L2.

**Table 9 The activity of anti-PD-1 chimeric antibody and anti-PD-1 humanized antibody for blocking human PD-1/human PD-L1 binding and human PD-1/human PD-L2 binding**

| Antibody name | PD-1-hFc/PDL1-mFc IC₅₀ (nM) | PD-1-hFc/PDL2-mFc IC₅₀ (nM) |
|---|---|---|
| 29C6-CH | 1.15 | 1.31 |
| 29C6-HZ1 | 3.11 | 3.63 |
| 29C6-HZ2 | 0.88 | 0.95 |
| 35E6-CH | 0.88 | 1.11 |
| 35E6-HZ1 | 0.94 | 1.15 |
| 91D5-CH | 0.96 | 1.08 |
| 91D5-HZ1 | 1.19 | 1.25 |
| 91D5-HZ2 | 1.13 | 1.22 |
| Control antibody 1 mean ± error | 0.98±0.13 | 1.42±0.22 |

### 6.4 Determination of functions of anti-PD-1 humanized antibodies by a luciferase reporter system

Cytological functions of anti-human PD-1 humanized antibodies were detected using a luciferase reporter system, including humanized antibodies 29C6-HZ2, 35E6-HZ1, 91D5-HZ2 and the control antibody 1, and see Example 4.6 for specific experimental steps. The results were shown in Fig. 6, wherein, EC₅₀ values from the luciferase reporter system activated by humanized antibodies 29C6-HZ2, 35E6-HZ1, 91D5-HZ2 and the control antibody 1 were 5.74 nM, 3.63 nM, 4.47 nM and 3.38 nM, respectively.

### 6.5 Anti-PD-1 humanized antibodies promote staphylococcal enterotoxin B (SEB) to stimulate PBMC cells to secret IL-2

In this experiment, the level of IL-2 cytokines secreted by SEB-stimulated PBMC cells under the effect of anti-PD-1 humanized antibodies 91D5-HZ2 and 29C6-HZ2, the control antibody 1, the control antibody 3 and the IgG control antibody, respectively, was measured by HTRF assay to evaluate the biological activity of these antibodies.

PBMC cells were prepared from human fresh blood from two donors A and B, respectively, by using a density gradient centrifugation. Fresh PBMC cells were resuspended in a medium containing 1 ng/mL SEB and adjusted to a cell density of 5×10⁵/mL, then evenly distributed into a 96-well plate at 100µL per well (5×10⁴ cells/well). The antibodies were diluted with 3-fold gradient, and added at 100µL per well. The antibody concentration gradient started at 1µM, 3-fold dilution, 2 replicates for each concentration of each antibody. After 72 hours, the cell supernatant was collected by centrifugation, and the secretion of IL-2 in the supernatant was detected by the IL-2 HTRF kit (CisBio).

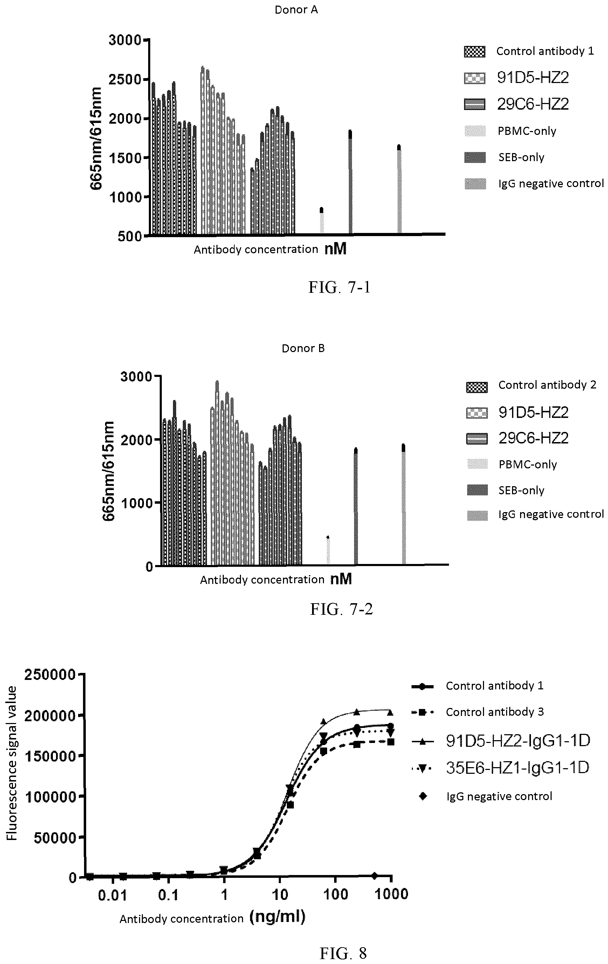

The results were shown in Fig. 7-1 and Fig. 7-2, wherein, in PBMC cells derived from two donors, with increased concentrations of antibodies 91D5-HZ2 and 29C6-HZ2, the control antibody 1, or the control antibody 2, the amount of IL-2 produced by PBMC cells also increased, showing the ability to enhance immune cell response. The above results indicated that 91D5-HZ2, 29C6-HZ2, the control antibody 1 and the control antibody 2 all had the ability to enhance T cell activity, and 91D5-HZ2 had a stronger promoting effect.

### EXAMPLE 7: Design and validation of anti-PD-1 humanized antibody Fc

### 7.1: IgG1-1D mutant anti-PD-1 humanized antibody

Since PD-1 is mainly expressed in activated T cells, at present, IgG4 subtype is used for all the PD-1 antibodies on market, in order to prevent PD-1 positive T cells from being killed. Many studies have shown that human IgG4 antibody subtypes have moderate ADCC, almost without CDC effector function. However, natural IgG4 has been found to be less stable under pressure conditions (such as in acidic buffers, or the like) (Boning Liu, Huaizhu Guo, Jin Xu et. al., Acid-induced aggregation property of nivolumab is dependent on the Fc. Mabs, 2016, 1942-0862).

The IgG1 subtype having better stability, and the hinge region mutation can weak ADCC and CDC functions, meanwhile eliminate the affinity of FcγRI, and avoid the clearance of T cells by monocytiv myeloid-derived suppressor cells (MDSC); on this basis, mutation of Fc constant region can enhance the affinity of FcRn and prolong the half-life in vivo, to obtain a better drug efficacy. The inventors used the constant region of mutant IgG1 subtype Fc, including L234A, L235A, D265A, N434A point mutations (EU numbering, SEQ ID NO: 83) to remove FcR binding and prolong the affinity of FcRn. As shown in Table 10, the nucleic acid coding sequences of heavy chain sequence (amino acid SEQ ID NO: 86) and light chain sequence (amino acid SEQ ID NO: 88) of 91D5-HZ2-IgG1-1D, and of heavy chain sequence (amino acid SEQ ID NO: 90) and light chain sequence (amino acid SEQ ID NO: 11) of 35E6-HZ1-IgG1-1D were constructed into pTT5 expression vectors, respectively, and transiently transfected into CHO-S cells by PEI for expression, after purification by ProA, 91D5-HZ2-IgG1-1D and 35E6-HZ1-IgG1-1D antibodies were obtained.

**Table 10 Amino acid sequence of IgG1-1D mutant anti-PD-1 humanized antibody**

| Name | Heavy chain variable region (SEQ ID NO:) | Light chain variable region (SEQ ID NO:) | Heavy chain constant region (SEQ ID NO:) | Light chain constant region (SEQ ID NO:) |
|---|---|---|---|---|
| 91D5-HZ2-IgG1-1D | 52 | 53 | 83 | 84 |
| 35E6-HZ1-IgG1-1D | 34 | 35 | | |

### 7.2 Detection of affinity of IgG1-1D mutant anti-PD-1 humanized antibody to PD-1 antigen by ELISA

The affinities of 91D5-HZ2-IgG1-1D, 35E6-HZ1-IgG1-1D, the control antibody 1 and the control antibody 3 for human PD-1-mFc and monkey PD-1-hFc were determined by ELISA. See Example 4.2 and Example 6.1 for specific experimental steps, and the results were shown in Table 11, wherein, the ELISA binding affinity of each of the four PD-1 antibodies to human PD-1-mFc and monkey PD-1-hFc was at the level of nM. For the binding activity to human PD-1-mFc, 91D5-HZ2-IgG1-1D was slightly stronger than the control antibody 1 and the control antibody 3, and 35E6-HZ1-IgG1-1D was comparable to the control antibody 1 and the control antibody 3.

**Table 11 Affinity and blocking activity of IgG1-1D mutant anti-PD-1 humanized antibody**

| Antibody name | Human PD-1-mFc EC₅₀ (nM) | Monkey PD-1-hFc EC₅₀ (nM) | CHO-hPD-1 cell affinity EC₅₀ (nM) | Blocking CHO-hPD-1/PD-L1 binding IC₅₀ (ng/mL) |
|---|---|---|---|---|
| 91D5-HZ2-IgG1-1D | 0.104 | 0.250 | 0.093 | 1.323 |
| 35E6-HZ1-IgG1-1D | 0.121 | 0.309 | 0.078 | 0.818 |
| Control antibody 1 | 0.121 | 0.320 | 0.090 | 1.299 |
| Control antibody 3 | 0.121 | 0.278 | 0.094 | 1.343 |

### 7.3 Detection of dynamic affinity of IgG1-1D mutant anti-PD-1 humanized antibody to PD-1 antigen

The dynamic affinities of IgG1-1D mutant anti-PD-1 humanized antibodies for PD-1-His were detected by Octet ForteBio^{®}, including 91D5-HZ2-IgG1-1D, 35E6-HZ1-IgG1-1D, the control antibody 1 and the control antibody 3, and see Example 4.5 for specific experimental steps. The experimental results were shown in Table 12, wherein, the dynamic affinities of the four antibodies were all at the level of nM, and the affinities of 91D5-HZ2-IgG1-1D and 35E6-HZ1-IgG1-1D were about 4-5 times stronger than those of the control antibody 1 and the control antibody 3, indicating they had a stronger binding activity.

**Table 12 Dynamic affinity of IgG1-1D mutant anti-PD-1 humanized antibody to PD-1-His**

| Antibody name | KD (M) | kon (1/Ms) | kdis (1/s) | Full R^2 |
|---|---|---|---|---|
| 91D5-HZ2-IgG1-1D | 1.17E-09 | 3.84E+05 | 2.30E-04 | 0.9992 |
| 35E6-HZ1-IgG1-1D | 1.32E-09 | 3.33E+05 | 3.06E-04 | 0.9987 |
| Control antibody 1 | 5.02E-09 | 8.87E+05 | 4.45E-04 | 0.9926 |
| Control antibody 3 | 5.95E-09 | 5.85E+05 | 3.48E-04 | 0.9952 |

### 7.4 Detection of affinity of IgG1-1D mutant anti-PD-1 humanized antibody toCHO-hPD-1.

To verify the binding of IgG1-1D mutant anti-PD-1 humanized antibody to PD-1 on the cell membrane surface, FACS technology was used to detect the affinity of murine antibodies to CHO-hPD-1 cells.

The specific experimental steps were as follows: CHO-hPD-1 cells were centrifuged at 500g for 3 minutes, and resuspended with PBS (containing 1% BSA) to 6×10⁶ cells/mL, and then 50µL was taken to corresponding 96-well plates; FITC labeled 91D5-HZ2-IgG1-1D, 35E6-HZ1-IgG1-1D, the control antibody 1 and the control antibody 3 were diluted with PBS (containing 1% BSA) (starting at 1µM, 4-fold gradient dilution, 11 concentration points), and then 50µL was added to corresponding 96-well plates, 1µM FITC-labeled IgG negative control antibody was used as negative control, followed by placing at 4°C for 1h; washing with PBS for 3 times, and detected by flow cytometry (manufacturer: Beckman, Model: CytExpert), and the data were imported into Graphprism to calculate the EC₅₀ values.

The experimental results were shown in Fig. 8 and Table 11, 91D5-HZ2-IgG1-1D and the control antibody 1 and the control antibody 3 had comparable binding affinities for PD-1 on the cell membrane surface of CHO-hPD-1, while 35E6-HZ1-IgG1-1D had slightly stronger affinity than the control antibody 1 and the control antibody 3.

### 7.5: Detection of activity of IgG1-1D mutant anti-PD-1 humanized antibody for blocking the binding of CHO-hPD-1 to PD-L1

To verify that IgG1-1D mutant anti-PD-1 humanized antibody blocks the binding of CHO-hPD-1 to PD-L1, FACS technology was used for detection, and see Example 4.4 for the specific experimental steps.

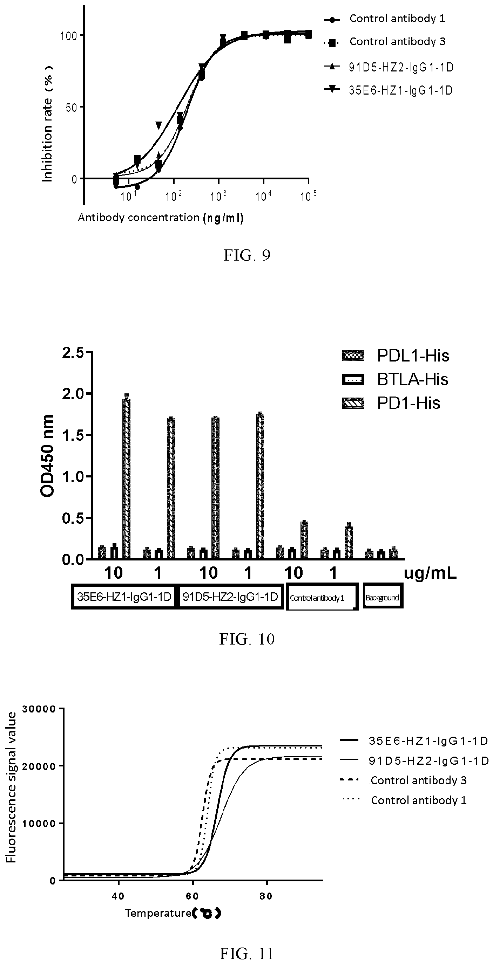

The experimental results were shown in Fig. 9 and Table 11. The blocking activity of 91D5-HZ2-IgG1-1D is comparable to that of the control antibody 1 and the control antibody 3, and the blocking activity of 35E6-HZ1-IgG1-1D is slightly stronger than that of the control antibody 1 and the control antibody 3.

### 7.6 Specific detection of IgG1-1D mutant anti-PD-1 humanized antibody

The nonspecific binding of IgG1-1D mutant anti-PD-1 humanized antibody to HUVEC and HEK293T epithelial cells was detected by FACS. In brief, the density of HUVEC or HEK293T cells was set to be 2×10⁵ cells per well. Test antibodies 91D5-HZ2-IgG1-1D, 35E6-HZ1-IgG1-1D, the control antibody 1 and the control antibody 3 were labeled by FITC and diluted to 500nM, then incubated with HUVEC or HEK293T cells at 4°C for 1 hour, respectively. After washing, the cells were detected and analyzed by flow cytometry (manufacturer: Beckman, model: CytExpert). The results showed that no nonspecific binding was observed.

The nonspecific binding of IgG1-1D mutant anti-PD-1 humanized antibody to BTLA-his, PD-L1-his and ICOS was detected by ELISA. The specific experimental steps were as follows: human PD-1-his, human BTLA-his, human ICOS and human PD-L1-his (purchased from Novoprotein) were diluted with CBS solution to 1µg/mL, then coated on a 96-well ELISA plate (purchased from Guangzhou Jiete Bio) at 4°C overnight. After washing, 100µL of phosphate buffer (PBS) containing 2% BSA was used for blocking at 37°C for 1 hour, followed by adding 100µL of 91D5-HZ2-IgG1-1D, 35E6-HZ1-IgG1-1D and the control antibody 1 (10µg/mL and 1µg/mL), setting 2 replicates, incubated at 37°C for 2 hours. After washing, a goat anti-human IgG secondary antibody labeled with horseradish peroxidase diluted in phosphate buffer (PBS) containing 2% BSA in a ratio of 1:15000 was added, followed by incubating at 37°C for 1 hour. After washing, the TMB chromogen solution was used for developing, and the absorbance value was read using a microplate reader at 450nm. The data were imported into Graphprism to calculate EC₅₀ values. The experimental results were shown in Fig. 10, wherein, as compared with the background (blank control), 91D5-HZ2-IgG1-1D, 35E6-HZ1-IgG1-1D and the control antibody 1 all specifically bind to human PD-1 antigen, but do not bind to BTLA-his, PD-L1-his or ICOS.

### 7.7 Determination of Tm of IgG1-1D mutant anti-PD-1 humanized antibody

The Tm values of 91D5-HZ2-IgG1-1D, 35E6-HZ1-IgG1-1D, the control antibody 1 and the control antibody 3 were detected by DSF (Differential Scanning Fluorimetry), and see Example 4.7 for specific experimental steps. The results were shown in Fig. 11, wherein, the Tm values of antibodies 91D5-HZ2-IgG1-1D (67.62°C) and 35E6-HZ1-IgG1-1D (66.58°C) were 4-5°C higher than that of the control antibody 1 (63.95°C) and the control antibody 3 (62.56°C), showing better thermal stability, favoring the drug development.

### 7.8 Determination of IgG1-1D mutant anti-PD-1 humanized antibody by a luciferase reporter system

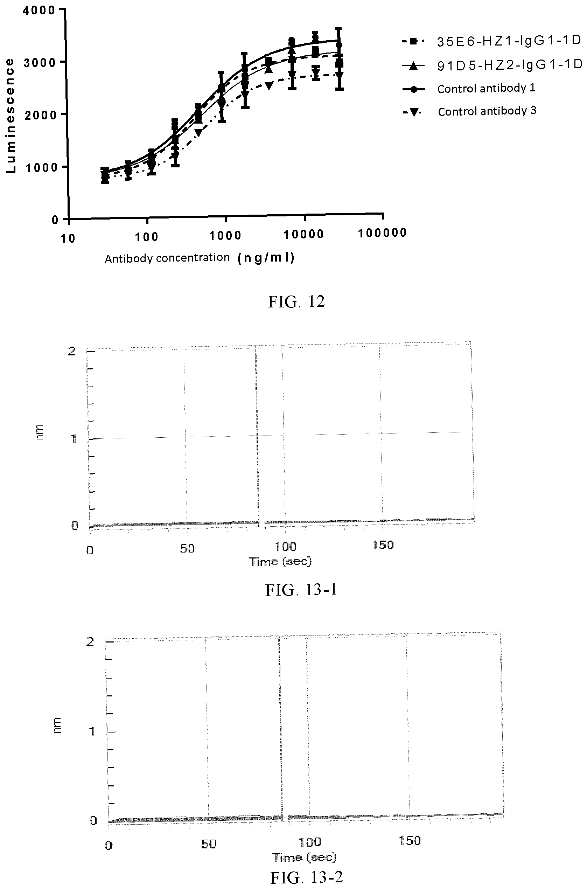

Cytological functions of IgG1-1D mutant anti-PD-1 humanized antibodies were determined by a luciferase reporter system, and see Example 4.6 for specific experimental steps. The experimental results were shown in Fig. 12, wherein, the EC₅₀ values of 91D5-HZ2-IgG1-1D, 35E6-HZ1-IgG1-1D, the control antibody 1 and the control antibody 3 by the luciferase reporter system were 3.48 nM, 2.81 nM, 3.22 nM, and 3.79 nM, respectively.

### 7.9 Detection of dynamic affinity of IgG1-1D mutant anti-PD-1 humanized antibody to CD64 Protein

The binding of antibody Fc to CD64 (i.e., FcγRI) is one important aspect in exerting ADCP function, and the dynamic affinity of IgG1-1D mutant anti-PD-1 humanized antibodies to CD64 (purchased from ACRO) was detected by Octet ForteBio^{®}, including 91D5-HZ2-IgG1-1D, 35E6-HZ1-IgG1-1D, the control antibody 1 and the control antibody 3. The specific experimental steps were as follows: by using SA biosenser (purchased from Pall), firstly immobilizing CD64 protein diluted to 4µg/mL with PBST; the antibody was diluted to 100 nM, 50 nM, 25 nM, 12.5 nM, 6.25 nM, 3.125 nM, 1.5625 nM, 0 nM with PBST (pH7.0); allowing for binding for 90 seconds, then dissociating for 120 seconds, and the data were analyzed by 1:1 model and global fitting.

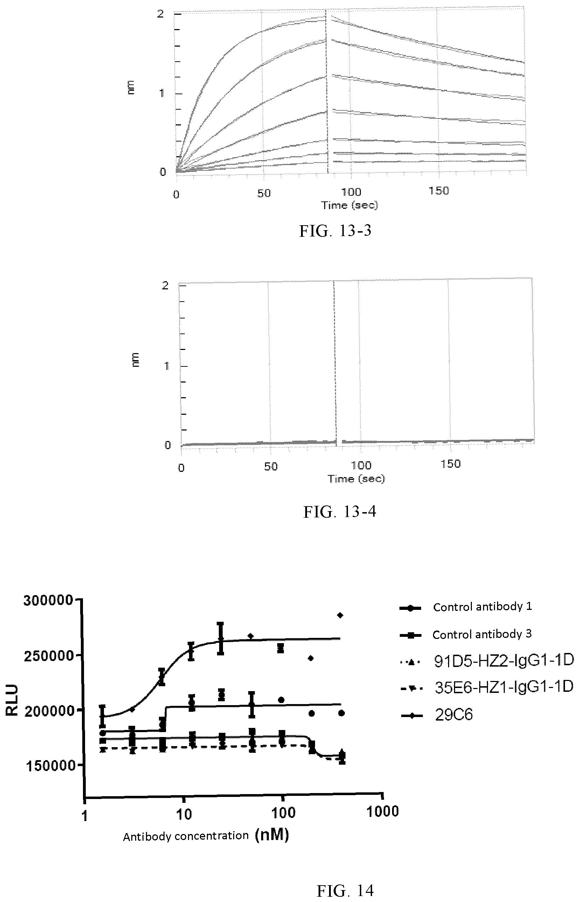

The experimental results were shown in Figs. 13-1 to 13-4, corresponding to the dynamic affinities of 91D5-HZ2-IgG1-1D, 35E6-HZ1-IgG1-1D, the control antibody 1 and the control antibody 3 for CD64, respectively, wherein, 91D5-HZ2-IgG1-1D, 35E6-HZ1-IgG1-1D and the control antibody 3 did not bind to CD64 at all, and the dynamic affinity values of the control antibody 1 were as follows: KD=6.94E-09 (M), Kon=2.07E-10 (1/Ms) and kdis=3.85E-04 (1/s), indicating that the antibodies 91D5-HZ2-IgG1-1D and 35E6-HZ1-IgG1-1D did not generate ADCP activity.

### 7.10 Detection of affinity of IgG1-1D mutant anti-PD-1 humanized antibody to FcRn protein

The antibody Fc can bind to FcRn so that it cannot be easily cleared in vivo, thus prolonging the half-life in vivo. The dynamic affinities of IgG1-1D mutant anti-PD-1 humanized antibodies to FcRn (purchased from ACRO) were measured by Octet ForteBio^{®}, including 91D5-HZ2-IgG1-1D, 35E6-HZ1-IgG1-1D, the control antibody 1 and the control antibody 3. The specific experimental steps were as follows: by using a SA biosenser (purchased from Pall), firstly immobilizing FcRn protein diluted to 3µg/mL with PBST; diluting the antibody to 200 nM, 100 nM, 50 nM, 25 nM, 12.5 nM, 6.25 nM, 3.125 nM, 0 nM with PBST (pH6.0); allowing for binding for 90 seconds, then dissociating for 120 seconds, and the data were analyzed by 1:1 model and global fitting.

The experimental results were shown in Table 13, wherein, the affinities of 91D5-HZ2-IgG1-1D and 35E6-HZ1-IgG1-1D for FcRn were at the level of E-09 (M), and the affinities of the control antibody 1 and the control antibody 2 for FcRn were at the level of E-08 (M). The affinity of IgG1-1D mutant anti-PD-1 humanized antibody was 2-3 times stronger than that of the control antibody, and its half-life in vivo may be prolonged.

**Table 13 Dynamic affinity of IgG1-1D mutant anti-PD-1 humanized antibody to FcRn**

| Antibody name | KD (M) | kon (1/Ms) | kdis (1/s) | Full R^2 |
|---|---|---|---|---|
| 91D5-HZ2-IgG1-1D | 5.33E-09 | 1.36E+06 | 7.25E-03 | 0.9976 |
| 35E6-HZ1-IgG1-1D | 7.89E-09 | 9.46E+05 | 7.46E-03 | 0.9977 |
| Control antibody 1 | 1.68E-08 | 1.24E+06 | 2.09E-02 | 0.9966 |
| Control antibody 3 | 1.45E-08 | 1.29E+06 | 1.87E-02 | 0.9969 |

### 7.11 Determination of ADCP activity of IgG1-1D mutant anti-PD-1 humanized antibody by a luciferase reporter system

The binding of the antibody Fc to CD32a is one important aspect in exerting ADCP function, and the ADCP function of IgG1-1D mutant anti-PD-1 humanized antibody was detected by a luciferase reporter system. Specific experimental steps were as follows: Jurkat-NFAT/CD32a and CHO-S-PD-1 cells (Sichuan Kelun Biotech Biopharmaceutical Co., Ltd.) were taken and centrifuged, resuspended with RPMI1640 + 1% FBS medium, then counted and diluted to 2×10⁶ cells/mL and 1×10⁶ cells/mL, respectively, and 50µL of cell suspension was added per well; 91D5-HZ2-IgG1-1D, 35E6-HZ1-IgG1-1D, the control antibody 1, the control antibody 3 and the murine PD-1 antibody 29C6 were diluted (starting at 1.2µM, 2-fold dilution, 2 replicates), and added to corresponding wells at 50µL/well; after incubation at 37°C for 5 hours, 20µL of one-glo detection reagent (purchased from Promega) was added per well for detection by a microplate reader.

The results were as shown in Fig. 14, wherein, murine PD-1 antibody 29C6 had strong ADCP activity, EC₅₀=6.09nM; the control antibody 1 had weak ADCP activity; while 91D5-HZ2-IgG1-1D, 35E6-HZ1-IgG1-1D and the control antibody 3 had no ADCP activity at all.

### 7.12 Determination of ADCC function of IgG1-1D mutant anti-PD-1 humanized antibody by a luciferase reporter system

The binding of the antibody Fc to CD16a is one important aspect in exerting ADCC function, and the ADCC function of IgG1-1D mutant anti-PD-1 humanized antibody was detected by a luciferase reporter system. Specific experimental steps were as follows: Jurkat-NFAT/CD16a and CHO-S-PD-1 cells (Sichuan Kelun Biotech Biopharmaceutical Co., Ltd.) were taken and centrifuged, resuspended with RPMI1640 + 1% FBS medium, then counted and diluted to 2×10⁶ cells/mL and 1×10⁶ cells/mL, respectively, 50µL of cell suspension was added per well; 91D5-HZ2-IgG1-1D, 35E6-HZ1-IgG1-1D, the control antibody 1, the control antibody 3 were diluted (starting at 450µg/mL, 10-fold dilution, 2 replicates), and the murine PD-1 antibody 42G1 (450µg/mL), which were added to corresponding wells at 50µL/well; after incubation at 37°C for 5 hours, 20µL of one-glo detection reagent (purchased from Promega) was added per well for detection by a microplate reader.

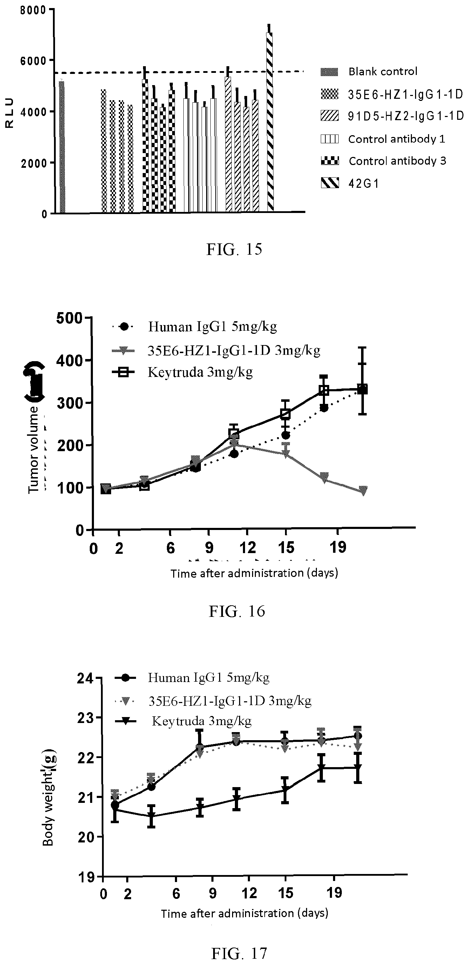

The results were as shown in Fig. 15, wherein, the murine PD-1 antibody 42G1 had strong ADCC activity, while 91D5-HZ2-IgG1-1D, 35E6-HZ1-IgG1-1D, the control antibody 1 and the control antibody 3 had no ADCC activity at all.

### EXAMPLE 8: In vivo drug efficacy test of IgG1-1D mutant anti-PD-1 humanized antibody

The inhibitory effects of 35E6-HZ1-IgG1-1D and 91D5-HZ2-IgG1-1D antibodies on the proliferation of subcutaneous transplantation tumor in human immune system reconstructed mice or transgenic mice were evaluated in Examples 8.1 to 8.3. Specifically, in the invention, the antibodies 35E6-HZ1-IgG1-1D and 91D5-HZ2-IgG1-1D were administered to a Hu-PBMC-B-NDG mouse model transplanted subcutaneously with human non-small cell lung cancer HCC827 and a hPD-1/hTIM3 transgenic mouse model transplanted subcutaneously with mouse colon cancer cell line MC38 by tail intravenous injection or intraperitoneal injection, twice a week for 3 weeks. The changes in tumor volume and body weight of animals were measured twice a week, and the efficacy (tumor growth inhibition) of the antibody of the invention on tumor-bearing mice was calculated.

Test drug: taking an appropriate amount of 35E6-HZ1-IgG1-1D or 91D5-HZ2-IgG1-1D antibody, calculating the dosage volume according to 10µL/g, and diluting the mother solution into corresponding volume of dosing solution with normal saline according to the dosage to be administered. The same dose of human anti-chicken lysozyme immunoglobulin Human IgG1 (Sichuan Kelun Biotech Biopharmaceutical Co., Ltd.) and human immunoglobulin Human IgG (Chengdu Rongsheng Pharmaceuticals Co., Ltd.) were used as negative control, and Keytruda (purchased from Merck, Cat. No. S001195) was used as positive control, and AB12T8 (prepared with reference to WO2019141092) was for combined administration.

Experimental animals and cell lines: B-NDG mice (Biocytogen Jiangsu Co., Ltd), hPD-1/hTIM-3 transgenic mice (Jiangsu GemPharmatech Co., Ltd.), mouse colon cancer cell line MC38 (Nanjing Cobioer Gene Technology Co., Ltd), human non-small cell lung cancer cell line HCC827 (ATCC), and human peripheral blood mononuclear lymphocyte PBMC (AllCELLS).

### Experiment grouping and evaluation methods

The tumor-bearing mice with an average tumor volume of 70-150 mm³ were randomly divided into groups (the number of groups was determined according to the number of samples). Human anti-chicken lysozyme immunoglobulin Human IgG1, intravenous human immunoglobulin Human IgG or the antibody of the invention was administered according to the groups, respectively, twice a week for 3 weeks, wherein the route of administration was intraperitoneal injection or tail vein injection, and the dosing volume was 10 mL/kg. The tumor diameter was measured by a vernier caliper twice a week after administration, and the tumor volume was calculated according to the following formula: V = 0.5 a × b², wherein a and b denote the long and short diameter of the tumor, respectively. Animal mortality was observed and recorded daily.

The tumor growth inhibition TGI (%) was calculated by the following formula to evaluate the tumor inhibition effect of the antibody of formula (I): TGI (%) = [1-(V_{Tend}-V_{T0})/(V_{Cend}-V_{C0})] ^{∗} 100%, wherein, V_{Tend}: the mean value of tumor volume at the end of the experiment in the treated group, V_{T0}: the mean value of tumor volume at the beginning of dosing in the treated group, V_{Cend}: the mean value of tumor volume at the end of the experiment in the negative control group, V_{C0}: the mean value of tumor volume at the beginning of dosing in the negative control group, and the relative tumor proliferation rate T/C (%) was calculated by the following formula to evaluate the anti-tumor efficacy of the antibody of formula (I): T/C = (V_{Tend}/V_{T0})/(V_{Cend}/V_{C0}).

### 8.1 Determination of in vivo drug efficacy of 35E6-HZ1-IgG1-1D in human immune system reconstructed mice hPBMC-B-NDG-HCC827 model 1

HCC827 (non-small cell lung cancer) cells were cultured with RPMI1640 culture medium containing 10% fetal bovine serum at 37°C, 5% CO₂. HCC827 cells in exponential growth phase were collected, then resuspended to a suitable concentration with PBS and inoculated subcutaneously into female B-NDG mice to establish a non-small cell lung cancer model. PBMC was thawed in the next day, resuspended to a suitable concentration with PBS, and then inoculated intraperitoneally into tumor-bearing mice to rebuild their immune systems. When tumors reaching the average volume of about 97 mm³, the mice were randomly divided into the following groups according to tumor size: human anti-chicken lysozyme immunoglobulin Human IgG1 (negative control) group, 35E6-HZ1-IgG1-1D antibody group and Keytruda group, injected via tail vein, twice a week for three weeks. After dosing, the tumor volume and body weight of mice were observed and measured regularly. The specific results were shown in Table 14, as shown in Figure 16-17, and compared to Human IgG1 negative control group, 35E6-HZ1-IgG1-1D antibody group significantly inhibited the tumor growth of HCC827 non-small cell lung cancer transplanted tumor model, with the tumor growth inhibition (TGI) of 104.94%, while Keytruda group did not show any obvious anti-tumor effect. Neither animal death nor significant animal weight loss nor obvious drug toxicity was observed in all treatment groups during the observation period, and there was a good tolerance in the mice during the treatment.

**Table 14. Drug efficacy result of 35E6-HZ1-IgG1-1D antibody in hPBMC-B-NDG-HCC827 human immune reconstitution model**

| Grouping | Group | P21 | | | |
|---|---|---|---|---|---|
| | | Tumor volume (mm³) (*x̅*± SEM) | TGI (%) | T/C (%) | P value (*Vs*. Group 1) |
| 1 | Human IgG1 5mg/kg | 326.09±243.95 | -- | -- | -- |
| 2 | 35E6-HZ1-IgG1-1D 3mg/kg | 85.40±25.96 | 104.97 | 26.16 | 0.04 |
| 3 | Keytruda 3mg/kg | 327.67±146.52 | -0.68 | 100.46 | 0.99 |

### 8.2 Determination of in vivo drug efficacy of 91D5-HZ2-IgG1-1D in human immune system reconstructed mice hPBMC-B-NDG-HCC827 model 2

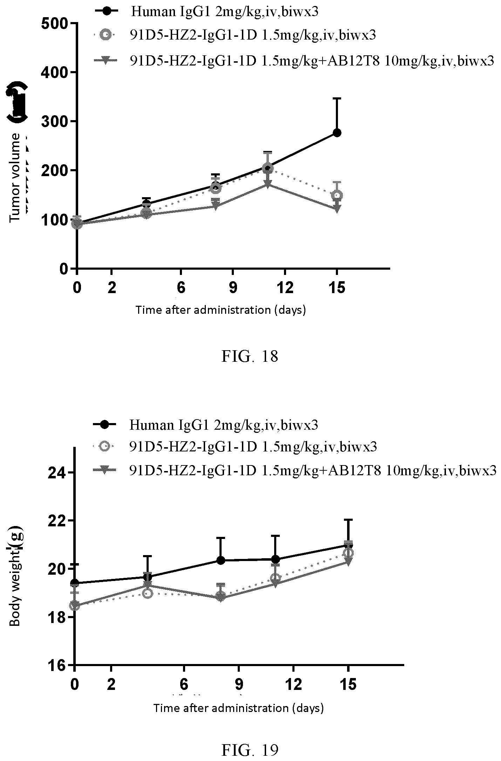

HCC827 cells were cultured with RPMI1640 culture medium containing 10% fetal bovine serum at 37°C, 5% CO₂. HCC827 cells in exponential growth phase were collected, then resuspended to a suitable concentration with PBS and inoculated subcutaneously into female B-NDG mice to establish a non-small cell lung cancer model. Five days after tumor cells were inoculated, PBMC was thawed, then resuspended to a suitable concentration with PBS, and then inoculated intraperitoneally into the tumor-bearing mice to rebuild their immune systems. When tumors reaching an average volume of about 91 mm³, the mice were randomly divided into the following groups according to tumor size: the group of human anti-chicken lysozyme immunoglobulin Human IgG1 (negative control), the group of single 91D5-HZ2-IgG1-1D antibody, and the group of 91D5-HZ2-IgG1-1D antibody in combination with LAG3 monoclonal antibody (AB12T8, Sichuan Kelun Biotech Biopharmaceutical Co., Ltd.), respectively, which were injected by tail vein, twice a week for 2 weeks. After administration, the tumor volume and body weight of mice were observed and measured regularly, and the specific results were shown in Table 15, as shown in Figure 18-19. Compared to Human IgG1 negative control group, the group of single 91D5-HZ2-IgG1-1D antibody had a good tumor inhibition trend, with TGI of 69.13%, and the group of 91D5-HZ2-IgG1-1D antibody in combination with LAG3 monoclonal antibody had further enhanced tumor inhibition effect, with TGI of 83.40%. Neither animal death nor significant animal weight loss nor obvious drug toxicity was observed in all treatment groups during the observation period, showing a good tolerance in the mice during the treatment.

**Table 15. Drug efficacy results of 91D5-HZ2-IgG1-1D antibody in hPBMC-B-NDG-HCC827 human immune reconstitution model**

| Grouping | Group | P15 | | | |
|---|---|---|---|---|---|
| | | Tumor volume (mm³) (*x̅*± SEM) | TGI (%) | T/C (%) | P value (*Vs*. Group 1) |
| 1 | Human IgG1 2mg/kg | 277.06±69.49 | -- | -- | -- |
| 2 | 91D5-HZ2-IgG1-1D 1.5 mg/kg | 149.25±27.13 | 69.13 | 53.98 | 0.12 |
| 3 | 91D5-HZ2-IgG1-1D 1.5 mg/kg + AB12T8 10 mg/kg | 121.51±18.96 | 83.40 | 44.63 | 0.06 |

### 8.3 Determination of in vivo drug efficacy of IgG1-1D mutant anti-PD-1 humanized antibody in hPD-1/hTIM-3 double transgenic model

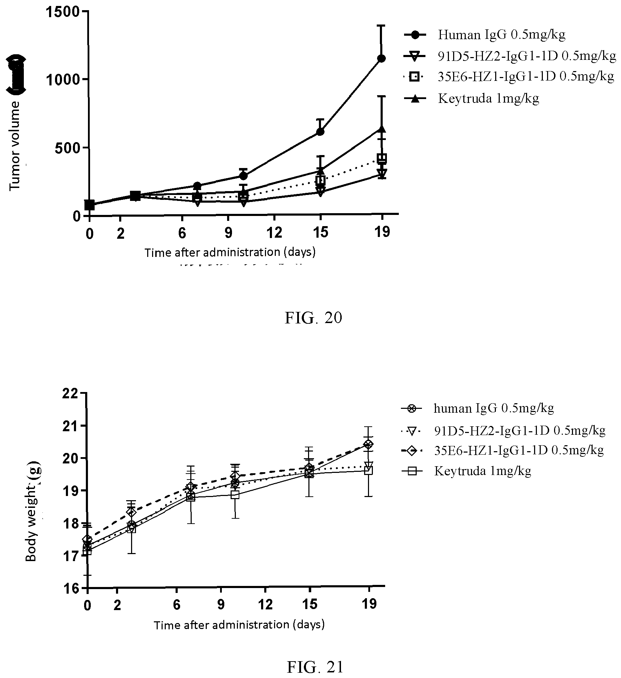

MC38 (mouse colon cancer cell line) cells were cultured with RPMI1640 culture medium containing 10% fetal bovine serum at 37°C, 5% CO₂. MC38 cells in exponential growth phase were collected, resuspended to a suitable concentration with PBS, and inoculated subcutaneously into hPD-1/hTIM-3 transgenic mice to establish a colon cancer model. When tumors reaching an average volume of about 83 mm³, the mice were randomly divided into the following groups according to tumor size: the group of intravenous human immunoglobulin Human IgG (negative control), the group of 91D5-HZ2-IgG1-1D antibody, the group of 35E6-HZ1-IgG1-1D antibody and the group of Keytruda, which were intraperitoneally injected, twice a week for 3 weeks. After administration, the tumor volume and body weight of mice were observed and measured regularly, and the specific results were shown in Table 16, as shown in Figure 20-21. Compared to Human IgG negative control group, 91D5-HZ2-IgG1-1D and 35E6-HZ1-IgG1-1D significantly inhibited the tumor growth of MC38 colon cancer transplanted tumor model, with TGI of 80.30% and 69.23%, respectively, showing better efficacy than Keytruda (TGI=48.69%). Neither animal death nor significant animal weight loss nor obvious drug toxicity was observed in all treatment groups during the observation period, showing a good tolerance in the mice during the treatment.

**Table 16. Drug efficacy of IgG1-1D mutant anti-PD-1 humanized antibody in hPD-1/hTIM-3 transgenic mouse model**

| Grouping | GROUP | P19 | | | |
|---|---|---|---|---|---|
| | | Tumor volume (mm³) (*x̅*± SEM) | TGI (%) | T/C (%) | P value (*Vs*. Group 1) |
| 1 | Human IgG 0.5 mg/kg | 1139.03±241.24 | / | / | / |
| 2 | 91D5-HZ2-IgG1-1D 0.5 mg/kg | 292.26±76.73 | 80.30 | 24.91 | 0.0074 |
| 3 | 35E6-HZ1-IgG1-1D 0.5 mg/kg | 404.70±142.48 | 69.26 | 36.37 | 0.0256 |
| 4 | Keytruda 1mg/kg | 628.24±235.02 | 48.69 | 52.51 | 0.1603 |

Although specific embodiments of the invention have been described in detail, those skilled in the art will understand that various modifications and changes may be made to the details in accordance with all the published teachings, which are also included in the scope of the invention. All the invention is given by the appended claims and any equivalents thereof.

## Claims

1. An antibody or antigen-binding fragment thereof specifically binding to PD-1, wherein the antibody or antigen-binding fragment thereof comprises the following complementary determining regions (CDRs):
(a) CDR-H1 or a sequence variant thereof, CDR-H2 or a sequence variant thereof, and CDR-H3 or a sequence variant thereof, contained in the heavy chain variable region (VH) as set forth in SEQ ID NO: 1; and/or
CDR-L1 or a sequence variant thereof, CDR-L2 or a sequence variant thereof, and CDR-L3 or a sequence variant thereof, contained in the light chain variable region (VL) as set forth in SEQ ID NO: 2;
(b) CDR-H1 or a sequence variant thereof, CDR-H2 or a sequence variant thereof and CDR-H3 or a sequence variant thereof, contained in the VH as set forth in SEQ ID NO: 20; and/or
CDR-L1 or a sequence variant thereof, CDR-L2 or a sequence variant thereof, and CDR-L3 or a sequence variant thereof, contained in the VL as set forth in SEQ ID NO: 21;
(c) CDR-H1 or a sequence variant thereof, CDR-H2 or a sequence variant thereof and CDR-H3 or a sequence variant thereof, contained in the VH as set forth in SEQ ID NO: 36; and/or
CDR-L1 or a sequence variant thereof, CDR-L2 or a sequence variant thereof, and CDR-L3 or a sequence variant thereof, contained in the VL as set forth in SEQ ID NO: 37; or
(d) CDR-H1 or a sequence variant thereof, CDR-H2 or a sequence variant thereof and CDR-H3 or a sequence variant thereof, contained in the VH as set forth in SEQ ID NO: 54; and/or
CDR-L1 or a sequence variant thereof, CDR-L2 or a sequence variant thereof, and CDR-L3 or a sequence variant thereof, contained in the VL as set forth in SEQ ID NO: 55;
wherein the sequence variant is a CDR having a substitution, deletion or addition of one or more amino acids (e.g., a substitution, deletion or addition of one, two or three amino acids) as compared to the CDR from which it is derived; preferably, the substitution is a conservative substitution; preferably, the CDR is defined by the Kabat or IMGT numbering system;
preferably, the VH and/or VL of the antibody or antigen-binding fragment thereof comprises framework regions (FRs) of an immunoglobulin derived from human or mouse;
preferably, the antibody or antigen-binding fragment thereof binds to human PD-1.

2. The antibody or antigen-binding fragment thereof according to claim 1, wherein the antibody or antigen-binding fragment thereof comprises:
(1) a heavy chain variable region (VH) and/or a light chain variable region (VL), wherein, as defined by the IMGT numbering system:
(1a) the VH comprises a complementary determining region (CDR)-H1 with a sequence as set forth in SEQ ID NO: 3, a CDR-H2 with a sequence as set forth in SEQ ID NO: 4 and a CDR-H3 with a sequence as set forth in SEQ ID NO: 5; and/or,
the VL comprises a CDR-L1 with a sequence as set forth in SEQ ID NO: 6, a CDR-L2 with a sequence as set forth in SEQ ID NO: 7 and a CDR-L3 with a sequence as set forth in SEQ ID NO: 8;
(1b) the VH comprises a CDR-H1 with a sequence as set forth in SEQ ID NO: 22, a CDR-H2 with a sequence as set forth in SEQ ID NO: 23 and a CDR-H3 with a sequence as set forth in SEQ ID NO: 24; and/or,
the VL comprises a CDR-L1 with a sequence as set forth in SEQ ID NO: 25, a CDR-L2 with a sequence as set forth in SEQ ID NO: 26 and a CDR-L3 with a sequence as set forth in SEQ ID NO: 27;
(1c) the VH comprises a CDR-H1 with a sequence as set forth in SEQ ID NO: 38, a CDR-H2 with a sequence as set forth in SEQ ID NO: 39 and a CDR-H3 with a sequence as set forth in SEQ ID NO: 40; and/or,
the VL comprises a CDR-L1 with a sequence as set forth in SEQ ID NO: 41, a CDR-L2 with a sequence as set forth in SEQ ID NO: 42 and a CDR-L3 with a sequence as set forth in SEQ ID NO: 43; or
(1d) the VH comprises a CDR-H1 with a sequence as set forth in SEQ ID NO: 56, a CDR-H2 with a sequence as set forth in SEQ ID NO: 57 and a CDR-H3 with a sequence as set forth in SEQ ID NO: 58; and/or,
the VL comprises a CDR-L1 with a sequence as set forth in SEQ ID NO: 59, a CDR-L2 with a sequence as set forth in SEQ ID NO: 60 and a CDR-L3 with a sequence as set forth in SEQ ID NO: 61;
or
(2) a heavy chain variable region (VH) and/or a light chain variable region (VL), wherein, as defined by the Kabat numbering system:
(2a) the VH comprises a CDR-H1 with a sequence as set forth in SEQ ID NO: 9, a CDR-H2 with a sequence as set forth in SEQ ID NO: 10 and a CDR-H3 with a sequence as set forth in SEQ ID NO: 12; and/or,
the VL comprises a CDR-L1 with a sequence as set forth in SEQ ID NO: 13, a CDR-L2 with a sequence as set forth in SEQ ID NO: 14 and a CDR-L3 with a sequence as set forth in SEQ ID NO: 15;
(2b) the VH comprises a CDR-H1 with a sequence as set forth in SEQ ID NO: 28, a CDR-H2 with a sequence as set forth in SEQ ID NO: 29 and a CDR-H3 with a sequence as set forth in SEQ ID NO: 30; and/or,
the VL comprises a CDR-L1 with a sequence as set forth in SEQ ID NO: 31, a CDR-L2 with a sequence as set forth in SEQ ID NO: 32 and a CDR-L3 with a sequence as set forth in SEQ ID NO: 33;
(2c) the VH comprises a CDR-H1 with a sequence as set forth in SEQ ID NO: 44, a CDR-H2 with a sequence as set forth in SEQ ID NO: 45 and a CDR-H3 with a sequence as set forth in SEQ ID NO: 47; and/or,
the VL comprises a CDR-L1 with a sequence as set forth in SEQ ID NO: 48, a CDR-L2 with a sequence as set forth in SEQ ID NO: 49 and a CDR-L3 with a sequence as set forth in SEQ ID NO: 50;
or
(2d) the VH comprises a CDR-H1 with a sequence as set forth in SEQ ID NO: 62, a CDR-H2 with a sequence as set forth in SEQ ID NO: 63 and a CDR-H3 with a sequence as set forth in SEQ ID NO: 64; and/or,
the VL comprises a CDR-L1 with a sequence as set forth in SEQ ID NO: 65, a CDR-L2 with a sequence as set forth in SEQ ID NO: 66 and a CDR-L3 with a sequence as set forth in SEQ ID NO: 67;
or
(3) a heavy chain variable region (VH) and/or a light chain variable region (VL), wherein, at least one CDR contains a mutation selected from a substitution, deletion or addition of one or more amino acids or any combination thereof (e.g. a substitution, deletion or addition of one, two or three amino acids, or any combination thereof), as compared to the VH and/or VL of any one of (1a), (1b), (1c), (1d) or (2a), (2b), (2c), (2d); preferably, the substitution is a conservative substitution.

3. The antibody or antigen-binding fragment thereof according to claim 1 or 2, wherein the antibody or antigen-binding fragment thereof comprises:
(a) a VH set forth in any one of SEQ ID NOs: 1, 20, 36 and 54, and/or a VL set forth in any one of SEQ ID NOs: 2, 21, 37 and 55;
(b) a VH set forth in any one of SEQ ID NOs: 16, 17, 34, 51 and 52, and/or, a VL set forth in any one of SEQ ID NOs: 18, 19, 35, and 53;
(c) a heavy chain variable region (VH) having at least 70%, at least 80%, at least 85%, at least 90%, at least 91%, at least 92%, at least 93%, at least 94%, at least 95%, at least 96%, at least 97%, at least 98%, or at least 99% sequence identity to the VH in either (a) or (b); and/or, a light chain variable region (VL) having at least 70%, at least 80%, at least 85%, at least 90%, at least 91%, at least 92%, at least 93%, at least 94%, at least 95%, at least 96%, at least 97%, at least 98%, or at least 99% sequence identity to the VL in either (a) or (b); or
(d) a heavy chain variable region (VH) having a substitution, deletion or addition of one or more amino acids or any combination thereof (e.g. a substitution, deletion or addition of 1, 2, 3, 4, 5, 6, 7, 8, 9 or 10 amino acids, or any combination thereof) as compared to the VH in either (a) or (b); and/or, a light chain variable region (VL) having a substitution, deletion or addition of one or more amino acids or any combination thereof (e.g. a substitution, deletion or addition of 1, 2, 3, 4, 5, 6, 7, 8, 9 or 10 amino acids, or any combination thereof) as compared to the VL in either (a) or (b); preferably, the substitution is a conservative substitution.

4. The antibody or antigen-binding fragment thereof according to any one of claims 1-3, wherein the antibody or antigen-binding fragment thereof comprises:
(a) a VH with a sequence as set forth in SEQ ID NO: 1 and a VL with a sequence as set forth in SEQ ID NO: 2;
(b) a VH with a sequence as set forth in SEQ ID NO: 16 and a VL with a sequence as set forth in SEQ ID NO: 18;
(c) a VH with a sequence as set forth in SEQ ID NO: 17 and a VL with a sequence as set forth in SEQ ID NO: 19;
(d) a VH with a sequence as set forth in SEQ ID NO: 20 and a VL with a sequence as set forth in SEQ ID NO: 21;
(e) a VH with a sequence as set forth in SEQ ID NO: 34 and a VL with a sequence as set forth in SEQ ID NO: 35;
(f) a VH with a sequence as set forth in SEQ ID NO: 36 and a VL with a sequence as set forth in SEQ ID NO: 37;
(g) a VH with a sequence as set forth in SEQ ID NO: 51 and a VL with a sequence as set forth in SEQ ID NO: 53;
(h) a VH with a sequence as set forth in SEQ ID NO: 52 and a VL with a sequence as set forth in SEQ ID NO: 53;
(i) a VH with a sequence as set forth in SEQ ID NO: 54 and a VL with a sequence as set forth in SEQ ID NO: 55;
(j) a heavy chain variable region (VH) and a light chain variable region (VL), the heavy chain variable region having at least 70%, at least 80%, at least 85%, at least 90%, at least 91%, at least 92%, at least 93%, at least 94%, at least 95%, at least 96%, at least 97%, at least 98%, or at least 99% sequence identity; and/or, the light chain variable region (VL) having at least 70%, at least 80%, at least 85%, at least 90%, at least 91%, at least 92%, at least 93%, at least 94%, at least 95%, at least 96%, at least 97%, at least 98%, or at least 99% sequence identity, compared to the VH and VL in any of (a) to (i); or
(k) a heavy chain variable region (VH) and a light chain variable region (VL), the heavy chain variable region (VH) having a substitution, deletion or addition of one or more amino acids or any combination thereof (e.g. a substitution, deletion or addition of 1, 2, 3, 4, 5, 6, 7, 8, 9 or 10 amino acids, or any combination thereof); and/or, the light chain variable region (VL) having a substitution, deletion or addition of one or more amino acids or any combination thereof (e.g. a substitution, deletion or addition of 1, 2, 3, 4, 5, 6, 7, 8, 9 or 10 amino acids, or any combination thereof), compared to the VH and VL in any of (a) to (i); preferably, the substitution is a conservative substitution.

5. The antibody or antigen-binding fragment thereof according to any one of claims 1-4, wherein the antibody or antigen-binding fragment thereof is a murine antibody, a chimeric antibody or a humanized antibody.

6. The antibody or antigen-binding fragment thereof according to any one of claims 1-5, wherein the antibody or antigen-binding fragment thereof further comprises:
(a) a heavy chain constant region (CH) of a human immunoglobulin or a variant thereof; and/or
(b) a light chain constant region (CL) of a human immunoglobulin or a variant thereof,
wherein the variant has at least 70%, at least 80%, at least 85%, at least 90%, at least 91%, at least 92%, at least 93%, at least 94%, at least 95%, at least 96%, at least 97%, at least 98%, or at least 99% sequence identity to the wild-type sequence from which it is derived; or, the variant has a substitution, deletion, addition of one or more amino acids or any combination thereof (e.g., a substitution, deletion, addition of up to 50, up to 45, up to 40, up to 35, up to 30, up to 25, up to 20, up to 15, up to 10, or up to 5 amino acids, or any combination thereof; for example, a substitution, deletion, addition of 1, 2, 3, 4, 5, 6, 7, 8, 9 or 10 amino acids, or any combination thereof) as compared to the wild-type sequence from which it is derived; preferably, the substitution is a conservative substitution;
preferably, the heavy chain constant region is an IgG heavy chain constant region, such as an IgG1, IgG2, IgG3 or IgG4 heavy chain constant region;
preferably, the antibody or antigen-binding fragment thereof comprises a heavy chain constant region selected from the group consisting of:
(1) a heavy chain constant region of human IgG1; or
(2) a heavy chain constant region of human IgG4;
preferably, the light chain constant region is kappa or lambda light chain constant region, and more preferably, the antibody or antigen-binding fragment thereof comprises a human kappa light chain constant region.

7. The antibody or antigen-binding fragment thereof according to claim 6, wherein the heavy chain constant region or the variant thereof comprises:
(1) a mutant of the heavy chain constant region of human IgG4 (S228P) according to the EU numbering system;
(2) mutants of the heavy chain constant region of human IgG1 having a mutation at at least one of positions 234, 235, 265 and 434 according to the EU numbering system, preferably a heavy chain constant region mutant of human IgG1 having one, two, three or four of the mutations: L234A, L235A, D265A and N434A (e.g., the heavy chain constant region set forth in SEQ ID NO: 83);
(3) the heavy chain constant region (CH) set forth in SEQ ID NO: 82 or a variant thereof, the variant having a conservative substitution of up to 20 amino acids (e.g. a conservative substitution of up to 20, up to 15, up to 10, or up to 5 amino acids; for example, a conservative substitution of 1, 2, 3, 4, 5, 6, 7, 8, 9, or 10 amino acids) as compared to SEQ ID NO: 82; or having at least 70%, at least 80%, at least 85%, at least 90%, at least 91%, at least 92%, at least 93%, at least 94%, at least 95%, at least 96%, at least 97%, at least 98%, or at least 99% sequence identity to SEQ ID NO: 82; or
(4) the heavy chain constant region (CH) set forth in SEQ ID NO: 83 or a variant thereof, the variant having a conservative substitution of up to 20 amino acids (e.g. a conservative substitution of up to 20, up to 15, up to 10, or up to 5 amino acids; for example, a conservative substitution of 1, 2, 3, 4, 5, 6, 7, 8, 9, or 10 amino acids) as compared to SEQ ID NO: 83; or having at least 70%, at least 80%, at least 85%, at least 90%, at least 91%, at least 92%, at least 93%, at least 94%, at least 95%, at least 96%, at least 97%, at least 98%, or at least 99% sequence identity to SEQ ID NO: 83;
wherein the mutation or substitution in (2) or (4) make the antibody or antigen-binding fragment to have no or reduced ADCP, ADCC and/or CDC activities compared to the corresponding antibody or antigen-binding fragment without the mutation or substitution;
and/or
the light chain constant region or the variant thereof comprises:
(5) a kappa light chain constant region; or
(6) the light chain constant region (CL) set forth in SEQ ID NO: 84 or a variant thereof, the variant having a conservative substitution of up to 20 amino acids (e.g. a conservative substitution of up to 20, up to 15, up to 10, or up to 5 amino acids; for example, a conservative substitution of 1, 2, 3, 4, 5, 6, 7, 8, 9, or 10 amino acids) as compared to SEQ ID NO: 84; or having at least 70%, at least 80%, at least 85%, at least 90%, at least 91%, at least 92%, at least 93%, at least 94%, at least 95%, at least 96%, at least 97%, at least 98%, or at least 99% sequence identity to SEQ ID NO: 84;
preferably, the antibody comprises a heavy chain constant region (CH) set forth in SEQ ID NO: 82 or SEQ ID NO: 83 and a light chain constant region (CL) set forth in SEQ ID NO: 84.

8. The antibody or antigen-binding fragment thereof according to any one of claims 1-7, wherein the antibody comprises:
(a) a heavy chain comprising a VH set forth in SEQ ID NO: 1 and a heavy chain constant region (CH) set forth in SEQ ID NO: 83, and a light chain comprising the VL set forth in SEQ ID NO: 2 and a light chain constant region (CL) set forth in SEQ ID NO: 84;
(b) a heavy chain comprising a VH set forth in SEQ ID NO: 16 and a CH set forth in SEQ ID NO: 83, and a light chain comprising the VL set forth in SEQ ID NO: 18 and a CL set forth in SEQ ID NO: 84;
(c) a heavy chain comprising a VH set forth in SEQ ID NO: 17 and a CH set forth in SEQ ID NO: 83, and a light chain comprising a VL set forth in SEQ ID NO: 19 and a CL set forth in SEQ ID NO: 84;
(d) a heavy chain comprising a VH set forth in SEQ ID NO: 20 and a CH set forth in SEQ ID NO: 83, and a light chain comprising a VL set forth in SEQ ID NO: 21 and a CL set forth in SEQ ID NO: 84;
(e) a heavy chain comprising a VH set forth in SEQ ID NO: 34 and a CH set forth in SEQ ID NO: 83, and a light chain comprising a VL set forth in SEQ ID NO: 35 and a CL set forth in SEQ ID NO: 84;
(f) a heavy chain comprising a VH set forth in SEQ ID NO: 36 and a CH set forth in SEQ ID NO: 83, and a light chain comprising a VL set forth in SEQ ID NO: 37 and a CL set forth in SEQ ID NO: 84;
(g) a heavy chain comprising a VH set forth in SEQ ID NO: 51 and a CH set forth in SEQ ID NO: 83, and a light chain comprising a VL set forth in SEQ ID NO: 53 and a CL set forth in SEQ ID NO: 84;
(h) a heavy chain comprising a VH set forth in SEQ ID NO: 52 and a CH set forth in SEQ ID NO: 83, and a light chain comprising a VL set forth in SEQ ID NO: 53 and a CL set forth in SEQ ID NO: 84;
(i) a heavy chain comprising a VH set forth in SEQ ID NO: 54 and a CH set forth in SEQ ID NO: 83, and a light chain comprising a VL set forth in SEQ ID NO: 55 and a CL set forth in SEQ ID NO: 84;
(j) a heavy chain and a light chain, wherein, the heavy chain having at least 70%, at least 80%, at least 85%, at least 90%, at least 91%, at least 92%, at least 93%, at least 94%, at least 95%, at least 96%, at least 97%, at least 98%, or at least 99% sequence identity, and/or, the light chain having at least 70%, at least 80%, at least 85%, at least 90%, at least 91%, at least 92%, at least 93%, at least 94%, at least 95%, at least 96%, at least 97%, at least 98%, or at least 99% sequence identity, to the heavy chain and the light chain in any of (a) to (i).

9. The antibody or antigen-binding fragment thereof according to any one of claims 1-8, wherein the antibody or antigen-binding fragment thereof comprises:
(a) a heavy chain and a light chain,
the heavy chain comprising:
(i) a sequence set forth in SEQ ID NO: 86;
(ii) a sequence having a substitution, deletion or addition of one or several amino acids or any combination thereof (e.g., a substitution, deletion or addition of up to 50, up to 45, up to 40, up to 35, up to 30, up to 25, up to 20, up to 15, up to 10, or up to 5 amino acids, or any combination thereof; for example, a substitution, deletion or addition of 1, 2, 3, 4, 5, 6, 7, 8, 9 or 10 amino acids, or any combination thereof) as compared to the sequence set forth in (i); or
(iii) a sequence having at least 80%, at least 85%, at least 90%, at least 91%, at least 92%, at least 93%, at least 94%, at least 95%, at least 96%, at least 97%, at least 98%, or at least 99% sequence identity to the sequence set forth in (i); and
the light chain comprising:
(iv) a sequence set forth in SEQ ID NO: 88;
(v) a sequence having a substitution, deletion or addition of one or several amino acids or any combination thereof (e.g., a substitution, deletion or addition of up to 50, up to 45, up to 40, up to 35, up to 30, up to 25, up to 20, up to 15, up to 10, or up to 5 amino acids, or any combination thereof; for example, a substitution, deletion or addition of 1, 2, 3, 4, 5, 6, 7, 8, 9 or 10 amino acids, or any combination thereof) as compared to the sequence set forth in (iv); or
(vi) a sequence having at least 80%, at least 85%, at least 90%, at least 91%, at least 92%, at least 93%, at least 94%, at least 95%, at least 96%, at least 97%, at least 98%, or at least 99% sequence identity to the sequence set forth in (iv);
preferably, the substitution in (ii) or (v) is a conservative substitution,
or
(b) a heavy chain and a light chain,
the heavy chain comprising:
(i) a sequence set forth in SEQ ID NO: 90;
(ii) a sequence having a substitution, deletion or addition of one or several amino acids or any combination thereof (e.g., a substitution, deletion or addition of up to 50, up to 45, up to 40, up to 35, up to 30, up to 25, up to 20, up to 15, up to 10, or up to 5 amino acids, or any combination thereof; for example, a substitution, deletion or addition of 1, 2, 3, 4, 5, 6, 7, 8, 9 or 10 amino acids, or any combination thereof) as compared to the sequence set forth in (i); or
(iii) a sequence having at least 80%, at least 85%, at least 90%, at least 91%, at least 92%, at least 93%, at least 94%, at least 95%, at least 96%, at least 97%, at least 98%, or at least 99% sequence identity to the sequence set forth in (i); and
the light chain comprising:
(iv) a sequence set forth in SEQ ID NO: 11;
(v) a sequence having a substitution, deletion or addition of one or several amino acids or any combination thereof (e.g., a substitution, deletion or addition of up to 50, up to 45, up to 40, up to 35, up to 30, up to 25, up to 20, up to 15, up to 10, or up to 5 amino acids, or any combination thereof; for example, a substitution, deletion or addition of 1, 2, 3, 4, 5, 6, 7, 8, 9 or 10 amino acids, or any combination thereof) as compared to the sequence set forth in (iv); or
(vi) a sequence having at least 80%, at least 85%, at least 90%, at least 91%, at least 92%, at least 93%, at least 94%, at least 95%, at least 96%, at least 97%, at least 98%, or at least 99% sequence identity to the sequence set forth in (iv);
preferably, the substitution in (ii) or (v) is a conservative substitution.

10. The antibody or antigen-binding fragment thereof according to any one of claims 1-9, wherein the antibody or antigen-binding fragment thereof is selected from the group consisting of scFv, Fab, Fab', F(ab')₂, Fv fragments, disulfide-linked Fv (dsFv), and a diabody;
preferably, the scFv comprising:
(a) a VH with a sequence as set forth in SEQ ID NO: 1 and a VL with a sequence as set forth in SEQ ID NO: 2;
(b) a VH with a sequence as set forth in SEQ ID NO: 16 and a VL with a sequence as set forth in SEQ ID NO: 18;
(c) a VH with a sequence as set forth in SEQ ID NO: 17 and a VL with a sequence as set forth in SEQ ID NO: 19;
(d) a VH with a sequence as set forth in SEQ ID NO: 20 and a VL with a sequence as set forth in SEQ ID NO: 21;
(e) a VH with a sequence as set forth in SEQ ID NO: 34 and a VL with a sequence as set forth in SEQ ID NO: 35;
(F) a VH with a sequence as set forth in SEQ ID NO: 36 and a VL with a sequence as set forth in SEQ ID NO: 37;
(g) a VH with a sequence as set forth in SEQ ID NO: 51 and a VL with a sequence as set forth in SEQ ID NO: 53;
(h) a VH with a sequence as set forth in SEQ ID NO: 52 and a VL with a sequence as set forth in SEQ ID NO: 53; or
(i) a VH with a sequence as set forth in SEQ ID NO: 54 and a VL with a sequence as set forth in SEQ ID NO: 55.

11. The antibody or antigen-binding fragment thereof according to any one of claims 1-10, wherein the antibody or antigen-binding fragment thereof carries a marker;
preferably, the antibody or antigen-binding fragment thereof carries a detectable marker such as an enzyme (e.g. horseradish peroxidase), a radionuclide, a fluorescent dye, a luminescent substance (e.g. a chemiluminescent substance) or biotin.

12. The antibody or antigen-binding fragment thereof according to any one of claims 1-11, wherein the antibody or antigen-binding fragment thereof has at least one of the following characteristics:
(a) binding to PD-1 (e.g., human PD-1) with a K_{D} of less than 50 nM, e.g., less than 20 nM, 10 nM, 1 nM, 0.1 nM, 0.01 nM, 1 pM, 0.1 pM or less; preferably, the K_{D} is measured by Bio-Layer Interferometry (BLI) or ELISA;
(b) binding to PD-1 (e.g., human PD-1) with an EC₅₀ of less than 50 nM, e.g., less than 20 nM, 10 nM, 1 nM, 0.9 nM, 0.8 nM, 0.7 nM, 0.6 nM, 0.5 nM, 0.4 nM, 0.3 nM, 0.2 nM, 0.1 nM, 0.01 nM, 1 pM, 0.1 pM or less; preferably, the EC₅₀ is measured by flow cytometry or cell competitive ELISA technology;
(c) competing for the binding of PD-1 to PD-L1 or PD-L2 with an IC₅₀ in a range of from about 0.1 ng/mL to about 1000 ng/mL; preferably, the IC₅₀ is measured by ELISA technology;
(d) increasing activation of T cells, e.g., increasing IFN-γ and/or IL-2 expression in T lymphocytes;
(e) not binding to BTLA, PD-L1 or ICOS;
(f) killing or inhibiting the growth of tumor cells;
(g) having a good thermal stability; or
(h) having no or reduced at least one of ADCP, ADCC, and CDC activities, for example, having no or reduced ADCC activity; having no or reduced ADCP; having no or reduced ADCP and having no or reduced ADCC activity; or, having no or reduced ADCP, ADCC and CDC activities.

13. An isolated nucleic acid molecule encoding the antibody or antigen-binding fragment thereof according to any one of claims 1-12, the heavy chain and/or the light chain thereof, or the heavy chain variable region and/or the light chain variable region thereof.

14. The isolated nucleic acid molecule according to claim 13, comprising a nucleic acid molecule encoding the heavy chain of the antibody and/or a nucleic acid molecule encoding the light chain of the antibody, wherein,
(a) the nucleic acid molecule encoding the heavy chain of the antibody has a sequence selected from the group consisting of:
(i) a nucleotide sequence set forth in SEQ ID NO: 85 or a degenerate sequence thereof,
(ii) a sequence substantially identical to the nucleotide sequence of (i) (e.g. a sequence having at least about 85%, 90%, 95%, 99% or more sequence identity, or a sequence having one or more nucleotide substitutions, as compared to the nucleotide sequence of (i)), or
(iii) a sequence which differs from the nucleotide sequence of (i) in not more than 3, 6, 15, 30 or 45 nucleotides,
and/or,
the nucleic acid molecule encoding the light chain of the antibody has a sequence selected from the group consisting of:
(iv) a nucleotide sequence set forth in SEQ ID NO: 87 or a degenerate sequence thereof,
(v) a sequence substantially identical to the nucleotide sequence of (iv) (e.g. a sequence having at least about 85%, 90%, 95%, 99% or more sequence identity, or a sequence having one or more nucleotide substitutions, as compared to the nucleotide sequence of (iv)), or
(vi) a sequence which differs from the nucleotide sequence of (iv) in not more than 3, 6, 15, 30 or 45 nucleotides;
or
(b) the nucleic acid molecule encoding the heavy chain of the antibody has a sequence selected from the group consisting of:
(i) a nucleotide sequence set forth in SEQ ID NO: 89 or a degenerate sequence thereof,
(ii) a sequence substantially identical to the nucleotide sequence of (i) (e.g. a sequence having at least about 85%, 90%, 95%, 99% or more sequence identity, or a sequence having one or more nucleotide substitutions, as compared to the nucleotide sequence of (i)), or
(iii) a sequence which differs from the nucleotide sequence of (i) in not more than 3, 6, 15, 30 or 45 nucleotides,
and/or,
the nucleic acid molecule encoding the light chain of the antibody has a sequence selected from the group consisting of:
(iv) a nucleotide sequence set forth in SEQ ID NO: 46 or a degenerate sequence thereof,
(v) a sequence substantially identical to the nucleotide sequence of (iv) (e.g. a sequence having at least about 85%, 90%, 95%, 99% or more sequence identity, or a sequence having one or more nucleotide substitutions, as compared to the nucleotide sequence of (iv)), or
(vi) a sequence which differs from the nucleotide sequence of (iv) in not more than 3, 6, 15, 30 or 45 nucleotides,
preferably, the nucleic acid molecule encoding the heavy chain of the antibody has a nucleotide sequence set forth in SEQ ID NO: 85 or a degenerate sequence thereof, and/or the nucleic acid molecule encoding the light chain of the antibody has a nucleotide sequence set forth in SEQ ID NO: 87 or a degenerate sequence thereof;
preferably, the nucleic acid molecule encoding the heavy chain of the antibody has a nucleotide sequence set forth in SEQ ID NO: 89 or a degenerate sequence thereof, and/or the nucleic acid molecule encoding the light chain of the antibody has a nucleotide sequence set forth in SEQ ID NO: 46 or a degenerate sequence thereof.

15. A vector comprising the isolated nucleic acid molecule according to claim 13 or 14; preferably, the vector is a cloning vector or an expression vector.

16. A host cell comprising the isolated nucleic acid molecule according to claim 13 or 14, or the vector according to claim 15.

17. A method for preparing the antibody or antigen-binding fragment thereof according to any one of claims 1-12, comprising culturing the host cell according to claim 16 under conditions allowing the expression of the antibody or antigen-binding fragment thereof, and recovering the antibody or antigen-binding fragment thereof from the cultured host cell culture.

18. A multispecific antibody comprising a first antibody or fragment thereof, and an additional antibody or fragment thereof, or an antibody mimetic; wherein the first antibody or fragment thereof is the antibody or antigen-binding fragment thereof specifically binding to PD-1 according to any one of claims 1-12;
preferably, the multispecific antibody is a bispecific antibody or a trispecific antibody or a tetraspecific antibody.

19. A conjugate comprising an antibody or antigen-binding fragment thereof and a coupling moiety, wherein the antibody or antigen-binding fragment thereof is the antibody or antigen-binding fragment thereof according to any one of claims 1-12, and the coupling moiety is a detectable marker, such as a radioisotope, a fluorescent substance, a luminescent substance, a colored substance or an enzyme, or the coupling moiety is an agent, such as a chemotherapy agent, a radionuclide or a toxin.

20. A cell expressing a chimeric antigen receptor (CAR), comprising or expressing the antibody or antigen-binding fragment thereof according to any one of claims 1-12, the nucleic acid according to claim 13 or 14 or the vector according to claim 15; preferably, the cell is derived from an immune cell, or the antigen-binding fragment is selected from scFv; more preferably, the cell is derived from T lymphocytes, NK cells, monocytes, macrophages or dendritic cells and any combination thereof.

21. An oncolytic virus comprising the nucleic acid according to claim 13 or 14 or the vector according to claim 15.

22. A fusion protein comprising an antibody or antigen-binding fragment thereof and a non-immunoglobulin moiety, wherein the antibody or antigen-binding fragment thereof is the antibody or antigen-binding fragment thereof according to any one of claims 1-12, and the non-immunoglobulin moiety is a polypeptide, such as a cytokine (e.g., IL-2, IL-15, IL-7, IL-12, IL-18, IL-21, TNF-alpha or IFNgamma).

23. A pharmaceutical composition comprising the antibody or antigen-binding fragment thereof according to any one of claims 1-12, the nucleic acid molecule according to claim 13 or 14, the vector according to claim 15, the host cell according to claim 16, the multispecific antibody according to claim 18, the conjugate according to claim 19, the CAR cell according to claim 20, the oncolytic virus according to claim 21, and/or the fusion protein according to claim 22, and a pharmaceutically acceptable carrier and/or excipient;
optionally, the pharmaceutical composition further comprises an additional pharmaceutically active agent, such as an anti-tumor agent, a radiopharmaceutical, an agent associated with an immunoregulatory effect, or an agent associated with an autoimmune disease; more preferably, the anti-tumor agent is selected from antibodies associated with an immunoregulatory effect, such as an anti-LAG-3 antibody (e.g., AB12T8) or a TIM-3 antibody (e.g., AB12S3).

24. The pharmaceutical composition according to claim 23, wherein the pharmaceutical composition comprises an effective dose of the antibody or antigen-binding fragment thereof sufficient to elicit at least one of the following biological activities in a subject:
(1) inhibiting or blocking the binding of PD-1 to PD-L1 or PD-L2,
(2) down-regulating or eliminating the activity of PD-1,
(3) reducing or relieving the immunosuppression induced by PD-1 or PD-L1 or PD-L2,
(4) increasing the production of IFN γ or IL-2 in T lymphocytes,
(5) enhancing the ability of T lymphocytes to kill tumor cells.

25. A kit comprising the antibody or antigen-binding fragment thereof according to any one of claims 1-12, and/or the nucleic acid according to claim 13 or 14, and/or the vector according to claim 15, and/or the host cell according to claim 16, and/or the multispecific antibody according to claim 18, and/or the conjugate according to claim 19, and/or the cell expressing a chimeric antigen receptor (CAR) according to claim 20, and/or the oncolytic virus according to claim 21, and/or the fusion protein according to claim 22, and/or the pharmaceutical composition according to claim 23 or 24, and optionally an instruction.

26. Use of the antibody or antigen-binding fragment thereof according to any one of claims 1-12, or the nucleic acid according to claim 13 or 14, or the vector according to claim 15, or the host cell according to claim 16, or the multispecific antibody according to claim 18, or the conjugate according to claim 19, or the cell expressing a chimeric antigen receptor (CAR) according to claim 20, or the oncolytic virus according to claim 21, or the fusion protein according to claim 22, or the pharmaceutical composition of claim 23 or 24, or the pharmaceutical composition of claim 23 or 24, and an additional pharmaceutically active agent (e.g. an anti-tumor agent, a radiopharmaceutical, an agent associated with an immunoregulatory effect, or an agent associated with an autoimmune disease) in the preparation of a medicament for preventing and/or treating a tumor, an infection, or an autoimmune disease,
optionally, the tumor is derived from one or more of the following tissues: skin, bone marrow, blood, lymph, head and neck, brain, lung, breast, stomach, gallbladder, liver, pancreas, intestine, ovary, prostate, adrenal gland, kidney, bladder, uterus, cervix, testis, penis and soft tissues, optionally, the infection is selected from the group consisting of a bacterial infection, a pathogen infection, a fungal infection, and a viral infection;
optionally, the infection is a viral infection, such as HIV, hepatitis B or hepatitis C; optionally, the infection is tuberculosis;
optionally, the autoimmune disease is selected from the group consisting of: diabetes, myasthenia gravis, gastritis, pemphigus, primary biliary cirrhosis, multiple sclerosis, lupus, colitis, rheumatoid diseases and thyroid diseases;
preferably, the tumor is selected from the group consisting of: lung cancer, liver cancer, ovarian cancer, cervical cancer, skin cancer, colon cancer, rectal cancer, glioma, bladder cancer, breast cancer, kidney cancer, esophageal cancer, gastric cancer, oral squamous cell cancer, urethral epithelial cell cancer and pancreatic cancer, and head and neck tumors;
preferably, the anti-tumor agent is selected from antibodies associated with an immunoregulatory effect, such as an anti-LAG-3 antibody (e.g., AB12T8) or a TIM-3 antibody (e.g., AB12S3); optionally, the pharmaceutical composition according to claim 23 or 24 and the additional pharmaceutically active agent are administered in combination or separately, concurrently or sequentially.

27. The use according to claim 26, wherein the medicament is for at least one of:
(1) inhibiting or blocking the binding of PD-1 to PD-L1 or PD-L2,
(2) down-regulating or eliminating the activity of PD-1,
(3) reducing or relieving the immunosuppression induced by PD-1 or PD-L1 or PD-L2,
(4) increasing the production of IFN γ or IL-2 in T lymphocytes,
(5) enhancing the ability of T lymphocytes to kill tumor cells.

28. A method for performing at least one of (1) inhibiting or blocking the binding of PD-1 to PD-L1 or PD-L2, (2) down-regulating or eliminating the activity of PD-1, (3) reducing or relieving the immunosuppression of PD-1 or PD-L1 or PD-L2, (4) increasing the production of IFN γ or IL-2 in T lymphocytes and (5) enhancing the ability of T lymphocytes to kill tumor cells, in vivo or in vitro, comprising:
administrating an effective amount of the antibody or antigen-binding fragment thereof according to any one of claims 1-12, or the nucleic acid according to claim 13 or 14, or the vector according to claim 15, or the host cell according to claim 16, or the multispecific antibody according to claim 18, or
the conjugate according to claim 19, or the cell expressing a chimeric antigen receptor (CAR) according to claim 20, or the oncolytic virus according to claim 21, or the fusion protein according to claim 22, or the pharmaceutical composition according to claim 23 or 24,
optionally, administrating one or several of an anti-tumor agent, a radiopharmaceutical, an agent associated with an immunoregulatory effect, or an agent associated with an autoimmune disease concurrently with, before or after administration of the antibody or antigen-binding fragment thereof,
the nucleic acid, the vector, the host cell, the multispecific antibody, the conjugate, or the pharmaceutical composition.

29. A method for preventing and/or treating a tumor, an infection, or an autoimmune disease in a subject, comprising administering to a subject in need thereof an effective amount of the pharmaceutical composition according to claim 23 or 24, wherein the subject is a mammal; preferably, the subject is human.

30. A method of detecting the presence or level of PD-1 in a sample, comprising contacting the sample with the antibody or antigen-binding fragment thereof according to any one of claims 1-12 under conditions allowing the formation of a complex between the antibody or antigen-binding fragment thereof and PD-1, and detecting the formation of the complex.
